# EUROPÄISCHE PATENTSCHRIFT

(11) **EP 3 119 489 B1**
(45) Veröffentlichungstag und Bekanntmachung des Hinweises auf die Patenterteilung: **25.08.2021**
(21) Anmeldenummer: 15709501.9
(22) Anmeldetag: 13.03.2015
(51) Int. Cl.: B01D 9/00, C07C 29/56, C07C 29/80, C07C 37/84, C07F 5/06, C07C 35/17, C07C 39/36, G01N 21/85, G01N 21/49, G01N 21/84

(54) **VERFAHREN ZUM ABTRENNEN EINES STOFFES AUS EINER LÖSUNG**
METHOD FOR SEPARATING A SUBSTANCE FROM A SOLUTION
PROCÉDÉ DE SÉPARATION D'UNE SUBSTANCE À PARTIR D'UNE SOLUTION

(30) Priorität: 19.03.2014 EP 14160645
(43) Veröffentlichungstag der Anmeldung: 25.01.2017
(60) Teilanmeldung: 21181477.7
(73) Patentinhaber: BASF SE, 67056 Ludwigshafen am Rhein (DE)
(72) Erfinder: RAULS, Matthias, 66440 Blieskastel (DE); ZIEGLER, Stefan, 76857 Eußerthal (DE); HAUBNER, Martin, 69214 Eppelheim (DE)
(74) Vertreter: BASF IP Association
(86) Internationale Anmeldenummer: PCT/EP2015/055279
(87) Internationale Veröffentlichungsnummer: WO 2015/140062

(56) Entgegenhaltungen:
- EP-A1- 2 338 579
- WO-A1-2010/095034
- US-A- 4 672 218
- US-B2- 7 608 742
- CHEN Z P ET AL: "On-line monitoring of batch cooling crystallization of organic compounds using ATR-FTIR spectroscopy coupled with an advanced calibration method", CHEMOMETRICS AND INTELLIGENT LABORATORY SYSTEMS, ELSEVIER SCIENCE PUBLISHERS B.V. AMSTERDAM, NL, Bd. 96, Nr. 1, 15. März 2009 (2009-03-15), Seiten 49-58, XP026003132, ISSN: 0169-7439, DOI: 10.1016/J.CHEMOLAB.2008.11.002 [gefunden am 2008-12-06]
- Gilles F?votte: "New perspectives for the on-line monitoring of pharmaceutical crystallization processes using in situ infrared spectroscopy", INTERNATIONAL JOURNAL OF PHARMACEUTICS, vol. 241, no. 2, 1 July 2002 (2002-07-01), pages 263-278, XP055256452, NL ISSN: 0378-5173, DOI: 10.1016/S0378-5173(02)00237-5
- Shih-Yao B Hu ET AL: "Application of Near-Infrared Spectra on Temperature-Controlled Protein Crystallization: A Simulation Study", Molecular Biotechnology, vol. 94, no. 2, 1 May 2001 (2001-05-01), pages 179-196, XP055255575, Boston ISSN: 1073-6085, DOI: 10.1385/ABAB:94:2:179
- Zai Qun Yu ET AL: "Seeding and Constant-Supersaturation Control by ATR-FTIR in Anti-Solvent Crystallization", ORGANIC PROCESS RESEARCH AND DEVELOPMENT, vol. 10, no. 4, 1 July 2006 (2006-07-01), pages 717-722, XP055256455, US ISSN: 1083-6160, DOI: 10.1021/op060058j
- ZOLTAN K. NAGY ET AL:: "Advances and New Directions in Crystallization Control", ANNUAL REVIEW OF CHEMICAL AND BIOMOLECULAR ENGINEERING, vol. 3, no. 1, 15 July 2012 (2012-07-15), pages 55-75, XP055255496, US ISSN: 1947-5438, DOI: 10.1146/annurev-chembioeng-062011-081043

## Beschreibung

Die vorliegende Erfindung betrifft ein Verfahren zum Abtrennen eines Stoffes aus einer Lösung. Die Abtrennung erfolgt dabei über ein Kristallisationsverfahren. Des Weiteren betrifft die Erfindung ein Verfahren zur Aufarbeitung eines Aluminium-haltigen Reaktionsprodukts aus der Herstellung von Isopulegol durch Cyclisierung von Citronellal in Gegenwart von Komplexverbindungen, umfassend wenigstens einen Liganden der Formel (I):

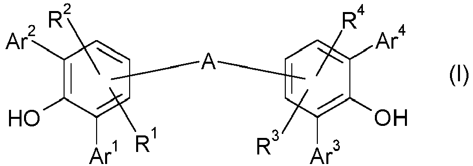

Darüber hinaus betrifft die Erfindung ein Verfahren zur Herstellung von Isopulegol sowie ein Verfahren zur Herstellung von Menthol.

Menthol stellt weltweit die mengenmäßig wichtigste Aromachemikalie dar. Der Bedarf an Menthol wird nach wie vor zu einem großen Teil durch Isolierung aus natürlichen Quellen gedeckt. Daneben existieren jedoch auch synthetische Zugänge zum Menthol, teils in racemischer Form, teils in Form des natürlichen Enantiomeren L-Menthol.

Ein wichtiges Zwischenprodukt zur Herstellung von racemischem wie optisch aktivem Menthol stellt Isopulegol dar, das üblicherweise durch cyclisierende Oxo-En-Reaktion von Citronellal in Gegenwart von Lewis-sauren Katalysatoren hergestellt wird und dabei in der Regel in Form von Gemischen der vier Diastereomere Isopulegol, iso-Isopulegol, neo-Isopulegol und neoiso-Isopulegol anfällt.

Als geeignete Katalysatoren zur Durchführung der vorstehend genannten Cyclisierung von Citronellal zu Isopulegol wurden sowohl heterogene Katalysatoren, wie SiO₂, Al₂O₃/SiO₂-, SiO₂/ZrO₂-, SiO₂/TiO₂-Mischkatalysatoren, Mordenite, Faujasite, Monmorillonite und Zeolithe - als auch homogene Katalysatoren, wie beispielsweise Sulfonsäuren oder Lewis-Säuren, wie beispielsweise SnCl₄, ZnCl₂ oder ZnBr₂ beschrieben.

Die EP-A 1 225 163 beschreibt die Cyclisierung von Citronellal zu Isopulegol in Gegenwart von Tris(2,6-diphenylphenol)-aluminium-Katalysatoren. Bei diesem Verfahren zur Cyclisierung von Citronellal zu Isopulegol werden teure und nur aufwändig herzustellende Katalysatorkomplexe eingesetzt. Nach dem beschriebenen, in homogener Phase durchzuführenden Verfahren wird der Katalysatorkomplex nach beendeter Reaktion hydrolysiert. Eine mögliche Wiedergewinnung und Wiedereinsetzbarkeit des dabei freigesetzten Liganden ist nicht beschrieben.

Demgegenüber beschreibt die WO 2006/092433 A1 Bis(diarylphenoxy)-AluminiumVerbindungen, die durch Umsetzung eines Bis(diarylphenol)-Liganden der Formel (I) mit einer geeigneten Aluminiumverbindung erhältlich sind, sowie Verfahren zur Herstellung von Isopulegol und Menthol in Gegenwart dieser Verbindungen. Dabei werden auch Verfahren offenbart, die eine Rückgewinnung der verwendeten Bis(diarylphenol)-Liganden der Formel (I) ermöglichen. Die Rückgewinnung erfolgt durch Kristallisation aus einem bei der destillativen Abtrennung von Isopulegol aus einem Reaktionsprodukt der Cyclisierung von Citronellal erhaltenen Sumpfprodukt. Eine solche Aufarbeitung führt jedoch, insbesondere bei einem kontinuierlichen Verfahren zur Herstellung von Isopulegol, zu Ausbeuten und Reinheiten, die nicht vollständig zufriedenstellend sind.

Aus der WO 2008/025851 A1 ist ein Verfahren zur Aufarbeitung eines Aluminium-haltigen Reaktionsprodukts aus der Herstellung von Isopulegol durch Cyclisierung von Citronellal bekannt, bei dem man das Reaktionsprodukt einer destillativen Auftrennung unter Erhalt eines an Isopulegol angereicherten Kopfprodukts und eines an Isopulegol abgereicherten Sumpfprodukts unterzieht und den Liganden aus dem Sumpfprodukt abtrennt.

Des Weiteren ist aus der WO 2009/068444 A2 ein Verfahren zur Herstellung von Menthol bekannt, bei dem Neral und/oder Geranial zu Citronellal katalytisch hydriert wird und Citronellal zu Isopulegol in Gegenwart eines sauren Katalysators cyclisiert wird. Isopulegol wird dann durch Kristallisation aufgereinigt und katalytisch zu Menthol hydriert.

Bei den vorstehend genannten Verfahren besteht ein großes Bedürfnis, den Liganden mit einer hohen Ausbeute zurückzugewinnen. Seine Synthese ist nämlich sehr aufwändig, sodass die nahezu vollständige Rückgewinnung des Liganden für die Wirtschaftlichkeit des Gesamtverfahrens von großer Bedeutung ist.

EP 2 338 579 A1 beschreibt ein Kristallisationsverfahren, bei dem die Anzahl der ausgefallenen Kristallteilchen gemessen wird, während eine Lösung gekühlt wird. Hieraus wird eine absolute Teilchenanzahl berechnet. Wenn die absolute Teilchenanzahl überschritten wurde, wird die gesättigte Lösung erhitzt, um die Lösung danach wieder abzukühlen. Die Teilchenanzahl wird dabei über ein Verfahren zum Messen der Größe der Teilchen bestimmt. Die Teilchengröße wird mittels FBRM *(Focus Beam Reflectance Method)* gemessen. Bei diesem Verfahren wird ein Laserstrahl in die Lösung eingestrahlt und die Reflektion dieses Laserstrahls gemessen.

In CHEN Z P ET AL, "On-line monitoring of batch cooling crystallization of organic compounds using ATR-FTIR spectroscopy coupled with an advanced calibration method" (CHEMOMETRICS AND INTELLIGENT LABORATORY SYSTEMS, ELSEVIER SCIENCE PUBLISHERS B.V. AMSTERDAM, NL, vol. 96, no. 1, doi:10.1016/J.CHEMOLAB.2008.11.002, ISSN 0169-7439, (20090315), Seiten 49-58) wird ein ATR-FTIR Verfahren zur Überwachung eines Kristallisationsverfahrens beschrieben. Bei einem solchen Verfahren wird eine ATR *(attenuated total reflection,* abgeschwächte Totalreflektion)-Sonde mit einem FTIR Spektrometer (Fourier-Transformations-Infrarotspektrometer) kombiniert. Bei dieser Kombination handelt es sich um eine spezielle Variante eines Messgeräts für Infrarotspektroskopie.

Aus der WO2010/095034 A1 ist ein Verfahren zur Herstellung von natürlichem L-Menthol bekannt. Bei diesem Verfahren wird der Impfprozess mittels detektiertem gestreutem Licht überwacht.

Schließlich beschreibt die US 4,672,218 ein Verfahren, bei dem das Einsetzen der Kristallisation bestimmt wird. Dabei wird Licht, welches an der Oberfläche eines Kristalls gestreut wurde, gemessen. Bei dem verwendeten Licht kann es sich beispielsweise um nahes Infrarotlicht handeln.

Der vorliegenden Erfindung liegt die Aufgabe zugrunde, ein Verfahren der eingangs genannten Art bereitzustellen, bei denen eine bessere Effizienz bei dem Kristallisationsverfahren erzielt wird, welches im Rahmen des Verfahrens zum Abtrennen eines Stoffes bzw. bei der Vorrichtung zum Abtrennen des Stoffes eingesetzt wird. Dabei soll außerdem die Zeit, die zum Gewinnen des Stoffes benötigt wird, verkürzt werden.

Erfindungsgemäß wird diese Aufgabe durch ein Verfahren mit dem Merkmal des Anspruchs 1 gelöst.

Dementsprechend wird bei dem erfindungsgemäßen Verfahren zum Abtrennen eines Stoffes durch Kristallisation aus einer Lösung des Stoffes eine Suspension von Impfkristallen erzeugt und, wenn eine gewünschte Menge an Impfkristallen vorliegt, ein Kristallisationsverfahren begonnen, bei dem Kristalle des Stoffes gewonnen werden, die anschließend abgetrennt werden. Zum Erzeugen der gewünschten Menge an Impfkristallen wird elektromagnetische Strahlung in die Lösung eingestrahlt, wobei die in die Lösung eingestrahlte elektromagnetische Strahlung die Form eines Strahls aufweist, dessen Öffnungswinkel größer als 5 Grad ist, und im sichtbaren Spektralbereich liegt oder Infrarotstrahlung ist. Zum Einstellen einer gewünschten Menge an Impfkristallen wird eine Intensität der elektromagnetischen Strahlung, die von in der Lösung befindlichen Kristallen gestreut wurde, detektiert. Die detektierte Intensität wird mit einer Soll-Intensität verglichen. Die Temperatur der Lösung wird in Abhängigkeit von der Differenz der detektierten Intensität und der Soll-Intensität so geregelt, dass sich der Betrag dieser Differenz verkleinert. Die gewünschte Menge an Impfkristallen für das Kristallisationsverfahren liegt dann vor, wenn der Betrag der Differenz der detektierten Intensität und der Soll-Intensität kleiner als ein Grenzwert ist.

Unter einer Lösung wird in dieser Schrift nicht nur eine klare Lösung verstanden, bei der alle Feststoffe gelöst sind, sondern auch eine partikelbeladene Lösung. Solch eine partikelbeladene Lösung wird auch als Suspension bezeichnet. Eine partikelbeladene Lösung kann Kristalle enthalten, die sich bei der Kristallisation gebildet haben. Bei der Kristallisation findet ggf. ein Übergang von einer klaren Lösung zu einer partikelbeladene Lösung, d.h. einer Suspension, statt. Im Folgenden werden die Bezeichnungen Lösung und Suspension daher synonym verwendet.

Die detektierte Intensität beinhaltet dabei zwar nicht die gesamte elektromagnetische Strahlung, die von den in der Lösung befindlichen Kristallen gestreut wurde, sondern nur einen Teil dieser Gesamtstreuintensität, insbesondere nur einen zu dieser Gesamtstreuintensität proportionalen Anteil. Allerdings kann mittels dieser Intensitätsmessung die Veränderung der Kristalloberfläche ausreichend erfasst werden, um die Temperatur für den Beginn des Kristallisationsverfahrens zu regeln.

Es wurde gefunden, dass die Kristallisation zum Gewinnen des Stoffes nur vergleichsweise langsam vonstatten geht, wenn der aus der Lösung abzutrennende Stoff eine sehr komplexe Molekülstruktur hat. Die Bildung neuer Kristallkeime und das Wachstum der Kristalle erfolgen in einem solchen Fall nur stark verzögert. Maßstab hierfür ist z.B. die Unterkühlbarkeit der Kristallisationslösung. Diese kann z.B. bei einem Liganden mit komplexer Molekülstruktur bis zu 50 K betragen, während die Unterkühlbarkeit der Lösungen einfacherer organischer Moleküle typischerweise nur 1 K bis 5 K beträgt.

Überraschenderweise wurde nun gefunden, dass die Kristallisation eines Stoffes mit komplexer Molekülstruktur in eine gut filtrierbare Kristallgröße und -morphologie insbesondere dann gelingt, wenn die Kristallisation mit einer sehr genau bemessenen Menge an Impfkristallen begonnen wird. Diese Impfkristalle können dann durch langsame Abkühlung der Lösung zu größeren, leicht filtrierbaren Kristallen gezüchtet werden. Überraschenderweise wurde außerdem gefunden, dass die richtige Menge an Impfkristallen sich nur innerhalb eines engen Intervalls befindet und dass diese ungewöhnlich exakt einzuhaltende Menge mittels der Detektion der Intensität der elektromagnetischen Strahlung, die von den in der Lösung befindlichen Kristallen gestreut wurde, relativ einfach eingestellt werden kann. Es hat sich gezeigt, dass sich der Filterwiderstand um mehr als eine Größenordnung ändert, wenn die Lösung zu stark oder zu gering angeimpft wird. Wegen der Linearität zwischen dem Filterwiderstand und der Dauer der Filtration bzw. alternativ zur Verfügung zu stellenden Filterflächen ergibt sich, dass die Filtration auf den zur Verfügung stehenden Apparaten bzw. in der zur Verfügung stehenden Zeit unmöglich wird, wenn die genau bemessene Impfkristallmenge zum Beginn des Kristallisationsverfahrens verfehlt wird.

Durch das erfindungsgemäße Verfahren wird es nun erreicht, dass die Anfangsbedingungen des im Rahmen des erfindungsgemäßen Verfahrens eingesetzten Kristallisationsverfahrens exakt geregelt werden. Hierdurch erreicht man, dass eine gewünschte Menge an Impfkristallen zu Beginn dieses Kristallisationsverfahrens vorliegt, um mittels des Kristallisationsverfahrens eine für die Abtrennung der Kristalle ideale Kristallgröße und -morphologie zu erreichen.

Das stabile Erzeugen bzw. Erhalten von Impfkristallen im Prozess setzt voraus, dass der Zusammenhang zwischen der aktuellen Konzentration des zu kristallisierenden Stoffes und der dazugehörigen Sättigungstemperatur genau bekannt ist. Es ist aber eine Eigentümlichkeit dieses komplexen chemischen Verfahrens, dass, abhängig von den genauen Bedingungen der vorhergehenden Reaktion, von der aktuellen Zusammensetzung des aufgearbeiteten, im Verfahren rückgeführten Lösemittels und von der sich einstellenden Konzentration lösender und nicht lösender Nebenkomponenten die Sättigungstemperaturen der vorgelegten Lösung in einem großen Bereich z.B. zwischen 85 °C bis 115 °C schwanken. Da andererseits empirisch bekannt ist, dass die Temperatur, bei der die Impfkristalle erzeugt werden, sehr genau, z.B. auf 1 K bis 3 K, kontrolliert werden muss, um die gewünschten Ergebnisse bei der Kristallisation zu erzielen, ist ein automatisches Einstellen dieser Bedingungen a priori nicht möglich. Das erfindungsgemäße Verfahren ermöglicht es nun, trotz der sich aus dem Prozess ergebenden Unsicherheiten eine einfache, automatisierbare Regelung des korrekten Animpfens bereitzustellen.

Als einfache Methode zur Umgehung des Problems des korrekten Animpfens würde es sich anbieten, die Kristallisation kontinuierlich durchzuführen. In einem als stationär angenommenen Zustand wird die heiße Feedlösung in den kontinuierlich betriebenen Kristallisator gefördert werden, dessen hoher Kristallgehalt ein Animpfen überflüssig macht. Durch Abziehen einer Kristallsuspension wird die Zufuhr der neuen Lösung ausgeglichen, sodass ein solcher Kristallisator mit stets konstantem Füllstand und Feststoffgehalt betrieben werden würde. Es hat sich jedoch herausgestellt, dass die extrem große Unterkühlbarkeit der Lösungen von Stoffen mit komplexer Molekülstruktur und die nur sehr träge Neigung zur Kristallisation eine solche Vorgehensweise unmöglich macht. Beim Einbringen der heißen Feedlösung in einen kühlen Kristallisator wird eine so hohe Übersättigung aufgeprägt, dass es zur Bildung sehr vieler, viel zu kleiner und damit schwer filtrierbarer Kristalle kommt. Diese Schwierigkeit könnte durch eine Kaskadierung verringert werden, bei der mehrere Kristallisatoren auf jeweils nur geringfügig unterschiedlichen Temperaturniveaus betrieben werden. Die Notwendigkeit zahlreicher Temperaturstufen und damit einer großen Zahl von Apparaten macht dieses Verfahren aber wirtschaftlich nachteilig.

Außerdem könnte man die aktuelle Konzentration des Stoffes mit physikalischen oder chemischen Methoden messen und aus der Zusammensetzung der Lösung eine Sättigungstemperatur und damit eine optimale Impftemperatur ableiten. Eine solche Messung kann inline z.B. mit spektroskopischen Methoden erfolgen oder offline in einem chemischen Labor geschehen. Letztere Methode bedeutet jedoch einen zu hohen Zeitaufwand; sie scheitert wie die spektroskopische Messung aber auch dann, wenn eine sehr komplexe Zusammensetzung der Lösung vorliegt. Diese Zusammensetzung wirkt sich nämlich auf die genaue Lösetemperatur aus. Es muss nämlich die genaue Konzentration aller anderen Inhaltsstoffe der Lösung gemessen werden, die, wie z.B. das Isopulegol, die Löslichkeit stark erhöhen können, oder die, wie die mit "Ester" bezeichnete Stoffgruppe, die Löslichkeit stark verringern. Zudem müsste ein exakter Zusammenhang zwischen der Konzentration aller Komponenten und der sich einstellenden Sättigungstemperatur bekannt sein, was für ein komplex zusammengesetztes Medium nicht erreicht werden kann.

Ist eine messtechnische Erfassung des Zusammenhangs zwischen der Konzentration und der Sättigungstemperatur nicht möglich, so könnte doch die empirische Bestimmung der Sättigungstemperatur ein Ausweg sein. Beispielsweise kann man die Sättigungstemperatur einer solchen Lösung durch gezielte Unterkühlung der Lösung, Erzwingen der Kristallbildung bei u.U. hoher Unterkühlung und Wiedererwärmung der Lösung unter Bestimmung der Lösetemperatur des letzten Kristalls bestimmen. Dieses Verfahren kann auch wieder aufwändig an einer Probe im Labor erfolgen; es ist auch denkbar, diese Messung automatisiert durch ein beliebiges Messsystem im Bypass zum eigentlichen Kristallisator durchzuführen. Bei einer hohen Unterkühlbarkeit der Lösung und wegen der Notwendigkeit, die Lösung sehr langsam zu erwärmen, um die Sättigungstemperatur genau zu bestimmen, würde eine solche Bestimmung zumindest viele Stunden dauern und wäre damit wirtschaftlich nachteilig.

Bei dem erfindungsgemäßen Verfahren wird zur automatisierten Einstellung der anfänglich zur Verfügung zu stellenden Menge an Impfkristallen eine optische Messmethode in Verbindung mit einer Regelung der Anfangsbedingungen für das im Rahmen des Verfahrens eingesetzte Kristallisationsverfahren verwendet. Dabei wird elektromagnetische Strahlung in die Lösung eingestrahlt und die von etwa vorhandenen Kristallen zurückgestreute elektromagnetische Strahlung detektiert. Diese Rückstreuung ist für Kristalle, die deutlich größer als die Wellenlänge der eingestrahlten elektromagnetischen Strahlung sind, in erster Näherung proportional zur Menge der in der Suspension vorhandenen Kristalloberfläche. Bei dem erfindungsgemäßen Verfahren wird somit nicht die Masse an Kristallen zu Beginn des Kristallisationsverfahrens erfasst, sondern die Kristalloberfläche. Hierdurch kann ein noch besseres Kristallwachstum und damit eine noch höhere Ausbeute und ein geringerer Zeitaufwand bei der Abtrennung der Kristalle erreicht werden, da nicht die Masse an Kristallen für das Gelingen der Kristallisation wichtig ist, sondern die zur Aufnahme von Übersättigung bereite Kristalloberfläche. Aus diesem Grund ist die Messung der Kristalle mittels der elektromagnetischen Strahlung zur Regelung der Bedingungen zu Beginn des Kristallisationsverfahrens besonders vorteilhaft.

Bei dem Grenzwert für den Betrag der Differenz der detektierten Intensität und der Soll-Intensität handelt es sich um eine tolerierte Abweichung von der Soll-Intensität. Dieser Grenzwert kann beispielsweise 5% oder 20% von der Soll-Intensität sein. Der Grenzwert kann allerdings auch auf der Basis von absoluten Abweichungen von der Soll-Intensität bestimmt werden. Liegt der Zielwert z. B. bei 0,1 so könnte man den Zielbereich von 0,15 bis 0,1 wählen. Liegt der Zielwert hingegen bei 0,5 so würde man den Zielbereich von 0,55 bis 0,5 wählen.

Gemäß einer Ausgestaltung des erfindungsgemäßen Verfahrens wird der kristalline Stoff durch Filtration abgetrennt. Bei der Regelung der Anfangsbedingungen des Kristallisationsverfahrens wurde eine Soll-Intensität gewählt, welche eine Kristalloberfläche bereitstellt, die zu einer Kristallgröße und -morphologie führt, die insbesondere für die Filtration bevorzugt ist. Auf diese Weise werden eine besonders hohe Ausbeute und ein geringerer Zeitaufwand bei der Abtrennung der Kristalle aus der Lösung erzielt. Des Weiteren kann der kristalline Stoff auch beispielsweise durch Flotation, Zentrifugation oder Siebung aus der Lösung isoliert, d.h. abgetrennt, werden.

Gemäß einer weiteren Ausgestaltung des erfindungsgemäßen Verfahrens wird die Soll-Intensität durch Referenzmessungen bestimmt. Bei diesen Referenzmessungen wird für die Lösung der Zusammenhang zwischen der Kristallgröße und/oder der Kristallmorphologie am Ende des Kristallisationsverfahrens und der detektierten Intensität zu Beginn des Kristallisationsverfahrens bestimmt. Hieraus wird die Soll-Intensität als diejenige Intensität gewählt, die der gewünschten Kristallgröße und/oder Kristallmorphologie zugeordnet ist. Auf diese Weise kann im Voraus bestimmt werden, welche Intensität der rückgestreuten elektromagnetischen Strahlung den idealen Anfangsbedingungen für das Kristallisationsverfahren entspricht, bei denen die gewünschten Kristalle für die nachfolgende Abtrennung gezüchtet werden. Die auf diese Weise bestimmte Soll-Intensität entspricht dann einer idealen Kristalloberfläche in einem Lösungsvolumen. Dies entspricht wiederum in etwa einer Kristallkonzentration. Auf Basis dieser Soll-Intensität der rückgestreuten elektromagnetischen Strahlung kann somit bei dem erfindungsgemäßen Verfahren die Temperatur für den Beginn des Kristallisationsverfahrens geregelt werden. Vorteilhafterweise kann dies ohne detaillierte Kenntnis der Konzentration des zu kristallisierenden Stoffes oder der Menge lösender und nicht lösender Substanzen erfolgen.

Gemäß einer Weiterbildung des erfindungsgemäßen Verfahrens wird die Lösung oder ein Teil der Lösung in ein Kristallisationsgefäß bei einer Temperatur eingeleitet, die bei einem definierten Anfangstemperaturwert liegt, der unterhalb der voraussichtlichen Sättigungstemperatur der Lösung liegt. Die Lösung wird dann erwärmt, bis der Betrag der Differenz der detektierten Intensität und der Soll-Intensität kleiner als der Grenzwert ist. Die Lösung wird insbesondere auf eine Temperatur weit unterhalb der voraussichtlichen Sättigungstemperatur gebracht, sodass sich spontan eine große Anzahl von Kristallen des zu kristallisierenden Stoffes bilden. Auf diese Weise werden in situ Impfkristalle gewonnen. Bei dieser Ausgestaltung des erfindungsgemäßen Verfahrens regelt man somit die Temperatur der Lösung von niedrigeren zu höheren Temperaturen, um eine ideale Ausgangstemperatur für das Kristallisationsverfahren zu erhalten. Bei dieser Regelung ist die vorhandene Kristallmenge zunächst sehr viel größer als gewünscht. Außerdem entspricht die Kristallgröße und -morphologie nicht der gewünschten Kristallgröße und -morphologie. Durch die Steigerung der Temperatur bei der Regelung lösen sich dann Kristalle auf, bis die detektierte Intensität der rückgestreuten elektromagnetischen Strahlung anzeigt, dass die gewünschte Kristalloberfläche in der Lösung vorliegt. Wenn bei dieser Regelung eine zu niedrige detektierte Intensität auftritt, kann durch Absenken der Temperatur der Lösung gegengesteuert werden, da sich dann erneut die Kristalloberfläche vergrößert.

Der Anfangstemperaturwert liegt insbesondere zumindest 10 K unterhalb der voraussichtlichen Sättigungstemperatur der Lösung. Andererseits kann der Anfangstemperaturwert auch aus der Soll-Intensität bestimmt werden. Beispielsweise wird die dem Anfangstemperaturwert zugeordnete Anfangsintensität als die x-fache Intensität der Soll-Intensität gewählt, wobei der Wert x in einem Bereich von 1,2 bis 10 liegt. Insbesondere liegt der Wert x in einem Bereich von 3 bis 10, bevorzugt in einem Bereich von 4 bis 9 und besonders bevorzugt in einem Bereich von 6 bis 9. Die Temperatur der Lösung wird dann so geregelt, bis die detektierte Intensität größer als die Anfangsintensität ist. Durch diese Vorgehensweise kann vorteilhafterweise auf sehr einfache Art sichergestellt werden, dass zunächst eine ausreichend große Menge an Kristallen in der Lösung vorhanden ist, welche dann bei der Regelung sukzessive verringert wird, bis die Soll-Intensität, d.h. die ideale Ausgangstemperatur für den Beginn des Kristallisationsverfahrens, vorliegt.

Gemäß einer Ausgestaltung des erfindungsgemäßen Verfahrens ist die Unterkühlbarkeit der verwendeten Lösung größer als 5 K, insbesondere größer als 10 K oder größer als 30 K. In solchen Fällen ist die Durchführung des Kristallisationsverfahrens besonders kritisch hinsichtlich der Bildung von Kristallen, die für eine spätere Filtration geeignet sind. In diesen Fällen ist es somit besonders wichtig, dass die Anfangsbedingungen, d.h. insbesondere die Kristalloberfläche zu Beginn des Kristallisationsverfahrens, ideal für das Kristallwachstum während des Kristallisationsverfahrens sind. Mit dem erfindungsgemäßen Verfahren kann durch die Einstrahlung der elektromagnetischen Strahlung und der Messung der Intensität der rückgestreuten Strahlung sichergestellt werden, dass ideale Bedingungen zu Beginn der Kristallisation vorliegen.

Gemäß einer Ausgestaltung des erfindungsgemäßen Verfahrens wird die elektromagnetische Strahlung eines Wellenlängenbereichs oder mehrerer Wellenlängenbereiche, der/die breiter als 20 nm (z.B. 740 nm bis 760 nm) ist/sind, in die Lösung bzw. Suspension eingestrahlt. Die eingestrahlte elektromagnetische Strahlung umfasst somit verschiedene Wellenlängen, die sich zumindest über einen Bereich von 20 nm erstrecken. Es wird somit insbesondere nicht wie bei Laserstrahlung monochromatisches Licht bzw. monochromatische Strahlung, d.h. Strahlung in einem sehr engen Frequenzbereich eingestrahlt, sondern Licht bzw. Strahlung verschiedener Wellenlängen. Der Wellenlängenbereich kann insbesondere auch noch sehr viel breiter sein und sich über 50 nm, 100 nm oder mehr nm erstrecken.

Die in die Lösung bzw. Suspension eingestrahlte elektromagnetische Strahlung weist die Form eines Strahls auf. Gemäß einer Ausgestaltung des erfindungsgemäßen Verfahrens ist der minimale Querschnitt dieses Strahls größer als 0,1 mm, insbesondere größer als 0,19 mm und bevorzugt größer als 0,39 mm. Des Weiteren ist der Strahl divergent, d.h. er besitzt einen Öffnungswinkel. Dieser Öffnungswinkel ist größer als 5°, insbesondere größer als 10° und bevorzugt größer als 20°. Da sich der Querschnitt eines solchen divergenten Strahls in Strahlungsrichtung vergrößert, ist der minimale Querschnitt eines solchen Strahls der Querschnitt des Strahls beim Eintritt in die Lösung bzw. Suspension.

Die eingestrahlte elektromagnetische Strahlung kann beispielsweise im sichtbaren Spektralbereich liegen. Bevorzugt wird jedoch Infrarotstrahlung in die Lösung eingestrahlt. Entsprechend wird die Intensität von Infrarotstrahlung detektiert. Die Infrarotstrahlung kann beispielsweise in einem Wellenlängenbereich von 780 nm bis 1000 nm, insbesondere in einem Bereich von 800 nm bis 900 nm und bevorzugt in einem Bereich von 820 nm bis 880 nm liegen. Die Wellenlänge der eingestrahlten elektromagnetischen Strahlung entspricht dabei der Wellenlänge der detektierten, rückgestreuten Strahlung.

Gemäß einer bevorzugten Ausgestaltung des erfindungsgemäßen Verfahrens wird die elektromagnetische Strahlung mittels einer Streulichtsonde in die Lösung eingestrahlt. Gleichermaßen wird die Intensität der rückgestreuten elektromagnetischen Strahlung mittels der Streulichtsonde detektiert. Dabei ist insbesondere die Einstrahlrichtung der eingestrahlten elektromagnetischen Strahlung im Wesentlichen parallel zu der Detektionsrichtung, aus der die Intensität der rückgestreuten elektromagnetischen Strahlung detektiert wird. Auf diese Weise wird verhindert, dass in die Lösung eingestrahlte elektromagnetische Strahlung direkt detektiert wird, ohne dass diese Strahlung an Kristallen gestreut worden ist.

Bei dem erfindungsgemäßen Verfahren kann durch die vorstehend charakterisierte Einstrahlung der elektromagnetischen Strahlung in die Lösung bzw. Suspension, insbesondere mittels einer Streulichtsonde, durch Detektion der Intensität der elektromagnetischen Strahlung, die von in der Lösung bzw. Suspension befindlichen Kristallen gestreut wurde, ein Signal gewonnen werden, das proportional zur Partikeloberfläche eines Partikelkollektivs in der Lösung bzw. Suspension ist. Durch dieses Signal kann die Anfangstemperatur für den Beginn des Kristallisationsverfahrens besonders genau eingestellt werden, da hierdurch die anfänglich zur Verfügung stehende Menge an Impfkristallen sehr genau erfasst werden kann.

Das erfindungsgemäße Verfahren weist dabei wesentliche Vorteile gegenüber einer Messung der Partikelgrößenverteilung und der Partikelanzahl auf, wie sie beispielsweise auch über FBRM (focused beam reflectance measurement)-Verfahren gewonnen werden. Bei dem FBRM-Verfahren wird die Partikelgrößenverteilung nicht direkt ermittelt, sondern über eine sogenannte Sehnenlängenverteilung. Hierfür wird ein Laserstrahl in die Lösung mit den Partikeln eingestrahlt. Der Laserstrahl hat einen sehr kleinen Querschnitt von einigen Mikrometern. Ferner rotiert er mit konstanter Geschwindigkeit von etwa 2 m/s. Vom rotierenden Laserstrahl getroffene Partikel werden auf diese Weise gescannt. Gemessen wird die elektromagnetische Strahlung, die durch Reflektion des Laserstrahls an den Partikeln von einem Sensor detektiert werden. Aus der vorgegebenen Rotationsgeschwindigkeit, mit welcher der Laserstrahl bewegt wird, und den Pulsdauern werden dann Sehnenlängenverteilungen berechnet. In der Literatur wird darauf hingewiesen, dass die Berechnung einer Partikelgrößenverteilung aus der Sehnenlängenverteilung sehr schwierig und fehlerbehaftet ist. So ergeben sich je nach der Relativgeschwindigkeit der vorbeiströmenden Partikel Fehler bei der Berechnung der Sehnenlängenverteilungen.

Wenn die Partikelgeschwindigkeit gegen Null geht, ergeben sich zusätzlich Fehler bei der Partikelzählrate, da fast alle Partikel mehrmals mit verschiedenen Sehnenlängen gemessen werden. Außerdem liegt der nur einige Mikrometer große, rotierende Fokus des Laserstrahls, welcher in die Lösung bzw. Suspension eingestrahlt wird, und des Strahls, welcher nach der Reflektion zum Detektor gelangt, sehr nahe an der Scheibe des Sensorkopfes. Daraus ergibt sich, dass weiter entfernte Partikel aufgrund des optischen Strahlenganges zu deutlich intensitätsärmeren Signalen mit geringerer Flankensteilheit führen. Bei einer Unterschreitung einer Mindestflankensteilheit werden diese Signale verworfen, da die geforderte räumliche Auflösung nicht mehr gegeben ist. Da sich bei einer sehr niedrigen Anzahl von Partikeln pro Raumvolumen nur sehr wenige Partikel pro Zeiteinheit direkt beim Sensorkopf aufhalten und damit vom rotierenden Fokuspunkt des Laserstrahls gescannt werden können, werden in diesem Fall auch nur sehr niedrige Zählraten erzielt. Selbst wenn dann die Mindestflankensteilheit als Parameter auf einen sehr niedrigen Wert gesetzt wird, können weiter entfernte Partikel nicht detektiert werden, da die Signalverarbeitungselektronik auf eine hohe Grenzfrequenz ausgelegt ist, um eine hohe zeitliche Auflösung des Signals zu gewährleisten. Die Signalverarbeitungselektronik ist in diesem Fall nicht auf eine hohe Lichtsensitivität ausgelegt. Das FBRM-Verfahren wurde daher für mittlere bis hohe Partikelkonzentrationen entwickelt.

Bei dem FBRM-Verfahren kann somit allenfalls eine Teilchenanzahl in der Lösung bzw. Suspension bestimmt werden. Eine zuverlässige Messung der Partikelgrößenverteilung ist nicht möglich, da diese Partikelgrößenverteilung nur aus einer Sehnenlängenverteilung gewonnen wird und die Umrechnung dieser Verteilung in eine Partikelgrößenverteilung fehlerbehaftet ist und bestimmte Annahmen trifft. So ist es beispielsweise erforderlich, ein Modell der dreidimensionalen geometrischen Formen der betreffenden Partikel zugrunde zu legen.

Bei dem erfindungsgemäßen Verfahren, bei welchem die eingestrahlte elektromagnetische Strahlung die vorstehend genannten Merkmale hat, wobei diese Strahlung insbesondere von einer sogenannten Streulichtsonde erzeugt wird, kann die Oberfläche von Partikelkollektiven in Suspensionen mit einer sehr geringen bis zu einer sehr hohen Anzahl von Partikeln pro Raumvolumen gemessen werden. Dabei ist es möglich, nur die Intensität, d.h. insbesondere die Gesamtintensität, der elektromagnetischen Strahlung, die von in der Lösung befindlichen Kristallen gestreut wurde, zu detektieren. Bei dem FBRM-Verfahren wird keine direkte Intensitätsmessung durchgeführt. Bei diesem Verfahren wird nämlich für jede Partikelart die Verstärkung von der Signalverarbeitungselektronik anders eingestellt, damit weder ein zu hohes noch ein zu niedriges Signal erfasst wird. Bei dem FBRM-Verfahren kommt es nämlich nur auf die Anzahl und die Dauer der Lichtimpulse an, die sich aus der Reflektion an den Partikeln ergeben, keinesfalls jedoch auf die Intensität der reflektierten Strahlung.

Bei dem erfindungsgemäßen Verfahren zum Abtrennen eines Stoffes aus einer Lösung bzw. Suspension, bei dem die Kristallkonzentration auf einen sehr geringen Wert geregelt werden soll, um eine bestimmte Partikeloberfläche zu erzielen, ist das Messverfahren mittels der Streulichtsonde bzw. mittels elektromagnetischer Strahlung, welche die vorstehend genannten Merkmale hat, besser geeignet als das FBRM-Verfahren, da es eine höhere Sensitivität besitzt. Außerdem ist es sehr viel kostengünstiger zu implementieren. Der Aufwand, der bei der Detektion betrieben werden muss, ist sehr viel geringer, da nur ein integrales Intensitätssignal erfasst werden muss.

Gemäß einer Ausgestaltung des erfindungsgemäßen Verfahrens wird die Lösung (Suspension) in ein Kristallisationsgefäß mit einer Temperatur eingeleitet, die bei einem Anfangstemperaturwert liegt. Die eingeleitete Lösung weist somit eine große Kristallmenge auf. Wenn sich die Streulichtsonde innerhalb der eingeleiteten Lösung befindet, d.h. wenn das Kristallisationsgefäß so weit gefüllt ist, dass der Füllstand der Lösung oberhalb der Streulichtsonde ist, wird die elektromagnetische Strahlung mittels der Streulichtsonde in die Lösung eingestrahlt und die Intensität der elektromagnetischen Strahlung detektiert, die von den in der Lösung befindlichen Kristallen gestreut wurde. Nun wird die Temperatur der Lösung beim weiteren Einleiten der Lösung in das Kristallisationsgefäß so geregelt, dass der Betrag der Differenz der detektierten Intensität und der Soll-Intensität kleiner als der Grenzwert wird. Im Idealfall ist bei dieser Verfahrensführung bei vollständiger Befüllung des Kristallisationsgefäßes die detektierte Intensität gleich der Soll-Intensität bzw. der Betrag der Differenz dieser Intensitäten kleiner als der Grenzwert, sodass bei vollständiger Befüllung des Kristallisationsgefäßes die gewünschte Impfkristallmenge vorhanden ist.

Auf diese Weise kann der Zeitaufwand für die Durchführung des erfindungsgemäßen Verfahrens verkürzt werden. Wenn nach der vollständigen Befüllung des Kristallisationsgefäßes der Betrag der Differenz der detektierten Intensität und der Soll-Intensität noch größer als der Grenzwert ist, kann noch durch eine Feinjustierung der Temperatur die detektierte Intensität der Soll-Intensität so weit angenähert werden, dass der Betrag der Differenz unterhalb des Grenzwertes liegt und somit dann die gewünschte Impfkristallmenge vorliegt.

Unter dem Kristallisationsverfahren, das im Rahmen des erfindungsgemäßen Verfahrens zum Abtrennen des Stoffes aus der Lösung verwendet wird, wird in dieser Schrift der Teil des Verfahrens, der beginnt, wenn der Betrag der Differenz der detektierten Intensität und der Soll-Intensität kleiner als ein Grenzwert ist, obwohl auch in vorhergehenden Teilen des Verfahrens sich Kristalle gebildet haben.

Bei dem Kristallisationsverfahren, das im Rahmen des erfindungsgemäßen Verfahrens zum Abtrennen des Stoffes aus der Lösung verwendet wird, handelt es sich insbesondere um ein Kühlkristallisationsverfahren. Nachdem somit die Temperatur der Lösung so geregelt wurde, dass der Betrag der Differenz der detektierten Intensität und der Soll-Intensität kleiner als der Grenzwert ist, wird dann die Lösung langsam wieder abgekühlt, sodass sich, unterstützt von den in situ gewonnenen Impfkristallen, wieder größere Kristalle bilden. Die Abkühlrate ist dabei zunächst relativ gering, später, wenn sich bereits größere Kristalle gebildet haben, kann die Abkühlrate auch zur Beschleunigung des Verfahrens erhöht werden.

Die Erfindung betrifft des Weiteren ein Verfahren zum Gewinnen eines Stoffes aus einer Lösung mittels Kristallisation, bei dem die Lösung in ein erstes Kristallisationsgefäß eingeleitet wird und der Stoff mittels Kristallisation in dem ersten Kristallisationsgefäß nach dem vorstehend beschriebenen Verfahren abgetrennt wird. Während der Durchführung des Kristallisationsverfahrens in dem ersten Kristallisationsgefäß wird die Lösung in ein zweites Kristallisationsgefäß eingeleitet und der Stoff mittels Kristallisation in dem zweiten Kristallisationsgefäß nach dem vorstehend beschriebenen Verfahren abgetrennt. Somit wird während der Durchführung des Kristallisationsverfahrens in dem ersten Kristallisationsgefäß, z.B. durch eine Kühlungskristallisation, das gleiche Verfahren in dem zweiten Kristallisationsgefäß von vorne begonnen. Auf diese Weise kann das Verfahren zum Abtrennen des Stoffes aus der Lösung im Wesentlichen kontinuierlich betrieben werden, da während der Kristallisation in dem einen Kristallisationsgefäß die Lösung in das andere Kristallisationsgefäß eingeleitet wird und dabei die Regelung durchgeführt wird, um zu erreichen, dass die gewünschte Kristalloberfläche für den Beginn des Kristallisationsverfahrens in dem zweiten Kristallisationsgefäß vorliegt. Gegebenenfalls können auch noch weitere Kristallisationsgefäße parallel geschaltet werden. Die Anzahl der Kristallisationsgefäße richtet sich beispielsweise danach, wie viel Zeit benötigt wird, um die gewünschten Anfangsbedingungen für das Kristallisationsverfahren herzustellen, und wie lange dann das eigentliche Kristallisationsverfahren benötigt. Dabei kann die Anzahl der Kristallisationsgefäße beispielsweise so gewählt werden, dass so lange Lösung in Kristallisationsgefäße eingeleitet wird, bis das Kristallisationsverfahren im ersten Kristallisationsgefäß abgeschlossen ist und in dieses wieder die Lösung eingeleitet werden kann.

Eine Vorrichtung zum Ausführen des erfindungsgemäßen Verfahrens zum Abtrennen eines Stoffes aus einer Lösung weist zumindest ein Kristallisationsgefäß auf, welches eine Öffnung zum Einleiten der Lösung umfasst. Des Weiteren umfasst die Vorrichtung eine Temperiereinrichtung zum Verändern der Temperatur der einzuleitenden und/oder der eingeleiteten Lösung. Des Weiteren ist ein Temperatursensor zum Messen der Temperatur der einzuleitenden und/oder eingeleiteten Lösung vorgesehen. Innerhalb des Kristallisationsgefäßes ist eine Streulichtsonde angeordnet, mit welcher elektromagnetische Strahlung in die Lösung einstrahlbar ist und eine Intensität der elektromagnetischen Strahlung detektierbar ist, die von in der Lösung befindlichen Kristallen gestreut wurde. Die Vorrichtung umfasst des Weiteren eine Regeleinheit, die mit dem Temperatursensor, der Streulichtsonde und der Temperiereinrichtung datentechnisch gekoppelt ist. Mit dieser Regeleinheit ist die Temperatur der Lösung in dem Kristallisationsgefäß so regelbar, dass sich der Betrag der Differenz der detektierten Intensität und einer Soll-Intensität verkleinert. Wenn der Betrag der Differenz der detektierten Intensität und der Soll-Intensität kleiner als ein Grenzwert ist, ist ein Kristallisationsverfahren ansteuerbar. Durch das Kristallisationsverfahren werden Kristalle des Stoffes gewonnen. Schließlich umfasst die Vorrichtung eine Abtrenneinheit zum Abtrennen der gewonnenen Kristalle.

Die Temperiereinrichtung ist insbesondere in der Leitung angeordnet, über welche die Lösung dem Kristallisationsgefäß zugeführt wird. Auf diese Weise kann die Temperatur der zugeführten Lösung geregelt werden.

Der Temperatursensor ist insbesondere im Kristallisationsgefäß angeordnet, sodass die Temperatur der in dem Kristallisationsgefäß befindlichen Lösung gemessen wird. Zusätzlich ist vorzugsweise noch ein Temperatursensor in der Leitung vorgesehen, über welche die Lösung dem Kristallisationsgefäß zugeführt wird.

Die Streulichtsonde weist insbesondere einen Emitter für Infrarotstrahlung auf. Die von dem Emitter emittierte Strahlung wird über einen Wellenleiter in das Kristallisationsgefäß geleitet. Dabei ist die Auskoppelfläche des Wellenleiters im unteren Bereich des Kristallisationsgefäßes angeordnet, sodass ab einem gewissen Füllstand elektromagnetische Strahlung in die Lösung eingestrahlt wird. Gleichermaßen weist die Streulichtsonde insbesondere eine Einkoppelfläche eines weiteren Wellenleiters im Kristallisationsgefäß auf. Das über die Einkoppelfläche eingekoppelte Streulicht wird über den weiteren Wellenleiter zu einem Detektor der Streulichtsonde geleitet.

Gemäß einer Ausgestaltung der Vorrichtung liegt die von der Streulichtsonde eingestrahlte elektromagnetische Strahlung in einem Wellenlängenbereich oder mehreren Wellenlängenbereichen, der/die breiter als 20 nm ist/sind. Der Wellenlängenbereich oder die Wellenlängenbereiche kann/können insbesondere jedoch auch breiter als 50 nm oder 100 nm sein. Gemäß einer weiteren Ausgestaltung der Vorrichtung weist der von der Streulichtsonde erzeugbare Strahl einen minimalen Querschnitt auf, der größer als 0,1 mm, insbesondere größer als 0,39 mm ist. Ferner weist der Strahl bevorzugt einen Öffnungswinkel auf, der größer als 5°, bevorzugt größer als 10° und insbesondere größer als 20° ist.

Mittels der Regeleinheit wird das Kristallisationsverfahren insbesondere so durchgeführt, dass die Lösung abgekühlt wird, sodass Kristalle des Stoffes gewonnen werden. Zum Abtrennen der gewonnenen Kristalle ist die Abtrenneinheit so ausgebildet, dass die Kristalle durch Filtration, Flotation, Zentrifugation oder Siebung aus der Lösung isoliert werden können.

Diese Vorrichtung ist insbesondere ausgebildet, das vorstehend beschriebene erfindungsgemäße Verfahren auszuführen. Sie weist somit dieselben Vorteile wie das Verfahren auf.

Gemäß einer Weiterbildung der Vorrichtung umfasst diese zwei Kristallisationsgefäße. Für beide Kristallisationsgefäße sind in diesem Fall eine Temperiereinrichtung, ein Temperatursensor und eine Streulichtsonde vorgesehen. Die Regeleinheit steuert in diesem Fall die Lösungsmittelzufuhr so, dass während der Durchführung des Kristallisationsverfahrens in dem ersten Kristallisationsgefäß die Lösung in das zweite Kristallisationsgefäß eingeleitet wird und anschließend dann auch in dem zweiten Kristallisationsgefäß das Kristallisationsverfahren durchgeführt wird.

Gegenstand der vorliegenden Erfindung ist des Weiteren ein Verfahren zur Aufarbeitung eines Aluminium-haltigen Reaktionsprodukts aus der Herstellung von Isopulegol durch Cyclisierung von Citronellal, enthaltend
i) Isopulegol,
ii) wenigstens einen Liganden der Formel (I), 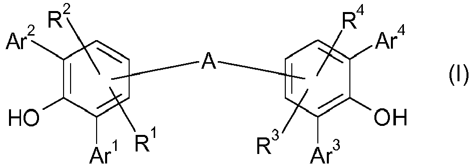 wobei
   - Ar¹, Ar², Ar³, Ar⁴: unabhängig voneinander ausgewählt sind unter C6-C15-Arylresten oder C₂-C₁₅-Heteroarylresten, die gegebenenfalls jeweils 1 bis 7 gleiche oder verschiedene Substituenten, ausgewählt unter C₁-C₆-Alkyl, C₁-C₆-Perfluoralkyl, C₁-C₆-Alkoxy, C₇-C₁₂-Aralkyl, Halogen, SiR^{5a}R^{6a}R^{7a}, gegebenenfalls substituiertem C₆-C₁₀-Aryl, NR^{8a}R^{9a}, SR^{10a}, NO₂ tragen können, bedeuten,
   - R¹, R², R³, R⁴: unabhängig voneinander ausgewählt sind unter Wasserstoff, C₁-C₆-Alkyl, C₁-C₆-Perfluoralkyl, C₁-C₆-Alkoxy, C₇-C₁₂-Aralkyl, Halogen, SiR^{5b}R^{6b}R^{7b}, gegebenenfalls substituiertem C₆-C₁₀-Aryl, NR^{8b}R^{9b}, SR^{10b}, NO₂ und wobei
   R¹ oder R² und/oder R³ oder R⁴ gemeinsam mit A einen aromatischen oder nicht-aromatischen Cyclus bilden kann, bedeuten und
   - A: für einen geradkettigen oder verzweigten und/oder cyclischen Kohlenwasserstoffrest mit 1 bis 25 C-Atomen steht, der gesättigt oder ein- oder mehrfach ungesättigt und/oder teilweise aromatisch sein kann und gegebenenfalls eines oder mehrere gleiche oder verschiedene Heteroatome, ausgewählt unter O, S, NR¹¹, und/oder eine oder mehrere gleiche oder verschiedene funktionelle Gruppen, ausgewählt unter den funktionellen Gruppen C(O), S(O), S(O)₂, aufweisen kann und gegebenenfalls einen oder mehrere gleiche oder verschiedene Substituenten, ausgewählt unter der Substituenten C₁-C₆-Alkyl, C₁-C₆-Perfluoralkyl, C₁-C₆-Alkoxy, C₁-C₁₀-Acyloxy, C₇-C₁₂-Aralkyl, Halogen, - SiR^{5c}R^{6c}R^{7c}, gegebenenfalls substituiertes C₆-C₁₀-Aryl, substituiertes oder unsubstituiertes C₂-C₁₀-Hetaryl, NR^{8c}R^{9c}, SR^{10c}, NO₂, C₁-C₁₂-Acyl, C₁-C₁₀-Carboxyl tragen kann, oder
   für einen C₆-C₁₅-Arylrest oder einen C₂-C₁₅-Heteroarylrest steht, der gegebenenfalls jeweils 1 bis 5 Substituenten, ausgewählt unter C₁-C₆-Alkyl, Ci-C6-Perfluoralkyl, C₁-C₆-Alkoxy, C₇-C₁₂-Aralkyl, Halogen, SiR^{5d}R^{6d}R^{7d}, substituiertes oder unsubstituiertes C₆-C₁₀-Aryl, NR^{8d}R^{9d}, SR^{10d}, NO₂ tragen können, oder
   für eine funktionelle Gruppe oder ein Heteroatom ausgewählt aus der Gruppe -O-, -S-, -N(R¹¹)-, -S(O)-, -C(O)-, -S(O)₂-, -P(R¹¹)-, -(R¹¹)P(O)-und -Si(R¹²R¹³) steht,
   wobei die Reste R^{5a}, R^{6a}, R^{7a}, R^{8a}, R^{9a}, R^{10a} bis R^{5d}, R^{6d}, R^{7d}, R^{8d}, R^{9d}, R^{10d}, R¹¹, R¹² und R¹³ jeweils unabhängig voneinander ausgewählt sind unter C₁-C₆-Alkyl, C₇-C₁₂-Aralkyl und/oder substituiertes oder unsubstituiertes C₆-C₁₀-Aryl und wobei die Reste R^{8a} und R^{9a}, R^{8b} und R^{9b}, R^{8c} und R^{9c}, R^{8d} und R^{9d} unabhängig voneinander jeweils gemeinsam auch einen cyclischen Kohlenwasserstoffrest mit 2 bis 8 Kohlenstoffatomen bilden können, der eines oder mehrere gleiche oder verschiedene Heteroatome, ausgewählt aus der Gruppe O, S, NR^{11a}, aufweisen kann und R^{11a} die für R¹¹ angegebenen Bedeutungen haben kann,
   in freier und/oder komplexgebundener Form,
   bei dem man
   a) das Aluminium-haltige Reaktionsprodukt einer destillativen Auftrennung unter Erhalt eines an Isopulegol angereicherten Kopfprodukts und eines an Isopulegol abgereicherten Sumpfprodukts unterzieht,
   b) das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt bringt,
   c) den Liganden der Formel (I) aus der organischen Phase abtrennt.

Die nach dem erfindungsgemäßen Verfahren erhaltene Bis(diarylphenol)-Liganden der Formel (I) können üblicherweise ohne weitere Aufreinigungsschritte im Rahmen einer neuen Charge mit den entsprechenden Aluminiumverbindungen der Formeln (II) bzw. (III), wie im Folgenden definiert, zum reaktiven Katalysatorkomplex umgesetzt werden, wobei bei derartig wiederhergestellten Katalysatorkomplexen keine bzw. keine nennenswerte Abschwächung der Reaktivität festzustellen ist.

Die Bis(diarylphenol)-Liganden der Formel (I) weisen zwei Phenolsysteme auf, die jeweils in beiden ortho-Positionen zur phenolischen Hydroxy-Gruppe durch Aromaten bzw. Heteroaromaten (Ar¹ bis Ar⁴) substituiert sind und über ein Strukturelement A miteinander verknüpft sind und gegebenenfalls noch weitere Substituenten (R¹ bis R⁴) tragen können.

Die aromatischen bzw. heteroaromatischen Substituenten Ar¹ bis Ar⁴ können unabhängig voneinander gleich oder verschieden sein. Bevorzugt sind die beiden jeweils an ein Phenolsystem gebundenen Substituenten (Ar¹ und Ar² bzw. Ar³ und Ar⁴) paarweise gleich. Insbesondere bevorzugt sind alle vier Substituenten Ar¹ bis Ar⁴ gleich.

Die genannten Substituenten Ar¹ bis Ar⁴ sind Arylreste mit 6 bis 15, bevorzugt 6 bis 10 Kohlenstoffatomen oder Heteroarylreste mit 2 bis 15, bevorzugt 3 bis 10 Kohlenstoffatomen, im aromatischen Ringsystem. Arylreste mit 6 bis 15 Kohlenstoffatomen sind beispielsweise Phenyl, Naphthyl, Anthracenyl, bevorzugt Phenyl und Naphthyl.

Die genannten Heteroarylreste mit 2 bis 15 Kohlenstoffatomen weisen 1 bis etwa 6, in der Regel 1 bis 3, gleiche oder verschiedene Heteroatome, die ausgewählt sind aus der Gruppe der Heteroatome O, S und N, auf. Beispielhaft seien dafür die folgenden Heteroarylreste genannt: 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-lmidazolyl, 4-lmidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, Benzofuryl, Isobenzofuryl, Benzothienyl, Indolyl, Isoindolyl, Carbazolyl, Pyridyl, Chinolyl, Isochinolyl und Pyrazyl. Bevorzugte Heteroarylreste sind beispielsweise: 2-Furyl, 2-Pyridyl, 2-Imidazoyl.

Die vorstehend für Ar¹ bis Ar⁴ genannten Aryl bzw. Heteroarylreste können jeweils unabhängig voneinander unsubstituiert sein oder 1 bis etwa 7, bevorzugt 1 bis 3, insbesondere 1 oder 2, gleiche oder verschiedene Substituenten tragen, die ausgewählt sind aus der Gruppe der Substituenten: C₁-C₆-Alkyl, C₁-C₆-Perfluoralkyl, C₁-C₆-Alkoxy, C₇-C₁₂-Aralkyl, Halogen, -SiR^{5a}R^{6a}R^{7a}, substituiertes oder unsubstituiertes C₆-C₁₀-Aryl, -NR^{8a}R^{9a}, -SR^{10a}, -NO₂, wobei die Reste R^{5a}, R^{6a}, R^{7a}, R^{8a}, R^{9a}, R^{10a} und R¹¹ bis R¹³ jeweils unabhängig voneinander für C₁-C₆-Alkyl, C₇-C₁₂-Aralkyl und/oder substituiertes oder unsubstituiertes C₆-C₁₀-Aryl stehen, und die Reste R^{8a} und R^{9a} unabhängig voneinander jeweils gemeinsam auch einen cyclischen Kohlenwasserstoffrest mit 2 bis 8 Kohlenstoffatomen bilden können, der eines oder mehrere gleiche oder verschiedene Heteroatome, ausgewählt aus der Gruppe O, S und NR^{11a}, aufweisen kann und R^{11a} die für R¹¹ angegebenen Bedeutungen haben kann.

Dabei können den genannten Substituenten im Rahmen der gesamten vorliegenden Erfindung die nachfolgend beispielhaft genannten Bedeutungen zukommen:
C₁-C₆-Alkyl wie beispielsweise Methyl, Ethyl, Propyl, 1-Methylethyl, Butyl, 1-Methylpropyl, 2-Methylpropyl, 1,1-Dimethylethyl, Pentyl, Cyclopentyl, 1-Methylbutyl, 2-Methylbutyl, 3-Methylbutyl, 2,2-Di-methylpropyl, 1-Ethylpropyl, Hexyl, Cyclohexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
C₁-C₆-Perfluoralkyl wie beispielsweise Trifluormethyl, Pentafluorethyl, Heptafluorpropyl, Heptafluorisopropyl, Nonafluorbutyl;
C₁-C₆-Alkoxy wie beispielsweise Methoxy, Ethoxy, Propoxy, 1-Methylethoxy, Butoxy, 1-Methylpropoxy, 2-Methylpropoxy und 1,1-Dimethylethoxy, Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
C₇-C₁₂-Aralkyl wie beispielsweise Benzyl, 1-Phenylethyl, 2-Phenylethyl;
C₁-C₁₀-Acyloxy wie beispielsweise Acetyloxy, Propionyloxy;
C₁-C₁₀-Carboxyl wie beispielsweise Methoxycarbonyl, Ethoxycarbonyl, Propyloxycarbonyl, Isopropyloxycarbonyl;
C₁-C₁₀-Acyl wie beispielsweise Formyl, Acetyl, Propionyl.

Der Ausdruck "substituiertes oder unsubstituiertes C₆-C₁₀-Aryl" steht im Rahmen der vorliegenden Erfindung für Arylreste, die wie vorstehend genannt einen oder mehrere, in der Regel 1 bis etwa 3, gleiche oder verschiedene Substituenten aufweisen, wobei die Substituenten beispielsweise unter C₁-C₆-Alkyl, C₁-C₆-Perfluoralkyl, C₁-C₆-Alkoxy, C₇-C₁₂-Aralkyl, Halogen, Silyl, Dialkylamino und Nitro ausgewählt sein können.

Der Begriff "Halogen" steht im Rahmen der vorliegenden Erfindung für Fluor, Chlor, Brom und Jod, bevorzugt für Fluor und Chlor.

Unter den Substituenten -SiR^{5a}R^{6a}R^{7a} bis -SiR^{5d}R^{6d}R^{7d} sind im Rahmen der vorliegenden Erfindung jeweils Silylsubstituenten mit jeweils unabhängig voneinander drei gleichen oder verschiedenen Resten, die ausgewählt sind unter den Resten C₁-C₆-Alkyl, C₇-C₁₂-Aralkyl und substituiertes oder unsubstituiertes C₆-C₁₀-Aryl zu verstehen. Beispielhaft seien dafür etwa die Silylsubstituenten Trimethylsilyl, Triethylsilyl, tert-Butyl-dimethylsilyl und tert-Butyl-diphenylsilyl genannt.

Die Substituenten -NR^{8a}R^{9a} bis -NR^{8d}R^{9d} stehen im Rahmen der vorliegenden Erfindung jeweils für Amino-Substituenten, die jeweils unabhängig voneinander zwei gleiche oder verschiedene, bevorzugt zwei gleiche Rest tragen, die ausgewählt sind unter den vorstehend beschriebenen Resten C₁-C₆-Alkyl, C₇-C₁₂-Aralkyl und/oder substituiertes oder unsubstituiertes C₆-C₁₀-Aryl. Beispielhaft seien als Amino-Substituenten genannt: Dimethylamino, Diethylamino, Dibenzylamino, Diallylamino, Diisopropylamino. Die Reste R^{8a} und R^{9a} bis R^{8d} und R^{9d} können im Rahmen der vorliegenden Erfindung unabhängig voneinander jeweils gemeinsam auch einen cyclischen Kohlenwasserstoffrest mit 2 bis 8 Kohlenstoffatomen bilden, der eines oder mehrere gleiche oder verschieden Heteroatome, ausgewählt aus der Gruppe O, S, NR^{11a}, aufweisen kann. Der Rest R^{11a} kann dabei wie vorstehend beschriebenes C₁-C₆-Alkyl, C₇-C₁₂-Aralkyl und/oder substituiertes oder unsubstituiertes C₆-C₁₀-Aryl bedeuten. Beispielhaft seien für diese cyclischen Substituenten R^{8a} und R^{9a} bis R^{8d} und R^{9d} etwa genannt: Piperidinyl, Morpholinyl, N-Methylpiperazinyl, N-Benzylpiperazinyl. Bei den Substituenten -SR^{10a} steht der Rest R^{10a} für wie vorstehend definiertes C₁-C₆-Alkyl, C₇-C₁₂-Aralkyl und/oder substituiertes oder unsubstituiertes C₆-C₁₀-Aryl, bevorzugt für Methyl, Ethyl, Isopropyl, Phenyl, Benzyl.

Im Rahmen der vorliegenden Erfindung sind bevorzugte aromatische oder heteroaromatische Substituenten Ar¹, Ar², Ar³, Ar⁴ beispielsweise Phenyl, 4-Methylphenyl, 2,4,6-Trimethylphenyl, Naphthyl, 2-Fluorphenyl, 4-Fluorphenyl, 4-Chlorphenyl, 4-Bromphenyl, 3-Fluorphenyl, 3-Chlorphenyl, 3,5-Difluorphenyl, 3,5-Dichlorphenyl, 2,3,6-Trichlorphenyl, 2,4,6-Trichlorphenyl, 2-Methylphenyl, 4-Methylphenyl, 2,4,5-Trimethylphenyl, 2,4,6-Trimethylphenyl. 2-Isopropylphenyl, 4-Isopropylphenyl, 4-tert.-Butylphenyl, 4-n-Butylphenyl, 3-Trifluormethylphenyl, 4-Trifluormethylphenyl, 3,5-Bis(trifluormethyl)phenyl, 4-Arylphenyl, 3-Nitrophenyl, bevorzugt 4-Fluorphenyl, 4-Chlorphenyl, 3-Chlorphenyl, 3,5-Dichlorphenyl, 3-Trifluormethylphenyl, 4-Trifluormethylphenyl. Im Rahmen einer bevorzugten Ausführungsform sind die Reste Ar¹, Ar², Ar³, Ar⁴ gleich und bedeuten bevorzugt 4-Fluorphenyl, 4-Chlorphenyl, 3-Chlorphenyl, 3,5-Dichlorphenyl, 3-Trifluormethylphenyl, 4-Trifluormethylphenyl, insbesondere bevorzugt Phenyl.

Die zu den jeweiligen phenolischen Hydroxygruppen meta- oder para-ständigen Substituenten R¹, R², R³, R⁴ können erfindungsgemäß gleich oder verschieden, bevorzugt gleich, sein und jeweils unabhängig voneinander Wasserstoff und/oder wie vorstehend genanntes C₁-C₆-Alkyl, C₁-C₆-Perfluoralkyl, C₁-C₆-Alkoxy, C₇-C₁₂-Aralkyl, Halogen, -SiR^{5b}R^{6b}, R^{7b}, substituiertes oder unsubstituiertes C₆-C₁₀-Aryl, -NR^{8b}R^{9b}, -SR^{10b} und/oder -NO₂ bedeuten.

Als bevorzugte Reste R¹, R², R³, R⁴ seien genannt: Methyl, Ethyl, Isopropyl, Halogen, insbesondere Fluor und/oder Chlor, Trifluormethyl, Phenyl, Methoxy, Nitro. Bevorzugt sind die Reste R¹, R², R³, R⁴ gleich und bedeuten insbesondere bevorzugt Wasserstoff.

Die Reste R¹ oder R² und/oder R³ oder R⁴ können gemeinsam mit dem Strukturelement A auch einen cyclischen aromatischen oder nicht-aromatischen Cyclus bilden. In diesen Fällen weisen die erfindungsgemäß einzusetzenden Bis(diarylphenol)-Liganden der Formel (I) ein tricyclisches Grundgerüst, beispielweise ein Anthracen-Grundgerüst der Formel (X) oder Grundgerüste des Typs (XI), auf:

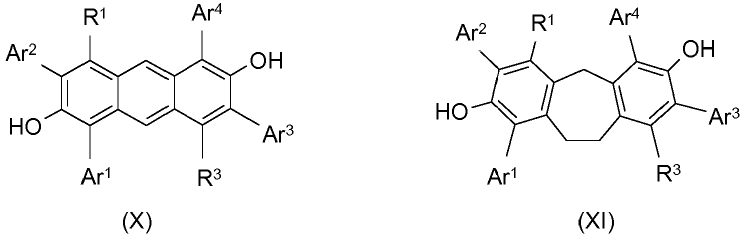

Weitere strukturelle Abwandlungen dieser tricyclischen Grundgerüste, gegebenenfalls auch solchen, die Heteroatome im Grundgerüst aufweisen, erschließen sich dem Fachmann und gehören zur Gruppe der erfindungsgemäß einsetzbaren Bis(diarylphenol)-Liganden.

Das Strukturelement A in Formel (I) kann für einen geradkettigen oder verzweigten und/oder cyclischen Kohlenwasserstoffrest mit 1 bis 25 Kohlenstoffatomen stehen, der gesättigt oder ein- oder mehrfach ungesättigt, normalerweise 1- bis etwa 6-fach ungesättigt sein kann und/oder teilweise aromatisch sein kann. Die genannten Kohlenwasserstoffreste können gegebenenfalls eines oder mehrere, in der Regel 1 bis 3, gleiche oder verschiedene Heteroatome, ausgewählt aus der Gruppe der Heteroatome O, S und NR¹¹ und/oder eine oder mehrere gleiche oder verschiedene funktionelle Gruppen, ausgewählt aus der Gruppe der funktionellen Gruppen C(O), S(O) und S(O)₂, aufweisen und gegebenenfalls einen oder mehrere gleiche oder verschiedene Substituenten, ausgewählt aus der Gruppe der Substituenten C₁-C₆-Alkyl, C₁-C₆-Perfluoralkyl, C₁-C₆-Alkoxy, C₁-C₁₀-Acyloxy, C₇-C₁₂-Aralkyl, Halogen, - SiR^{5c}R^{6c}R^{7c}, substituiertes oder unsubstituiertes C₆-C₁₆-Aryl, substituiertes oder unsubstituiertes C₂-C₁₀-Hetaryl, -NR^{8c}R^{9c}, -SR^{10c}, -NO₂, C₁-C₁₂-Acyl und C₁-C₁₀-Carboxyl tragen.

Bevorzugt steht das Strukturelement A in Formel (I) für einen geradkettigen oder verzweigten und/oder cyclischen Kohlenwasserstoffrest mit 1 bis 25, bevorzugt 1 bis 15, und besonders bevorzugt 1 bis 10 Kohlenstoffatomen , der gesättigt oder ein- bis dreifach ungesättigt sein kann und/oder teilweise aromatisch sein kann. Die bevorzugten Kohlenwasserstoffreste können gegebenenfalls eines oder mehrere, in der Regel 1 bis 3, gleiche oder verschiedene Heteroatome, ausgewählt aus der Gruppe der Heteroatome O, S und NR¹¹ und/oder eine oder mehrere C(O)-Gruppen aufweisen und gegebenenfalls einen oder mehrere gleiche oder verschiedene Substituenten, ausgewählt aus der Gruppe der Substituenten C₁-C₆-Alkyl, C₁-C₆-Perfluoralkyl, C₁-C₆-Alkoxy, C₁-C₁₀-Acyloxy, C₇-C₁₂-Aralkyl, Halogen, substituiertes oder unsubstituiertes C₆-C₁₀-Aryl, -NO₂, C₁-C₁₂-Acyl und C₁-C₁₀-Carboxyl tragen.

Als Beispiele für Strukturelemente A in der Formel (I) seien ohne jeden einschränkenden Charakter die folgenden Strukturelemente 1 bis 44 genannt, wobei die Schlangenlinien jeweils, wie im Rahmen der gesamten vorliegenden Offenbarung, die Anknüpfungsstellen zum Rest der jeweiligen Ligandenstruktur markieren:

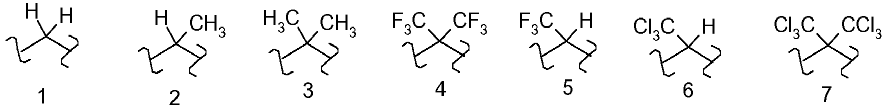

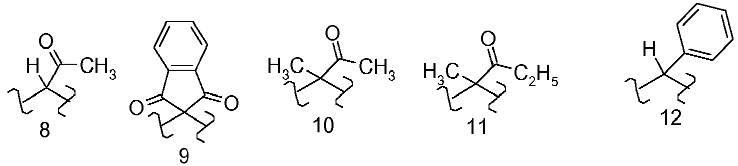

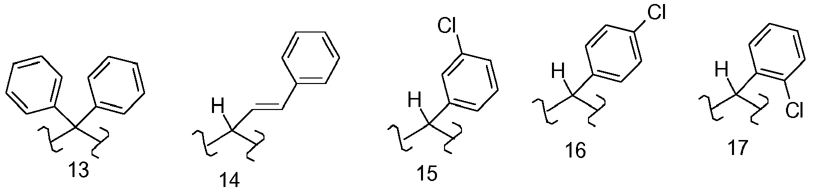

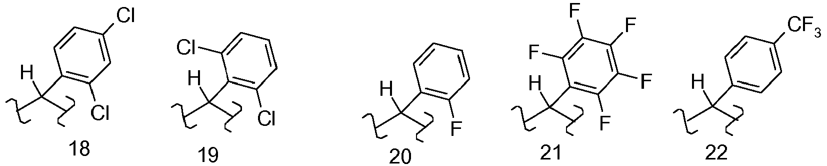

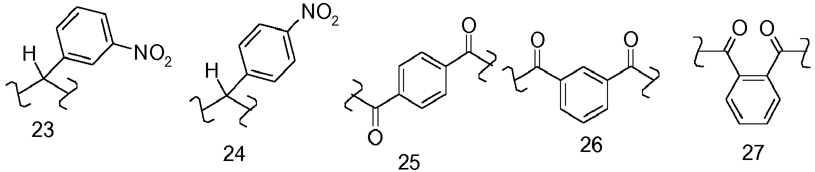

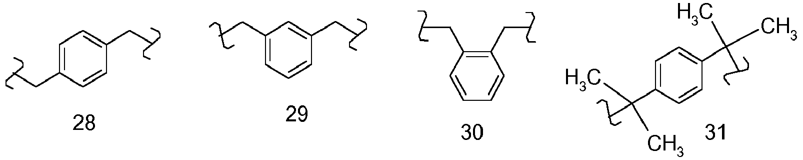

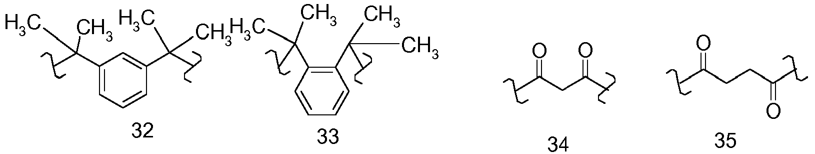

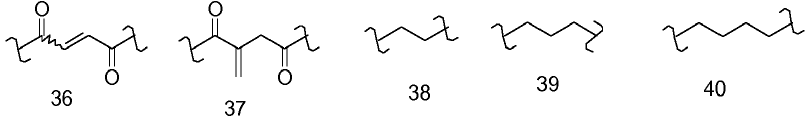

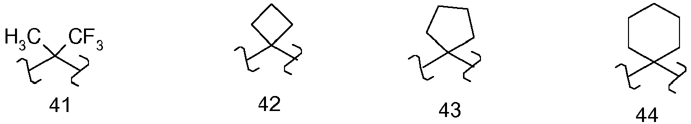

Auch die dargestellten Strukturelemente 1 bis 44 können jeweils die wie vorstehend bezeichneten Substituenten tragen und gegebenenfalls weitere, üblicherweise 1 oder 2 ethylenische Doppelbindungen aufweisen.

Das Strukturelement A kann auch für einen Arylrest mit 6 bis 15, bevorzugt 6 bis 10 Kohlenstoffatomen, speziell einen Phenylen-, Naphthylen- oder Anthracenylenrest stehen oder für einen wie vorstehend definierten Heteroarylrest mit 2 bis 15, bevorzugt 3 bis 10 Kohlenstoffatomen stehen.

Die genannten Aryl- bzw. Heteroarylreste können gegebenenfalls jeweils 1 bis 5 Substituenten tragen, die ausgewählt sind aus der Gruppe der wie vorstehend beschriebenen Substituenten C₁-C₆-Alkyl, C₁-C₆-Perfluoralkyl, C₁-C₆-Alkoxy, C₇-C₁₂-Aralkyl, Halogen, -SiR^{5d}R^{6d}, R^{7d}, substituiertes oder unsubstituiertes C₆-C₁₀-Aryl, - NR^{8d}R^{9d}, SR^{10d} und NO₂.

Des Weiteren kann das Strukturelement A auch für eine funktionelle Gruppe oder ein Heteroatom stehen, die ausgewählt sind aus der Gruppe -O-, -S-, -N(R¹¹)-, -S(O)-, -C(O)-, -S(O)₂-, -P(R¹¹)-, -(R¹¹)P(O)-, -OP(O)O-, -OP(O)₂O- und -Si(R¹²)(R¹³)-, wobei die Reste R¹¹, R¹², R¹³ unabhängig voneinander jeweils für wie vorstehend beschriebenes C₁-C₆-Alkyl, C₇-C₁₂-Aralkyl und/oder substituiertes oder unsubstituiertes C₆-C₁₀-Aryl stehen. Im Rahmen dieser Gruppe steht das Strukturelement A bevorzugt für -O-, -S-, -S(O)-, -S(O)₂- oder -Si(R¹²)(R¹³)-.

Im Rahmen der vorliegenden Erfindung umfasst der Begriff "Ligand in freier oder komplexgebundener Form" sowohl die freie Form des Liganden als auch sämtliche denkbaren Formen, die unter den Verfahrensbedingungen in die freie Form überführbar sind. Beispielhaft hierfür seinen Alkoholate des Liganden genannt, die durch die basische Hydrolyse in die freie Form des Liganden überführt werden.

Im Rahmen der vorliegenden Erfindung umfasst der Ausdruck "wässrige Base" allgemein wässrige Lösungen, deren pH-Wert größer als 7 ist. Insbesondere handelt es sich um wässrige Lösungen von Alkali- und Erdalkalimetallhydroxiden, speziell wässrige Lösungen von KOH und NaOH.

Der Ausdruck "Aluminium-haltiges Reaktionsprodukt" beschreibt im Rahmen der vorliegenden Erfindung ein Reaktionsprodukt, das wenigstens eine Verbindung umfasst, die Aluminium ionisch, kovalent oder komplexgebunden enthält. Dabei handelt es sich um Verbindungen des Aluminiums, wie sie unter den Bedingungen des erfindungsgemäßen Verfahrens aus den bei der Cyclisierung von Citronellal eingesetzten Verbindungen der Formel (R¹⁴)₃₋ₚAIHₚ (II) oder MAIH₄ (III), wie hierunter definiert, resultieren.

Unter dem Ausdruck "Hauptmenge" soll im Rahmen der vorliegenden Erfindung ein prozentualer Mengenanteil an der vorhandenen Gesamtmenge einer Verbindung verstanden werden, der größer 50 %, bevorzugt größer 80 % und insbesondere bevorzugt größer 90 % ist.

### Schritt a):

In Schritt a) des erfindungsgemäßen Verfahrens wird das Aluminium-haltige Reaktionsprodukt aus der Herstellung von Isopulegol durch Cyclisierung von Citronellal einer destillativen Auftrennung unter Erhalt eines an Isopulegol angereicherten Kopfprodukts und eines an Isopulegol abgereicherten Sumpfprodukts unterzogen.

In einer speziellen Ausführungsform wird in Schritt a) ein höher als das Isopulegol siedendes Lösungsmittel eingesetzt. Somit kann eine unerwünschte thermische Beanspruchung des Sumpfinhalts vermieden werden. Insbesondere liegen die darin enthaltenen Liganden der Formel (I) während der Abtrennung des Isopulegols nicht frei von Lösungsmittel vor. Das höhersiedende Lösungsmittel kann dem Aluminium-haltigen Reaktionsprodukt vor und/oder während der destillativen Auftrennung hinzugefügt werden. Vorzugsweise wird ein höhersiedendes Lösungsmittel eingesetzt, dessen Siedepunkt unter den Bedingungen der Destillation über dem Siedepunkt des Isopulegols liegt. Bevorzugt liegt der Siedepunkt des zugeführten Lösungsmittels unter den Bedingungen der Destillation wenigstens 5 °C, bevorzugt wenigstens 10 °C und insbesondere wenigstens 20 °C, über dem Siedepunkt des Isopulegols.

Bevorzugte höhersiedende Lösungsmittel, die einen solchen Siedepunkt aufweisen, sind beispielsweise Kohlenwasserstoffe, wie Phenylcyclohexan, Benzyltoluol, Dibenzyltoluol, 1-Methylnaphthalin und Tridecan, 1-Decanol, 1,2-Propylencarbonat, Ether, wie Diethylenglycoldibutylether, Tetraethylenglycoldimethylether und Dibenzylether, sowie technische Gemische dieser Lösungsmittel. Insbesondere bevorzugt sind Gemische, die als Hauptbestandteil Phenylcyclohexan enthalten.

Bei Einsatz wenigstens eines höhersiedenden Lösungsmittels wird als an Isopulegol abgereichertes Sumpfprodukt in Schritt a) eine organische Phase erhalten, umfassend das höhersiedende Lösungsmittel, die Hauptmenge der Liganden der Formel (I) sowie gegebenenfalls wenigstens eine Aluminium-haltige Verbindung.

Vorzugsweise erfolgt die destillative Abtrennung von Isopulegol in Schritt a) bei einer Sumpftemperatur von vorzugsweise höchstens 250 °C, bevorzugt höchstens 150 °C und besonders bevorzugt höchstens 100 °C. Die untere Sumpftemperatur ist in der Regel unkritisch und beträgt im Allgemeinen wenigstens 0 °C, vorzugsweise wenigstens 20 °C. Zur Einhaltung dieser Maximaltemperaturen kann die Destillation gewünschtenfalls unter einem geeigneten Vakuum durchgeführt werden.

Der Druck in Schritt a) der erfindungsgemäßen Verfahren liegt unabhängig von der speziellen Ausführungsform im Allgemeinen in einem Bereich von 0,1 bis 1500 mbar, bevorzugt in einem Bereich von 1 bis 500 mbar und besonderst bevorzugt in einem Bereich von 5 bis 100 mbar.

Unabhängig von der Zusammensetzung des Aluminium-haltigen Reaktionsprodukts aus der Cyclisierung von Citronellal und vom Einsatz eines höhersiedenden Lösungsmittels kann die destillative Abtrennung des Isopulegols kontinuierlich oder diskontinuierlich, vorzugsweise kontinuierlich erfolgen. In einer geeigneten Vorgehensweise wird dem Reaktionsprodukt aus der Cyclisierung von Citronellal das höhersiedende Lösungsmittel vor der destillativen Auftrennung zugegeben und im Verlauf der Destillation die im Sumpf vorhandene Menge an hochsiedendem Lösungsmittel im Weiteren konstant gehalten.

Zur destillativen Auftrennung in Schritt a) können die üblichen dem Fachmann bekannten Vorrichtungen eingesetzt werden (siehe z. B. Sattler, Thermische Trennverfahren, 2. Auflage 1995, Weinheim, S. 135ff; Perry's Chemical Engineers Handbook, 7. Auflage 1997, New York, Section 13). Dazu zählen Destillationssäulen und -kolonnen, die mit Packungen, Einbauten etc. versehen sein können. Die eingesetzten Destillationssäulen können trennwirksame Einbauten enthalten, wie Trennböden, z. B. Lochböden, Glockenböden oder Ventilböden, geordnete Packungen, z. B. Blech- oder Gewebepackungen, oder regellose Schüttungen von Füllkörpern. Die in der/den eingesetzten Kolonne(n) notwendige Stufenzahl und das Rücklaufverhältnis richten sich im Wesentlichen nach den Reinheitsanforderungen und der relativen Siedelage der Bestandteile des Aluminium-haltigen Reaktionsprodukts aus der Herstellung von Isopulegol durch Cyclisierung von Citronellal und des höhersiedenden Lösungsmittels, wobei der Fachmann die konkreten Auslegungs- und Betriebsdaten nach bekannten Methoden ermitteln kann. Die destillative Auftrennung kann z. B. in einer oder mehreren miteinander gekoppelten Destillationskolonnen erfolgen.

Zur destillativen Auftrennung in Schritt a) eignen sich ebenfalls übliche Verdampfer, vorzugsweise Verdampfer mit Zwangsumlauf, besonders bevorzugt Fallfilmverdampfer.

In Abhängigkeit gegebenenfalls enthaltener zusätzlicher Komponenten im Aluminium-haltigen Reaktionsprodukt aus der Cyclisierung von Citronellal kann die Zusammensetzung des bei der destillativen Auftrennung erhaltenen Kopfprodukts es erforderlich machen, dieses gegebenenfalls einem weiteren Aufarbeitungsschritt zu unterziehen.

In einer speziellen Ausführungsform des erfindungsgemäßen Verfahrens zur Aufbereitung eines Aluminium-haltigen Reaktionsprodukts aus der Herstellung von Isopulegol durch Cyclisierung von Citronellal, enthält das Reaktionsprodukt zusätzlich ein niedriger siedendes Lösungsmittel (iii).

Der Ausdruck "niedriger siedendes Lösungsmittel (iii)" bezieht sich im Rahmen der vorliegenden Erfindung auf den Siedepunkt des Isopulegols. Insbesondere eignen sich hierfür solche Lösungsmittel oder Lösungsmittelgemische, die unter den Bedingungen der destillativen Auftrennung einen Siedepunkt aufweisen, der wenigstens 5 °C, bevorzugt 10 °C und insbesondere 20 °C, unter dem des Isopulegols bei den jeweiligen Bedingungen liegt.

Bevorzugte Lösemittel mit einem solchen Siedepunkt sind im Rahmen der vorliegenden Erfindung inerte organische Lösungsmittel oder Mischungen davon, wie beispielsweise aromatische Lösungsmittel, z. B. Toluol, Ethylbenzol oder Xylol, halogenierte Lösungsmittel, z. B. Dichlormethan , Dichlorethan oder Chlorbenzol, aliphatische Lösungsmittel, z. B. Pentan, Hexan oder Cyclohexan, Ether, z. B. Tetrahydrofuran, Diethylether, Methyl-tert-Butylether, Ester, z. B. Essigsäureethylester, oder Dimethylformamid (DMF), Dimethylsulfoxid (DMSO) und dergleichen mehr. Besonderst bevorzugt handelt es sich um Toluol.

Enthält das aufzuarbeitende Aluminium-haltige Reaktionsprodukt ein solches niedriger siedendes Lösungsmittel, so wird dieses in einer geeigneten Ausführungsform vor der destillativen Abtrennung des Isopulegols zumindest teilweise aus dem Reaktionsprodukt entfernt. Die Abtrennung des niedriger siedenden Lösungsmittels erfolgt vorzugsweise ebenfalls destillativ. In Abhängigkeit vom Siedepunkt des niedriger siedenden Lösungsmittels können die üblichen zuvor genannten Destillationsvorrichtungen eingesetzt werden.

In einer weiteren geeigneten Ausführungsform erfolgt die destillative Auftrennung des Aluminium-haltigen Reaktionsprodukts in Schritt a) unter Erhalt eines an Isopulegol angereicherten Kopfprodukts, das gleichzeitig zumindest einen Teil, vorzugsweise die Hauptmenge des niedriger siedenden Lösungsmittels, enthält. In diesem Fall kann das Kopfprodukt einer weiteren Auftrennung, bevorzugt ebenfalls destillativ, unterzogen werden.

Das abgetrennte niedriger siedende Lösungsmittel wird mit Vorteil in die Cyclisierung des Citronellals zurückgeführt, in dem es als Lösungsmittel eingesetzt wird. Auf diese Weise erfordert das erfindungsgemäße Verfahren - bis auf Ergänzungen, die durch unvermeidliche Verluste erforderlich werden - die lediglich einmalige Bereitstellung einer Menge des niedriger siedenden Lösungsmittels.

In einer speziellen Ausführungsform des erfindungsgemäßen Verfahrens zur Aufbereitung eines Aluminium-haltigen Reaktionsprodukts aus der Herstellung von Isopulegol durch Cyclisierung von Citronellal, enthält das Reaktionsprodukt zusätzlich einen Hilfsstoff (iv).

Der Begriff "Hilfsstoff (iv)" bezieht sich im Rahmen der vorliegenden Erfindung auf Verbindungen, die bei der Cyclisierung von Citronellal zugesetzt werden, um unerwünschte Nebenreaktionen zu unterdrücken. Bevorzugt sind die Hilfsstoffe (iv) ausgewählt unter organischen Säuren, Carbonsäureanhydriden, Aldehyden, Ketonen und Vinylethern.

Speziell sind die Hilfsstoffe (iv) ausgewählt unter Säuren, vorzugsweise organische Säuren. Beispielhaft seien als organische Säuren genannt: Essigsäure, Propionsäure, Benzoesäure, Toluolsulfonsäure, Methansulfonsäure, bevorzugt Essigsäure.

In einer weiteren speziellen Ausführungsform der vorliegenden Erfindung sind die Hilfsstoffe (iv) ausgewählt unter Carbonsäureanhydriden, Aldehyden, Ketonen und Vinylethern.

Die Hilfsstoffe (iv) der genannten Substanzklassen können jeweils einzeln oder in Form von Gemischen in dem aufzuarbeitenden Reaktionsprodukt vorliegen. Bevorzugte Gemischen sind solche, die aus Verbindungen einer Substanzklasse bestehen. Besonders bevorzugt enthält das Reaktionsprodukt einen einzelnen Hilfsstoff.

Vorzugsweise werden die im Reaktionsprodukt aus der Cyclisierung von Citronellal enthaltenen Hilfsstoffe (iv) ebenfalls zumindest teilweise entfernt und soweit wie möglich in die Cyclisierung von Citronellal zurückgeführt.

Weisen die Hilfsstoffe (iv) unter den Bedingungen der Destillation einen Siedepunkt auf, der unterhalb oder nur geringfügig, d. h. weniger als 30 °C, über dem Siedepunkt des Isopulegols liegt, können diese durch Destillation aus der ausreagierten Mischung weitgehend und in dem Maße, in dem sie nicht gegebenenfalls selbst umgesetzt wurde, zurück gewonnen werden. In Abhängigkeit vom Siedepunkt des Hilfsstoffs können die üblichen zuvor genannten Destillationsvorrichtungen eingesetzt werden.

Weisen die Hilfsstoffe (iv) unter den Bedingungen der Destillation einen Siedepunkt auf, der deutlich oberhalb, d. h. wenigstens 30 °C, über dem Siedepunkt des Isopulegols liegt, verbleiben diese im Sumpfprodukt und werden gegebenenfalls in Schritt b) des erfindungsgemäßen Verfahrens entfernt, wenn Ihre physikalischen Eigenschaften dies erlauben.

In einer weiteren geeigneten Ausführungsform erfolgt die destillative Auftrennung des Reaktionsprodukts in Schritt a) unter Erhalt eines an Isopulegol angereicherten Kopfprodukts, das gleichzeitig zumindest einen Teil, vorzugsweise die Hauptmenge des Hilfsstoffs (iv), enthält. Gegebenenfalls kann dieses Hauptprodukt ein niedriger siedendes Lösungsmittel, wie zuvor ausgeführt, enthalten. In diesem Fall kann das Kopfprodukt einer weiteren Auftrennung, bevorzugt ebenfalls destillativ, unterzogen werden. Der abgetrennte Hilfsstoff (iv) wird, gegebenenfalls gemeinsam mit dem niedriger siedenden Lösungsmittel, mit Vorteil in die Cyclisierung des Citronellals zurückgeführt, in der er z. B. zur Unterdrückung unerwünschter Nebenreaktionen eingesetzt wird. Auf diese Weise erfordert das erfindungsgemäße Verfahren - bis auf Ergänzungen, die durch unvermeidliche Verluste erforderlich werden - die lediglich einmalige Bereitstellung einer Menge des Hilfsstoffs (iv).

Die Isopulegolabtrennung, die Zufuhr des höhersiedenden Lösungsmittels und gegebenenfalls die Leichtsiederabtrennung, d. h. die Abtrennung von gegebenenfalls vorhandenen Lösungsmitteln und flüchtigen Hilfsstoffen aus der Cyclisierung von Citronellal, können auf unterschiedliche Weisen kombiniert werden:
In einer geeigneten Ausführungsform verwendet man zur Destillation eine so genannte Trennwandkolonne, d. h. Zulaufstelle und ein Seitenabzug befinden sich auf entgegen gesetzten Seiten einer Trennwand, die sich über einen Abschnitt der Längsausdehnung der Kolonne erstreckt. Derartige Destillationskolonnen, die eine Trennwand enthalten, sind dem Fachmann an sich bekannt. Sofern sich Seitenabzug und Zulauf im Bereich der Trennwand befinden, entsteht eine zu einer Brugma- oder Petlyuk-Schaltung analoge Schaltung. Derartige Destillationen unter Verwendung von Trennwandkolonnen sind in der DE-A-33 02 525 und EP-A-0 804 951 beschrieben. In diesem Fall kann z. B. als Kopfprodukt eine an Leichtsiedern angereicherte Fraktion und als Seitenabzug einen den Hauptteil an Isopulegol enthaltenden Strom abgezogen werden. Das höhersiedende Lösungsmittel wird unterhalb der Zulaufstelle, bevorzugt in den Sumpf der Kolonne oder kurz oberhalb des Sumpfes, zugefahren. Eine Lösung der Hauptmenge des Liganden der Formel (I) in dem höhersiedenden Lösungsmittel fällt als Sumpfprodukt an.

In einer alternativen Ausführungsform verwendet man zur Destillation gekoppelte Kolonnen. Diese Ausführungsform kann von Vorteil sein, wenn das Reaktionsprodukt der Cyclisierung von Citronellal ein Lösungsmittel und/oder einen flüchtigen Hilfsstoff enthält, wie im Folgenden näher ausgeführt.

In diesem Fall können Gemische aus Isopulegol und niedriger oder geringfügig höhersiedenden Lösungsmitteln und/oder Hilfsstoff (iv) das Kopfprodukt der ersten Kolonne bilden und in der zweiten Kolonne einer Auftrennung unter Erhalt eines zumindest die Hauptmenge des Isopulegols enthaltenden Stroms und eines an Isopulegol abgereicherten, die niedriger siedenden Lösungsmittel und/oder Hilfsstoffe der Cyclisierung enthaltenden Stroms, unterzogen werden.

Ströme, die niedriger siedende Lösungsmittel (iii) und Hilfsstoff (iv) der Cyclisierung enthalten, können in der Regel ohne weitere Auftrennung in die Cyclisierung zurückgeführt werden.

Die Liganden der Formel (I) fallen, gegebenenfalls in Form ihrer Komplexe oder anderer Derivate, als Sumpfprodukt der ersten Kolonne an.

### Schritt b):

In Schritt b) des erfindungsgemäßen Verfahrens wird das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt gebracht. Bevorzugte wässrige Basen sind die zuvor genannten.

Das in Schritt a) erhaltene, an Isopulegol abgereicherte Sumpfprodukt kann neben dem Liganden der Formel (I) in freier oder komplexgebundener Form wenigstens eine weitere schwerflüchtige Komponente enthalten. Dazu zählen z. B. in Schritt a) zugesetzte höhersiedende Lösungsmittel, die Umsetzungsprodukte der zur Cyclisierung von Citronellal zu Isopulegol eingesetzten Aluminium-haltigen Verbindungen sowie gegebenenfalls in Schritt a) nicht abgetrennte Hilfsstoffe (iv). Da sich Aluminium-haltige Komponenten und/oder die Hilfsstoffe (iv) insbesondere bei einem kontinuierlichen Verfahren anreichern und sich speziell auf die Ausbeute und Reinheit des Abtrennens in Schritt c) negativ auswirken, ist es vorteilhaft, diese Verbindungen möglichst vollständig zu entfernen. Dies gilt speziell für die Aluminium-haltigen Verbindungen.

Das Inkontaktbringen in Schritt b) erfolgt vorzugsweise durch Extraktion. Die Zahl der Extraktionsstufen liegt vorzugsweise in einem Bereich von 1 bis 20 Stufen.

Als Extraktionsmittel dienen die zuvor genannten wässrigen Basen. Daher werden diese Ausdrücke im Rahmen der vorliegenden Erfindung synonym verwendet.

Zur Extraktion bringt man das an Isopulegol abgereicherte Sumpfprodukt aus Schritt a) mit einer wässrigen Base innig in Kontakt. Nach Trennung der Phasen erhält man eine die Hauptmenge des Liganden der Formel (I) enthaltende Phase und eine an Aluminium-haltigen Verbindungen angereicherte wässrige Phase. Anschließend entfernt man die wässrige Phase. Das Inkontaktbringen kann kontinuierlich oder diskontinuierlich erfolgen.

Zur diskontinuierlichen Durchführung bringt man unter mechanischer Bewegung, z. B. durch Rühren, das an Isopulegol abgereicherte Sumpfprodukt aus Schritt a) und das wässrige Extraktionsmittel in einem geeigneten Gefäß in Kontakt, lässt das Gemisch zur Phasentrennung ruhen und entfernt eine der Phasen, indem man zweckmäßigerweise die dichtere Phase am Boden des Gefäßes abzieht.

Mehrere diskontinuierliche Trennoperationen können kaskadenartig hintereinander durchgeführt werden, wobei die von der wässrigen Phase abgetrennte, die Hauptmenge des Liganden der Formel (I) enthaltende Phase jeweils mit einer frischen Portion des wässrigen Extraktionsmittels in Kontakt gebracht wird und/oder das wässrige Extraktionsmittel im Gegenstrom geführt wird.

Vorzugsweise erfolgt die Extraktion kontinuierlich. Zur kontinuierlichen Durchführung der Extraktion führt man das wässrige Extraktionsmittel und den Strom des an Isopulegol abgereicherten Sumpfprodukts aus Schritt a) geeigneten Apparaturen in analoger Weise zur diskontinuierlichen Variante kontinuierlich zu. Gleichzeitig wird der Apparatur, in der die Trennung der Phasen stattfindet, ein Austrag der die Hauptmenge des Liganden der Formel (I) enthaltende Phase und ein Austrag der an Aluminium-haltigen Verbindungen angereicherte wässrige Phase kontinuierlich entnommen.

Die Extraktion erfolgt mindestens einstufig, z. B. in einer Mischer-Abscheider-Kombination. Geeignete Mischer sind sowohl dynamische als auch statische Mischer. Eine Extraktion in mehreren Stufen erfolgt beispielsweise in mehreren Mischer-Abscheidern oder Extraktionskolonnen.

In einer geeigneten Ausführungsform wird zur Verbesserung der Phasentrennung wenigstens eine Koalesziervorrichtung eingesetzt. Diese ist vorzugsweise ausgewählt unter Koaleszierfiltern, Elektrokoaleszern und Kombinationen davon. Beim Einsatz von Mischer-Abscheider-Vorrichtungen zur Extraktion hat sich der Einsatz von Koaleszierfiltern, wie Kerzen- oder Sandfiltern, als vorteilhaft zur Verbesserung der Phasentrennung herausgestellt. Der Filter kann dabei direkt nach dem Mischer (Rührbehälter) und/oder im organischen Ablauf des Abscheiders installiert werden. Des Weiteren bevorzugt zur Verbesserung der Phasentrennung ist der Einsatz von Elektrokoaleszern. Diese haben sich zur Abtrennung wässriger Fremdphasen von bis zu 5 Massen-% bewährt. Der Einsatz von Koalesziervorrichtungen eignet sich in dem erfindungsgemäßen Verfahren auch vorteilhaft zur Abscheidung von feindispergierter wässriger Phase aus dem die Hauptmenge des Liganden der Formel (I) enthaltenden organischen Austrag einer Extraktionskolonne.

In einer geeigneten Ausgestaltung erfolgt die Extraktion in wenigstens einer Mischer-Abscheider-Kombination für die Extraktion Aluminium-haltiger Komponenten aus dem an Isopulegol abgereicherten Sumpfprodukt aus Schritt a). Die Verwendung einer weiteren Mischer-Abscheider-Kombination ist insbesondere vorteilhaft, um Anteile des Liganden der Formel (I) oder gegebenenfalls des höhersiedenden Lösungsmittels, die gegebenenfalls mit den abzutrennenden Aluminium-haltigen Verbindungen teilweise in das Extraktionsmittel übergehen, nachträglich zu re-extrahieren und somit in das Verfahren zurückzuführen.

Bevorzugt wird die Extraktion kontinuierlich in zwei hintereinander geschalteten, beheizbaren Mischern durchgeführt, wobei die wässrige Base mit dem an Isopulegol abgereicherten Sumpfprodukt aus Schritt a) in den ersten Rührapparat eingeleitet wird und die so entstandene Mischung in einen zweiten Rührapparat überführt wird. Aus diesem zweiten Rührapparat wird die Mischung dann in einen Abscheider eingeleitet, in dem die Phasentrennung in eine schwerere wässrige und eine leichtere organische Phase erfolgt. Durch diese Kaskadierung der Mischer wird eine vollständigere Hydrolyse bzw. Extraktion der Aluminium-haltigen Verbindungen erreicht. Als Rührapparate kommen dem Fachmann bekannte mit Rührwerken ausgestattete und mit Dampf bzw. Warmwasser beheizbare Behälter (Rührkessel) zum Einsatz. Als Phasentrennbehälter wird vorteilhaft ein liegend installierter, ebenfalls beheizbarer Behälter eingesetzt, der so temperiert wird, dass sich keine Feststoffe aus den einzelnen Phasen abscheiden können.

Unter bestimmten Umständen kann es vorteilhaft sein, die die Hauptmenge an Liganden der Formel (I) enthaltende organischen Phase vor dem Abtrennen des Liganden in Schritt c) oder im Anschluss an das Abtrennen einem Trocknungsschritt zu unterziehen. Geeignete Trocknungsverfahren sind die üblichen, dem Fachmann bekannten, insbesondere die Adsorption an wasserentziehenden Mitteln, z. B. unter Verwendung eines zeolithischen Molekularsiebs.

In einer alternativen Ausführungsform des erfindungsgemäßen Verfahrens wird, nach dem Inkontaktbringen des an Isopulegol abgereicherten Sumpfprodukts mit der wässrigen Base, das Wasser vollständig oder wenigstens teilweise destillativ entfernt.

Um ein frühzeitiges Abtrennen, speziell durch Kristallisation, des Liganden der Formel (I) zu verhindern, sollte zu keinem Zeitpunkt während Schritt b) die Konzentration des Liganden in der organischen Phase dessen Löslichkeit überschreiten. Dies kann durch geeignete Wahl der Temperatur und/oder der Menge und Art gegebenenfalls zugegebener Lösungsmittel erfolgen.

Demzufolge wird in einer bevorzugten Ausführungsform des erfindungsgemäßen Verfahrens ein Austrag des erwärmten Sumpfprodukts aus Schritt a) mit einer erwärmten wässrigen Base in innigen Kontakt gebracht.

Der Ausdruck "erwärmt" bezeichnet im Rahmen der vorliegenden Erfindung eine Temperatur oberhalb der Raumtemperatur und unterhalb der jeweiligen Siedepunktstemperaturen der wässrigen oder organischen Lösungen bei den jeweiligen Reaktionsbedingungen. Insbesondere bezeichnet erwärmt eine Temperatur im Bereich von 25 °C bis 150 °C, speziell im Bereich von 70 °C bis 100 °C.

In Abhängigkeit der gegebenenfalls bei der Cyclisierung von Citronellal verwendeten Hilfsstoffe kann das an Isopulegol abgereicherte Sumpfprodukt gegebenenfalls weitere in Schritt a) nicht abgetrennte Komponenten enthalten. Diese werden vorzugsweise in Schritt b) abgetrennt. In diesem Fall kann man die erhaltene wässrige Phase einem geeigneten Trennverfahren unterziehen, um diese Komponenten, z. B. einen Hilfsstoff (iv), zurückzugewinnen.

### Schritt c):

In Schritt c) des erfindungsgemäßen Verfahrens wird der vorstehend beschriebene Ligand der Formel (I) aus der in Schritt b) erhaltenen die Hauptmenge des Liganden enthaltenden organischen Phase durch das vorstehend beschriebene Verfahren zum Abtrennen eines Stoffes aus einer Lösung aus der organischen Phase abgetrennt. Wie vorstehend beschrieben, wird bei diesem Verfahren elektromagnetische Strahlung in die Lösung eingestrahlt und die Intensität der elektromagnetischen Strahlung detektiert, die von den in der Lösung befindlichen Kristallen gestreut wurde. In diesem Fall handelt es sich um Kristalle des Liganden. Die detektierte Intensität wird dann mit der Soll-Intensität verglichen und die Temperatur der Lösung wird in Abhängigkeit von der Differenz der detektierten Intensität und der Soll-Intensität so geregelt, dass sich diese Differenz verkleinert. Wenn schließlich der Betrag der Differenz der detektierten Intensität und der Soll-Intensität kleiner als der Grenzwert ist, wird das Kristallisationsverfahren, insbesondere die Kühlkristallisation, begonnen. Die gewonnenen Kristalle des Liganden werden anschließend abgetrennt.

Bei dem erfindungsgemäßen Verfahren wird insbesondere der folgende Aluminium-Phenolat Katalysator mit einem sterisch sehr anspruchsvollen Liganden eingesetzt:

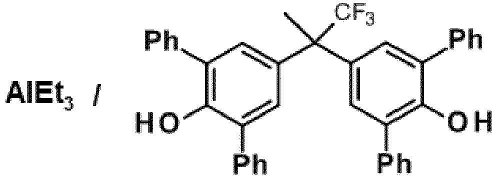

Bei der Rückgewinnung dieses Liganden durch Kühlungskristallisation aus einer Lösung in Phenylcyclohexan geht aufgrund der komplexen Struktur des Moleküls die Kristallbildung nur vergleichsweise langsam vonstatten. Die Unterkühlbarkeit der Kristallisationslösung kann bei diesem Liganden bis zu 50 K betragen. Es ist somit besonders schwer, zu einer Kristallisation des Liganden in einer gut filtrierbaren Kristallgröße und -morphologie zu gelangen. Maßstab hierfür ist die Erreichung eines Filterwiderstands, der für ein gut filtrierendes Produkt bei 5*10¹³ mPasm⁻² liegt. Wird die Lösung zu stark oder zu gering angeimpft ändert sich der Filterwiderstand um mehr als eine Größenordnung auf über 10¹⁵ mPasm⁻².

Wenn der vorstehende beschriebene Ligand aus der Lösung abgetrennt werden soll, wird die Lösung oder ein Teil der Lösung in einem Kristallisationsgefäß bevorzugt auf eine Temperatur gebracht, die geringer als 95 °C, insbesondere geringer als 90 °C ist. Die Temperatur wird dann erhöht, bis sich die detektierte Intensität der gestreuten elektromagnetischen Strahlung wie beschrieben der Soll-Intensität angenähert hat. Danach wird die Temperatur für die Kühlkristallisation wieder gesenkt. Die Abkühlrate ist zu Beginn in einem Bereich von 1 K/h bis 5 K/h.

Durch das erfindungsgemäße Verfahren wird eine genau bemessene Impfkristallmenge zu Beginn des Kristallisationsverfahrens bereitgestellt. Hierdurch wird erreicht, dass trotz der komplexen Molekülstruktur des Liganden, dieser in kurzer Zeit mit hoher Ausbeute zurückgewonnen werden kann.

Bei diesem Kristallisationsverfahren kann es sich neben dem beschriebenen Kühlkristallisationsverfahren im Übrigen auch um ein Verdampfungskristallisationsverfahren, ein Vakuumkristallisationsverfahren und ein Verfahren handeln, das Kristallisierrinnen oder Sprühkristallisatoren einsetzt.

Im Allgemeinen erfolgt die Kristallisation bei einer Temperatur im Bereich von -50 °C bis 150 °C, bevorzugt im Bereich von 0 °C bis 120 °C und speziell in einem Bereich von 30 °C bis 110 °C.

Der kristalline Ligand der Formel (I) kann beispielsweise durch Filtration, Flotation, Zentrifugation oder Siebung aus der Lösung isoliert werden.

Der so zurückerhaltene Ligand der Formel (I) kann gegebenenfalls durch geeignete Trocknungsverfahren getrocknet werden. Verfahren hierfür sind dem Fachmann bekannt. Beispielsweise sind zur technischen Ausgestaltung des Verfahrens übliche Walzentrockner, Tellertrockner, Kammertrockner, Fließbetttrockner oder Strahlungstrockner geeignet.

Die an Ligand der Formel (I) abgereicherte organische Phase kann dem Verfahren erneut vor oder während Schritt a) zugeführt werden.

In einer bevorzugten Ausführungsform des Verfahrens zur Aufarbeitung eines Reaktionsprodukts aus der Herstellung von Isopulegol ist der Ligand der Formel (I) ausgewählt unter Bis(diarylphenol)-Liganden der Formel (I.a) 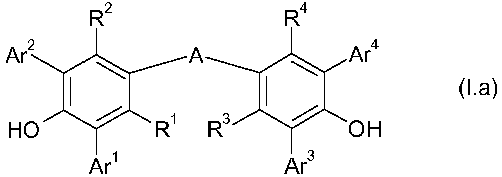 wobei Ar¹, Ar², Ar³, Ar⁴, R¹, R², R³, R⁴ und A die zuvor gegebenen Bedeutungen besitzen.

Die Liganden der Formel (I.a) weisen ebenfalls zwei Phenolsysteme auf, die jeweils in beiden ortho-Positionen zur phenolischen Hydroxy-Gruppe durch Aromaten bzw. Heteroaromaten (Ar¹ bis Ar⁴) substituiert sind und über ein Strukturelement A miteinander verknüpft sind und gegebenenfalls noch weitere Substituenten (R¹ bis R⁴) tragen können, wobei das Strukturelement A jeweils in para-Position zur phenolischen Hydroxy-Gruppe mit den beiden Phenolsystemen verknüpft ist. Dabei können den Resten Ar¹, Ar², Ar³, Ar⁴, den Resten R¹, R², R³, R⁴ und dem Strukturelement A die gleichen Bedeutungen zukommen wie vorstehend für Formel (I) genannt.

Insbesondere bevorzugte Liganden sind erfindungsgemäß solche, bei denen die Arylreste Ar¹, Ar², Ar³ und Ar⁴ gleich sind und die vorstehend für Formel (I) angegebenen bevorzugten Bedeutungen besitzen. Insbesondere bevorzugt als Arylreste Ar¹ bis Ar⁴ sind Phenyl, Naphthyl, 4-Fluorphenyl, 4-Chlorphenyl, 3-Chlorphenyl, 3,5-Dichlorphenyl, 4-Methylphenyl, 3-Trifluormethylphenyl, 4-Trifluormethylphenyl, ganz besonders bevorzugt Phenyl.

Bei den erfindungsgemäß bevorzugten Liganden der Formel (I.a) sind die Reste R¹, R², R³, R⁴ gleich oder verschieden, bevorzugt gleich, und bedeuten vorzugsweise: Wasserstoff, Halogen, insbesondere Fluor oder Chlor, Methyl, Trifluormethyl, Isopropyl, tert-Butyl, Phenyl, Nitro.

Dem Strukturelement A in Formel (I.a) kommen die vorstehend für Formel (I) genannten Bedeutungen zu. Bevorzugte Strukturelemente A in Formel (I.a) sind insbesondere auch die Strukturelemente 1 bis 44, die in der genannten Weise substituiert sein können.

Insbesondere bevorzugte Liganden sind solche der Formeln (I.a₁) bis (I.a₃), wobei den genannten Resten Ar¹ bis Ar⁴, R¹ bis R⁴ und R¹⁵ bis R¹⁸ bevorzugt die tabellarisch beispielhaft aufgeführten Bedeutungen zukommen:

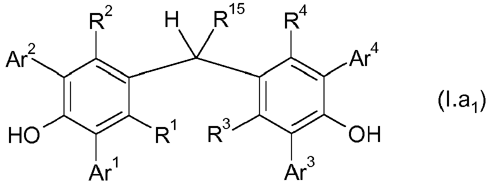

**Tabelle 1:**

| Verbindung | Ar¹ | Ar² | Ar³ | Ar⁴ | R¹ | R² | R³ | R⁴ | R¹⁵ |
|---|---|---|---|---|---|---|---|---|---|
| Ia₁-1 | Ph | Ph | Ph | Ph | H | H | H | H | H |
| Ia₁-2 | Ph | Ph | Ph | Ph | H | H | H | H | CH₃ |
| Ia₁-3 | Ph | Ph | Ph | Ph | H | H | H | H | Ph |
| Ia₁-4 | Ph | Ph | Ph | Ph | H | H | H | H | CF₃ |
| Ia₁-5 | Ph | Ph | Ph | Ph | H | H | H | H | CCl₃ |
| Ia₁-6 | Ph | Ph | Ph | Ph | H | H | H | H | 4-Cl-Ph |
| Ia₁-7 | Ph | Ph | Ph | Ph | H | H | H | H | CH₂CH₃ |
| Ia₁-8 | Ph | Ph | Ph | Ph | H | H | H | H | 3-NO₂-Ph |
| Ia₁-9 | Ph | Ph | Ph | Ph | H | H | H | H | 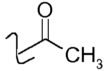 |

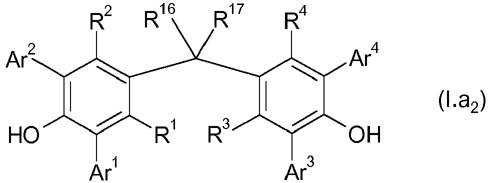

**Tabelle 2:**

| Verbindung | Ar¹ | Ar² | Ar³ | Ar⁴ | R¹ | R² | R³ | R⁴ | R¹⁶ | R¹⁷ |
|---|---|---|---|---|---|---|---|---|---|---|
| Ia₂-1 | Ph | Ph | Ph | Ph | H | H | H | H | CF₃ | CF₃ |
| Ia₂-2 | Ph | Ph | Ph | Ph | H | H | H | H | CCl₃ | CCl₃ |
| Ia₂-3 | Ph | Ph | Ph | Ph | H | H | H | H | CH₃ | CF₃ |
| Ia₂-4 | Ph | Ph | Ph | Ph | H | H | H | H | CH₃ | CCl₃ |
| Ia₂-5 | Ph | Ph | Ph | Ph | H | H | H | H | CH₂CH₃ | CF₃ |
| Ia₂-6 | Ph | Ph | Ph | Ph | H | H | H | H | CH₃ | CH₃ |
| Ia₂-7 | Ph | Ph | Ph | Ph | H | H | H | H | CH₃ | C(O)OCH₃ |
| Ia₂-8 | Ph | Ph | Ph | Ph | H | H | H | H | CH₃ | C(O)OC₂H₅ |
| Ia₂-9 | Ph | Ph | Ph | Ph | H | H | H | H | -(CH₂)₃- | |
| Ia₂-10 | Ph | Ph | Ph | Ph | H | H | H | H | -(CH₂)₄- | |
| Ia₂-11 | Ph | Ph | Ph | Ph | H | H | H | H | -(CH₂)₅- | |

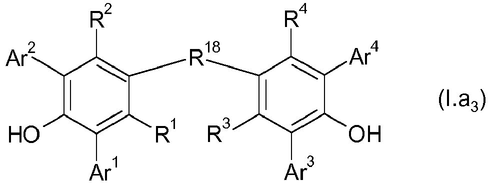

**Tabelle 3:**

| Verbindung | Ar¹ | Ar² | Ar³ | Ar⁴ | R¹ | R² | R³ | R⁴ | R¹⁸ |
|---|---|---|---|---|---|---|---|---|---|
| Ia₃-1 | Ph | Ph | Ph | Ph | H | H | H | H | -(CH₂)₂- |
| Ia₃-2 | Ph | Ph | Ph | Ph | H | H | H | H | 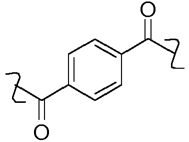 |
| Ia₃-3 | Ph | Ph | Ph | Ph | H | H | H | H | 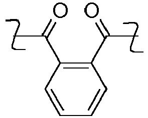 |
| Ia₃-4 | Ph | Ph | Ph | Ph | H | H | H | H | 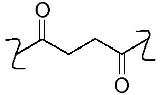 |
| Ia₃-5 | Ph | Ph | Ph | Ph | H | H | H | H | 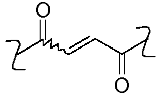 |
| Ia₃-6 | Ph | Ph | Ph | Ph | H | H | H | H | 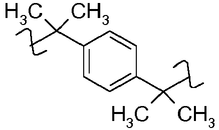 |
| Ia₃-7 | Ph | Ph | Ph | Ph | H | H | H | H | 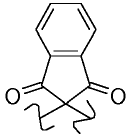 |

Dabei steht in den Tabellen 1 - 3 Ph für einen Phenylrest und C(O) steht im Rahmen der vorliegenden Erfindung für eine Carbonylgruppe. Generell können die Reste R¹⁵, R¹⁶ und R¹⁷ unabhängig voneinander für wie vorstehend definiertes C₁-C₆-Alkyl, C₁-C₁₀-Acyl, C₁-C₁₀-Carboxyl oder C₆-C₁₀-Aryl stehen, wobei die genannten Reste einen oder mehrere gleiche oder verschiedene Halogen- und/oder NO₂-Substituenten tragen können und wobei die Reste R¹⁶ und R¹⁷ gemeinsam auch ein cyclisches Strukturelement, bevorzugt eine Alkylenbrücke bilden können.

In einer bevorzugten Ausführungsform des zuvor beschriebenen Verfahrens zum Abtrennen eines Stoffes aus einer Lösung ist der Stoff ein Ligand der Formel (I) ausgewählt unter Bis(diarylphenol)-Liganden der vorstehenden Formel (I.a). Besonders bevorzugte Liganden sind solche der vorstehenden Formeln (I.a₁) bis (I.a₃), wobei den genannten Resten Ar¹ bis Ar⁴, R¹ bis R⁴ und R¹⁵ bis R¹⁸ bevorzugt die vorstehend tabellarisch beispielhaft aufgeführten Bedeutungen zukommen.

Ein weiterer Gegenstand der vorliegenden Erfindung betrifft ein Verfahren zur Herstellung von Isopulegol der Formel (IV) 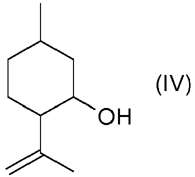 umfassend
α) die Cyclisierung von Citronellal der Formel (V) 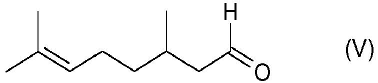 in Gegenwart eines Katalysators, der erhältlich ist durch Umsetzung eines Bis(diarylphenol)-Liganden der Formel (I), wie in den Ansprüchen 1 und/oder 10 definiert,
   mit einer Aluminium-Verbindung der Formel (II),

   (R¹⁴)₃-ₚAIHₚ (II)

   wobei
   - Al: Aluminium bedeutet,
   - R¹⁴: einen verzweigten oder unverzweigten Alkylrest mit 1 bis 5 Kohlenstoffatomen und
   - p: für 0 oder eine ganze Zahl von 1 bis 3 steht,
   und/oder
   mit einer Aluminium-Verbindung der Formel (III),

   MAIH₄ (III)

   wobei
   - Al: Aluminium bedeutet und
   - M: Lithium, Natrium oder Kalium bedeutet,
β) die Rückgewinnung des Bis(diarylphenol)-Liganden der Formel (I) nach erfolgter Umsetzung, indem man
   a) das in Schritt a) erhaltene Aluminium-haltige Reaktionsprodukt einer destillativen Auftrennung unter Erhalt eines an Isopulegol angereicherten Kopfprodukts und eines an Isopulegol abgereicherten Sumpfprodukts unterzieht,
   b) das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt bringt und
   c) den Liganden der Formel (I) aus der organischen Phase abtrennt.

Das Abtrennen des Liganden der Formel (I) erfolgt dabei durch Kristallisation, und zwar im Rahmen des vorstehend beschriebenen Verfahrens zum Abtrennen eines Stoffes aus einer Lösung.

Bezüglich der bevorzugten Ausführungsformen des erfindungsgemäßen Verfahrens zur Aufarbeitung eines Reaktionsprodukts aus der Herstellung von Isopulegol durch Cyclisierung von Citronellal sowie für die bevorzugten Liganden der Formel (I) wird im vollen Umfang auf die zuvor genannten bevorzugten Ausführungsformen verwiesen.

Die zur Herstellung der erfindungsgemäß verwendeten Bis(diarylphenoxy)-AluminiumVerbindungen einsetzbaren Bis(diarylphenol)-Liganden der Formeln (I) bzw. (I.a) lassen sich durch dem Fachmann an sich bekannte Methoden leicht herstellen. Verbindungen des Strukturtyps (I.a₁) erhält man beispielsweise durch Umsetzung der entsprechenden bis-ortho-Arylphenole mit einem Aldehyd R¹⁵CHO in Gegenwart einer Lewis-Säure, beispielsweise AlCl₃, wie u. a. beschrieben von Z. Y. Wang, A. S. Hay in Synthesis 1989, 471-472 oder in der US 3,739,035. Liganden des Strukturtyps (I.a₂) sind beispielsweise zugänglich durch Umsetzung der entsprechenden bis-ortho-Arylphenole mit einem geeigneten Keton der Formel R¹⁶C(O)R¹⁷, wie beispielsweise in der US 3,739,035 beschrieben. Liganden des Strukturtyps (I.a₃) sind beispielsweise zugänglich durch Friedel-Crafts-Acylierung der entsprechenden Phenole oder O-geschützten Phenole mit Dicarbonsäurechloriden wie beispielsweise von F. F. Blicke et al. in J. Am. Chem. Soc. 1938, 60, 2283-2285; der CH 350461 oder von G. Maier et al. in Chem. Ber. 1985, 118, 704-721 beschrieben. Eine weitere Möglichkeit zur Herstellung von Liganden des Strukturtyps (Ia₃) besteht auch in der Friedel-Crafts-Alkylierung der entsprechenden Phenole mit tertiären Diolen, wie beispielsweise in DE-A 25 34 558 beschrieben oder mit Dihalogeniden, wie beispielsweise von J. Zavada, in Collect. Czech. Chem. Commun., 1976, 41, 1777-1790, beschrieben.

Die erfindungsgemäß verwendeten Bis(diarylphenoxy)-Aluminium-Verbindungen erhält man beispielsweise, indem man die vorstehend beschriebenen Bis(diarylphenol)-Liganden der Formeln (I) bzw. (I.a) mit einer Aluminium-Verbindung der Formel (II)

(R¹⁴)₃₋ₚAIHₚ (II),

umsetzt. Dabei steht R¹⁴ für einen verzweigten oder unverzweigten Alkylrest mit 1 bis 5 Kohlenstoffatomen wie beispielsweise Methyl, Ethyl, Propyl, Isopropyl, Butyl, Isobutyl, Pentyl, Isopentyl oder Neopentyl. Der Index p steht für 0 oder eine ganze Zahl von 1 bis 3. Bevorzugt steht der Index p für 1 oder 0, besonders bevorzugt für 0. Bevorzugte Verbindungen der Formel (II) sind beispielsweise Trimethylaluminium, Triethylaluminium, Diisobutylaluminiumhydrid, besonders bevorzugt Trimethylaluminium und Triethylaluminium.

Alternativ dazu erhält man die erfindungsgemäß verwendeten Bis(diarylphenoxy)-Aluminium-Verbindungen auch durch Umsetzung der wie vorstehend beschriebenen Bis(diarylphenol)-Liganden der Formeln (I) bzw. (I.a) mit einer Aluminium-Verbindung der Formel (III)

MAIH₄ (III),

wobei M Lithium, Natrium oder Kalium bedeutet. Demzufolge eignen sich zur Herstellung der erfindungsgemäß verwendeten Bis(diarylphenoxy)-AluminiumVerbindungen durch Umsetzung der wie vorstehend beschriebenen Bis(diarylphenol)-Liganden der Formeln (I) bzw. (I.a) auch Lithiumaluminiumhydrid, Natriumaluminiumhydrid und Kaliumaluminiumhydrid sowie Gemische derselben. Darüber hinaus sind auch Gemische der genannten Verbindungen der Formeln (II) und (III) zur Herstellung erfindungsgemäß verwendeten Bis(diarylphenoxy)-AluminiumVerbindungen durch Umsetzung mit den wie vorstehend beschriebenen Bis(diarylphenol)-Liganden der Formeln (I) bzw. (I.a) geeignet.

Die Umsetzung wird vorteilhaft so durchgeführt, dass einer der wie vorstehend beschriebenen Bis(diarylphenol)-Liganden der Formeln (I) bzw. (I.a) mit einer Verbindung der Formel (II) oder (III) in Kontakt gebracht wird. Vorteilhaft führt man die Umsetzung in einem inerten organischen Lösungsmittel wie beispielsweise Toluol, Cyclohexan, Dichlormethan, Xylol, Ethylbenzol, Chlorbenzol, Tetrahydrofuran, Diethylether, Methyl-tert-Butylether, Essigsäureethylester, Pentan, Hexan, Dichlorethan, Dimethylformamid (DMF), Dimethylsulfoxid (DMSO) und dergleichen mehr durch, wobei der Einsatz vorgetrockneter bzw. wasserfreier Lösungsmittel als besonders vorteilhaft anzusehen ist. Üblicherweise erfolgt die Umsetzung bei Temperaturen im Bereich von etwa -100 °C bis etwa 100 °C, bevorzugt bei etwa -50 °C bis etwa 50 °C, besonders bevorzugt bei etwa -30 °C bis etwa 30 °C.

Bei der Herstellung der erfindungsgemäßen Bis(diarylphenoxy)-AluminiumVerbindungen reagieren die phenolischen Hydroxy-Gruppen der eingesetzten Bis(diarylphenol)-Liganden der Formeln (I) bzw. (I.a) mit der bzw. den Verbindungen der Formeln (II) und (III). Theoretisch kann jedes Aluminium-Atom mit 1 bis 3 phenolischen Hydroxy-Gruppen regieren. Aufgrund der sterischen Eigenschaften bzw. Anforderungen der eingesetzten Bis(diarylphenol)-Liganden der Formeln (I) bzw. (I.a) kann es dabei zur Ausbildung höhermolekularer Strukturen wie linearen Strukturen oder Netzwerken kommen.

Vorteilhaft wählt man dabei das molare Verhältnis der eingesetzten Bis(diarylphenol)-Liganden der Formeln (I) bzw. (I.a) zu den eingesetzten Verbindungen der Formel (II) und/oder (III) so, dass die Menge an nicht abreagierten Verbindungen der Formeln (II) und/oder (III) möglichst gering ist. Vorzugsweise wählt man das genannte Verhältnis so, dass nach dem Inkontaktbringen der Bis(diarylphenol)-Liganden der Formeln (I) bzw. (I.a) mit der bzw. den Verbindungen der Formeln (II) und (III) keine nicht umgesetzte Verbindung der Formel (II) und/oder (III) mehr vorliegt. Unter Berücksichtigung des wirtschaftlichen Aspekts ist es empfehlenswert, den Überschuss der eingesetzten Liganden der Formeln (I) bzw. (I.a) gering zu halten. Besonders bevorzugt setzt man Bis(diarylphenol)-Liganden der Formeln (I) bzw. (I.a) und die Verbindungen der Formeln (II) und/oder (III) in einem molaren Verhältnis von etwa 4:1 bis etwa 1:1, ganz besonders bevorzugt von etwa 3:1 bis etwa 1,5:1 und am meisten bevorzugt im molaren Verhältnis von etwa 1,5:1 ein.

Zur Herstellung der erfindungsgemäß verwendeten Bis(diarylphenoxy)-AluminiumVerbindungen geht man im Rahmen einer bevorzugten Ausführungsform der vorliegenden Erfindung so vor, das man, je nach Löslichkeit, eine etwa 0,001 bis etwa 1 molare Lösung des gewählten Liganden der Formel (I) bzw. (I.a) in einem geeigneten organischen Lösungsmittel, beispielsweise Toluol, bei einer Temperatur von etwa -10 bis etwa 30 °C vorlegt und eine Aluminiumverbindung der Formel (II) und/oder (III), vorzugsweise in Form einer Lösung, beispielsweise eine Lösung von Trimethyl- oder Triethylaluminium in Toluol, zugibt.

Die Reaktion zwischen den eingesetzten Liganden der Formel (I) bzw. (I.a) und den Aluminiumverbindungen der Formeln (II) und/oder (III) erfolgt in der Regel rasch und ist meist, in Abhängigkeit von den gewählten Reaktionsbedingungen, nach etwa 10 min bis etwa 2 h, oft nach etwa 1 h, abgeschlossen. Bei Einsatz reaktionsträgerer Reaktanden kann es vorteilhaft sein, die Temperatur des Reaktionsgemisches kurzzeitig zu erhöhen.

In Abhängigkeit von den gewählten Reaktionsbedingungen, insbesondere im Hinblick auf die Löslichkeit der umzusetzenden Liganden der Formel (I) bzw. (I.a) und der Aluminiumverbindung der Formel (II) und/oder (III) in den gewählten Lösungsmitteln, den Konzentrationen sowie den Reaktionstemperaturen, erhält man die erfindungsgemäßen Bis(diarylphenoxy)-Aluminium-Verbindungen in Form eines Feststoffes, einer Suspension oder einer Lösung im eingesetzten Lösungsmittel bzw. Lösungsmittelgemisch. Die so erhaltenen erfindungsgemäß verwendeten Bis(diarylphenoxy)-Aluminium-Verbindungen können in der jeweils erhaltenen Form weiterverwendet werden oder abgetrennt und von den eingesetzten Lösungsmitteln befreit werden.

Die Isolierung kann dabei nach dem Fachmann bekannten und vorteilhaft erscheinenden Methoden erfolgen. Vorzugsweise führt man die Isolierung, Lagerung bzw. Weiterbehandlung der erfindungsgemäß verwendeten Bis(diarylphenoxy)-Aluminium-Verbindungen unter weitgehendem Ausschluss von Sauerstoff und Feuchtigkeit durch.

Zur Durchführung des erfindungsgemäßen Verfahrens zur Herstellung von Isopulegol geht man vorteilhaft so vor, dass man zunächst eine Lösung der erfindungsgemäß verwendeten Bis(diarylphenoxy)-Aluminium-Verbindungen in einem geeigneten Lösungsmittel, wie vorstehend beschrieben, bereitstellt. Dieser Lösung setzt man dann erfindungsgemäß das zu cyclisierende racemische oder nicht-racemische Citronellal zu. Das Citronellal kann dabei als solches oder in Form einer Lösung, vorteilhaft in einem der vorstehend genannten geeigneten Lösungsmittel zugegeben werden. Im Rahmen einer bevorzugten Ausführungsform des erfindungsgemäßen Verfahrens bereitet man zunächst eine Lösung des gewählten Liganden der Formeln (I) bzw. (I.a) in Toluol und setzt dann, vorteilhaft unter Rühren, die gewählte Aluminium-Verbindung der Formel (II) und/oder (III), bevorzugt Trimethyl- oder Triethylaluminium in toluolischer, Lösung zu.

Als Ausgangsstoff zur Durchführung des erfindungsgemäßen Cyclisierungsverfahrens eignet sich Citronellal, das nach jedwedem Verfahren hergestellt sein kann. Bevorzugt setzt man Citronellal ein, das eine Reinheit von etwa 90 bis etwa 99,9 Gew.-%, besonders bevorzugt von etwa 95 bis etwa 99,9 Gew.-%, aufweist.

Die Zugabe des zu cyclisierenden Citronellals erfolgt vorteilhaft bei Temperaturen im Bereich von etwa -40 °C bis etwa 40 °C, bevorzugt im Bereich von etwa -20 °C bis etwa 20 °C. Dazu wird vorteilhaft die bereitete Lösung der erfindungsgemäß verwendeten Bis(diarylphenoxy)-Aluminium-Verbindung auf eine Temperatur in diesem Bereich, z. B. auf eine Temperatur im Bereich von -10 °C bis 10 °C abgekühlt und vorgekühltes Citronellal bzw. eine vorgekühlte Lösung von Citronellal zugegeben.

Die Zugabe des Citronellals bzw. der Lösung davon kann so vorgenommen werden, dass man entweder die gesamte Menge auf einmal zusetzt oder sie portionsweise oder auch kontinuierlich zur bereiteten Katalysatorlösung gibt. Als Lösungsmittel eignen sich wiederum die vorstehend genannten Lösungsmittel, insbesondere Toluol. Bevorzugt setzt man das zu cyclisierende Citronellal als solches, d. h. ohne weiteren Zusatz von Lösungsmitteln, ein. Bei Einsatz eines Lösungsmittels wird die gesamte Lösungsmittelmenge (für Katalysatorherstellung und zur Durchführung der Cyclisierungsreaktion) vorteilhaft so gewählt, dass das volumenbezogene Verhältnis von umzusetzendem Citronellal zum Lösungsmittel etwa 2:1 bis etwa 1:20, bevorzugt von etwa 1,5:1 bis etwa 1:10 beträgt.

Das Mengenverhältnis zwischen dem umzusetzendem Citronellal und der eingesetzten Menge der erfindungsgemäß verwendeten Bis(diarylphenoxy)-Aluminium-Verbindung wird durch die Menge der zur Herstellung derselben eingesetzten Verbindungen der Formel (I) bzw. (I.a) und der Formel (II) und/oder (III), also durch das Mengenverhältnis von eingesetztem Ligand zu eingesetzter Aluminium-Verbindung der Formel (II) und/oder (III) bestimmt.

Erfindungsgemäß wählt man die Menge von umzusetzendem Citronellal zur eingesetzten Menge an Aluminium-Verbindung der Formel (II) und/oder (III) so, dass das molare Verhältnis etwa 5:1 bis etwa 1000:1, bevorzugt etwa 10:1 bis etwa 500:1, besonders bevorzugt etwa 50:1 bis etwa 200:1 beträgt.

Unabhängig davon kann das Verhältnis zwischen eingesetztem Ligand der Formel (I) bzw. (I.a) und der eingesetzten Aluminium-Verbindung der Formel (II) und/oder (III) in den vorstehend zur Herstellung der erfindungsgemäßen Bis(diarylphenoxy)-Aluminium-Verbindung genannten Grenzen variiert werden.

Die Cyclisierung von Citronallal zu Isopulegol erfolgt je nach Wahl der Reaktionspartner und Reaktionsbedingungen in der Regel rasch und ist üblicherweise nach etwa 0,5 bis etwa 10 h, oft nach etwa 5 h, weitgehend abgeschlossen. Der Reaktionsfortschritt kann durch dem Fachmann an sich bekannte Methoden, beispielsweise durch chromatographische, speziell gaschromatographische Methoden oder auch HPLC-Methoden leicht verfolgt werden.

Im Rahmen einer bevorzugten Ausführungsform des erfindungsgemäßen Verfahrens führt man die Cyclisierung von Citronellal zu Isopulegol in Gegenwart eines Hilfsstoffs (iv), beispielsweise einer Säure, vorzugsweise einer organischen Säure durch. Beispielhaft seien als vorteilhaft einsetzbare organische Säuren genannt: Essigsäure, Propionsäure, Benzoesäure, Toluolsulfonsäure, Methansulfonsäure, bevorzugt Essigsäure. Die genannten Säuren werden vorteilhaft in einer Menge von etwa 0,5 bis etwa 10 Gew.-%, bezogen auf die Menge an umzusetzendem Citronellal, eingesetzt. Vorteilhaft werden sie zusammen mit dem Citronellal, z. B. in Form eines Gemisches, dem Reaktionsgemisch zugesetzt.

In einer besonders bevorzugten Ausführungsform führt man das erfindungsgemäße Verfahren zur Herstellung von Isopulegol durch Cyclisierung von Citronellal in Gegenwart mindestens eines Hilfsstoffs (iv) durch, der ausgewählt ist unter Carbonsäureanhydriden, Aldehyden, Ketonen und Vinylethern.

Die Hilfsstoffe (iv) der genannten Substanzklassen können jeweils einzeln oder in Form von Gemischen untereinander eingesetzt werden. Bevorzugt setzt man im Fall von Gemischen solche ein, die aus Verbindungen einer Substanzklasse bestehen. Besonders bevorzugt setzt man einzelne Verbindungen ein. Unter wie nachfolgend beschriebenem Einsatz der genannten Verbindungen gelingt es in der Regel, die Bildung unerwünschter Nebenprodukte weitgehend zu unterdrücken.

Im Rahmen einer bevorzugten Ausführungsform führt man die Cyclisierung von Citronellal in Gegenwart eines Carbonsäureanhydrids der Formel (VI) 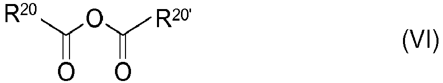 durch wobei die Reste R²⁰ und R^{20'} gleich oder verschieden, bevorzugt gleich, sein können und einen verzweigten oder unverzweigten C₁-C₁₂-Alkylrest oder C₇-C₁₂-Aralkylrest oder einen C₆-C₁₀-Arylrest bedeuten, wobei die genannten Reste jeweils einen oder mehrere, in der Regel 1 bis etwa 3, gleiche oder verschiedene Substituenten, ausgewählt aus der Gruppe OR^{10e}, SR^{10f} NR^{8e}R^{9e} und Halogen aufweisen können und wobei R²⁰ und R^{20'} gemeinsam auch einen 5- bis 8-gliedrigen Ring bilden können, der eine oder mehrere ethylenische Doppelbindungen und ein oder mehrere gleiche oder verschiedene Heteroatome, ausgewählt aus der Gruppe O, S und NR^{11b} aufweisen kann und wobei R^{10e}, R^{10f}, R^{8e}, R^{9e} und R^{11b} die vorstehend für R¹¹ angegebenen Bedeutungen haben können.

Im Rahmen einer weiteren bevorzugten Ausführungsform führt man die Cyclisierung von Citronellal in Gegenwart eines Aldehyds der Formel (VII) 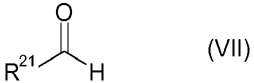 durch, wobei der Rest R²¹ einen verzweigten oder unverzweigten C₁-C₁₂-Alkylrest oder C₇-C₁₂-Aralkylrest oder einen C₆-C₁₀-Arylrest bedeutet, wobei die genannten Reste jeweils einen oder mehrere, bevorzugt 1 bis 3, gleiche oder verschiedene Substituenten, ausgewählt aus der Gruppe OR^{10e}, SR^{10f} NR^{8e}R^{9e} und Halogen aufweisen können, wobei R^{10e}, R^{10f}, R^{8e} und R^{9e} die vorstehend für R¹¹ angegebenen Bedeutungen haben können.

Im Rahmen einer weiteren bevorzugten Ausführungsform führt man Cyclisierung von Citronellal in Gegenwart eines Ketons der Formel (VIII) 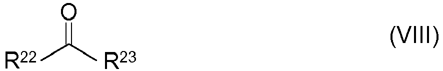 durch, wobei die Reste R²² und R²³ jeweils gleich oder verschieden sein können und einen verzweigten oder unverzweigten C₁-C₁₂-Alkylrest oder C₇-C₁₂-Aralkylrest oder einen C₆-C₁₀-Arylrest oder einen C₁-C₆-Alkoxycarbonylrest bedeuten, wobei die genannten Reste jeweils einen oder mehrere, bevorzugt 1 bis 3, gleiche oder verschiedene Substituenten, ausgewählt aus der Gruppe OR^{10e}, SR^{10f} NR^{8e}R^{9e} und Halogen aufweisen können, und wobei R²² und R²³ gemeinsam auch einen 5- bis 8-gliedrigen Ring bilden können, der eine oder mehrere ethylenische Doppelbindungen und ein oder mehrere gleiche oder verschiedene Heteroatome, ausgewählt aus der Gruppe O, S, NR^{11b} aufweisen kann und wobei R^{10e}, R^{10f}, R^{8e}, R^{9e} und R^{11b} die vorstehend für R¹¹ angegebenen Bedeutungen haben können.

Alternativ zu den zuvor genannten Carbonylverbindungen lassen sich auch Vinylether der allgemeinen Formel (IX) 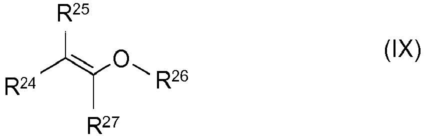 im Rahmen des erfindungsgemäßen Verfahrens einsetzen, wobei die Reste R²⁴, R²⁵, R²⁶ und R²⁷ unabhängig voneinander jeweils gleich oder verschieden sein können und einen verzweigten oder unverzweigten C₁-C₁₂-Alkylrest oder C₇-C₁₂-Aralkylrest oder einen C₆-C₁₀-Arylrest bedeuten, wobei die genannten Reste jeweils einen oder mehrere, bevorzugt 1 bis 3, gleiche oder verschiedene Substituenten, ausgewählt unter Oxo, OR^{10e}, SR^{10f} NR^{8e}R^{9e} und Halogen aufweisen können und wobei R²⁵ und R²⁶ gemeinsam auch einen 5- bis 8-gliedrigen Ring bilden können, der eine oder mehrere ethylenische Doppelbindungen und ein oder mehrere, üblicherweise 1 oder 2, gleiche oder verschiedene Heteroatome, ausgewählt aus der Gruppe O, S, NR^{11b} aufweisen kann und wobei R^{10e}, R^{10f}, R^{8e}, R^{9e} und R^{11b} die vorstehend für R¹¹ angegebenen Bedeutungen haben können.

C₁-C₁₂-Alkyl steht dabei für wie vorstehend beschriebenes C₁-C₆-Alkyl und darüber hinaus beispielsweise für Heptyl, Octyl, Nonyl, Decyl, Undecyl oder Dodecyl. In den Fällen, in denen zwei Alkylreste gemeinsam einen Ring ausbilden, sind unter Alkylresten auch Alkylenylreste zu verstehen. C₇-C₁₂-Aralkylresten und C₆-C₁₀-Arylresten können beispielhaft die vorstehend genannten Bedeutungen zukommen. Beispielhaft seien als C₁-C₆-Alkoxycarbonylrest genannt: Methoxycarbonyl, Ethoxycarbonyl, Propoxycarbonyl und Isopropoxycarbonyl, bevorzugt Methoxycarbonyl und Ethoxycarbonyl.

Im Rahmen einer bevorzugten Ausführungsform des erfindungsgemäßen Verfahrens führt man die Cyclisierung von Citronellal in Gegenwart eines Carbonsäureanhydrids der Formel (VI) durch, wobei die Reste R²⁰ und R^{20'} gleich sind und einen verzweigten oder unverzweigten C₁-C₁₂-Alkylrest oder C₇-C₁₂-Aralkylrest oder einen C₆-C₁₀-Arylrest bedeuten, und wobei R²⁰ und R^{20'} gemeinsam auch einen 5- bis 8-gliedrigen Ring bilden können, der eine oder mehrere ethylenische Doppelbindungen und ein oder mehrere gleiche oder verschiedene Heteroatome, ausgewählt aus der Gruppe OR^{10e}, SR^{10f,} NR^{11b} aufweisen kann und R^{10e}, R^{10f} und R^{11b} unabhängig voneinander die vorstehend für R¹¹ angegebenen Bedeutungen haben kann.

Insbesondere bevorzugt setzt man solche Carbonsäureanhydride ein, bei denen die Reste R²⁰ und R^{20'} gleich sind und einen verzweigten oder unverzweigten C₁-C₁₂-Alkylrest oder einen C₆-C₁₀-Arylrest bedeuten. Beispielhaft seien als erfindungsgemäß besonders bevorzugt einzusetzende Carbonsäureanhydride genannt: Essigsäureanhydrid, Propionsäureanhydrid, Pivalinsäureanhydrid und Benzoesäureanhydrid.

Als erfindungsgemäß ebenfalls bevorzugt einsetzbare Aldehyde der Formel (VII) seien beispielhaft Acetaldeyhd, Propionaldehyd und Chloral (Trichloracetaldehyd) genannt. Führt man die Cyclisierung von Citronellal im Rahmen einer weiteren bevorzugten Ausführungsform in Gegenwart eines Ketons der Formel (VIII) durch, setzt man mit Vorteil solche mit einer aktivierten, d. h. elektronenarmen, Carbonylfunktion ein. Beispielhaft seien die folgenden Ketone genannten, die sich in besonderem Maße zum Einsatz im Rahmen des erfindungsgemäßen Verfahrens eigenen: 1,1,1-Trifluoraceton, 1,1,1-Trifluoracetophenon, Hexafluoraceton, Brenztraubensäuremethylester und Brenztraubensäureethylester.

Als erfindungsgemäß ebenfalls bevorzugt einsetzbare Vinylether der Formel (IX) seien beispielhaft genannt: Methylvinylether, Ethylvinylether, Isobutylvinylether und 3,4-Dihydro-2H-pyran.

Die genannten Verbindungsklassen können gleichermaßen mit gutem Erfolg im Rahmen dieser bevorzugten Ausführungsform des erfindungsgemäßen Verfahrens eingesetzt werden. Im Hinblick auf praktische Gesichtspunkte wie beispielsweise eine höhere Reaktionsgeschwindigkeit hat sich der Einsatz von Aldehyden und/oder elektronenarmen Ketonen als vorteilhaft erweisen.

Die Menge an erfindungsgemäß zu verwendendem Carbonsäureanhydrid, Aldehyd, Keton und/oder Vinylether kann in breiten Grenzen variiert werden und richtet sich nach Art des eingesetzten Stoffes und dem Reinheitsgrad bzw. dem Vorhandensein noch nicht näher identifizierter Verunreinigungen. Üblicherweise setzt man die genannten Verbindungen bzw. deren Gemische in einer Menge von etwa 0,01 mol-% bis etwa 5 mol-%, bevorzugt von etwa 0,1 mol-% bis etwa 2 mol-%, bezogen auf die eingesetzte Menge an Citronellal, ein.

An die Art und Weise der Reaktionsführung, beispielsweise die Ausgestaltung von Reaktoren oder die Reihenfolge der Zugabe einzelner Reaktionspartner, sind keine besonderen Anforderungen zu stellen, solange eine Reaktionsführung unter weitgehendem Ausschluss von Sauerstoff und Wasser gewährleistet ist.

Zur Durchführung des erfindungsgemäßen Verfahrens im Rahmen dieser bevorzugten Ausführungsform geht man vorteilhaft so vor, dass man zunächst eine Lösung der erfindungsgemäß einzusetzenden Bis(diarylphenoxy)-Aluminium-Verbindung in einem geeigneten Lösungsmittel wie vorstehend beschrieben bereitstellt. Dieser Lösung setzt man dann erfindungsgemäß bevorzugt ein Gemisch des zu cyclisierenden racemischen oder nicht-racemischen Citronellals mit dem gewählten Carbonsäureanhydrid, dem Aldehyd, dem aktivierten Keton und/oder dem Vinylether zu. Alternativ dazu kann man beispielsweise auch die Lösung der erfindungsgemäß einzusetzenden Bis(diarylphenoxy)-Aluminium-Verbindung zunächst mit dem gegebenenfalls jeweils gewählten Carbonsäureanhydrid, dem Aldehyd, dem Keton und/oder dem Vinylether versetzen und im Anschluss daran das zu cyclisierende Citronellal zugeben.

Es sich als vorteilhaft erwiesen, das Citronellal bzw. das Gemisch von Citronellal mit der gewählten Verbindung innerhalb eines Zeitraums von etwa 30 min bis etwa 6 h, bevorzugt innerhalb von etwa 2 h bis etwa 4 h, zur Katalysatorlösung bzw. dem Reaktionsgemisch zuzudosieren. Das Citronellal kann dabei als solches oder in Form einer Lösung, vorteilhaft in einem der vorstehend genannten geeigneten Lösungsmittel, zugegeben werden. Im Rahmen einer wiederum bevorzugten Ausführungsform des erfindungsgemäßen Verfahrens bereitet man zunächst eine Lösung des gewählten Liganden der Formeln (I) bzw. (I.a) in Toluol und setzt dann, zweckmäßigerweise unter Rühren, die gewählte Aluminium-Verbindung der Formel (II) und/oder (III), bevorzugt Trimethyl- oder Triethylaluminium in toluolischer Lösung zu.

Die Zugabe des zu cyclisierenden Citronellals bzw. des Gemischs von Citronellal mit dem gewählten Carbonsäureanhydrid, Aldehyd, aktivierten Keton und/oder dem Vinylether erfolgt im Rahmen dieser Ausführungsform vorteilhaft bei Temperaturen im Bereich von etwa -40 °C bis etwa 40 °C, bevorzugt im Bereich von etwa -20 °C bis etwa 20 °C. Dazu wird vorteilhaft die bereitete Lösung oder Suspension der erfindungsgemäßen Bis(diarylphenoxy)-Aluminium-Verbindung auf eine Temperatur in diesem Bereich, z. B. auf eine Temperatur im Bereich von -10 °C bis 10 °C, abgekühlt und die weiteren Reaktanden in vorgekühlter Form zugegeben.

Die Zugabe des Gemischs von Citronellal und der gewählten weiteren Verbindung kann so vorgenommen werden, dass man entweder die gesamte Menge Citronellal auf einmal zusetzt oder sie portionsweise oder auch kontinuierlich zur bereiteten Katalysatorlösung gibt. Als Lösungsmittel eignen sich wiederum bevorzugt die vorstehend genannten Lösungsmittel, insbesondere Toluol. Bevorzugt setzt man das zu cyclisierende Citronellal in Form eines Gemisches mit dem gewählten Carbonsäureanhydrid, Aldehyd, aktivierten Keton und/oder Vinylether ohne weiteren Zusatz von Lösungsmitteln ein. Bei Einsatz eines Lösungsmittels wählt man die gesamte Lösungsmittelmenge vorteilhaft so, dass das volumenbezogene Verhältnis von umzusetzendem Citronellal zum Lösungsmittel etwa 1 : 1 bis etwa 1 : 20, bevorzugt von etwa 1 : 1 bis etwa 1 : 10 beträgt.

Es wurde gefunden, dass üblicherweise während der Reaktion ein Teil des Katalysatorkomplexes deaktiviert wird. Dies ist unter anderem auf Ligandenaustausch-Prozesse zwischen den jeweils eingesetzten Bis(diarylphenol)-Liganden der Formel der eingesetzten Bis(diarylphenoxy)-Aluminium-Verbindungen und dem durch Cyclisierung entstehenden Isopulegol zurückzuführen. Die deaktivierte Form des Katalysators ist, in Abhängigkeit von der Wahl der eingesetzten Lösemittel, üblicherweise im Gegensatz zum aktiven polymeren Katalysator in der Reaktionsmischung löslich.

In einer bevorzugten Ausführungsform kann durch einfache physikalische Trennmethoden (z. B. durch Abfiltration oder Zentrifugation des noch aktiven Katalysators) der deaktiverte Teil des Katalysators zusammen mit der übrigen Reaktionsmischung abgetrennt werden. Der zurückgehaltene, immer noch aktive Teil des Katalysators kann gewünschtenfalls mit frischem Katalysator ergänzt werden und ohne nennenswerten Aktivitätsverlust wieder eingesetzt werden, vorzugsweise im Rahmen einer weiteren erfindungsgemäßen Cyclisierungsreaktion von Citronellal zu Isopulegol.

Alternativ kann die eingesetzte Katalysatormenge so gewählt werden, dass der gesamte eingesetzte Katalysatorkomplex im Laufe bzw. nach Beendigung der erfindungsgemäßen Cyclisierungsreaktion deaktiviert und somit löslich ist, was an einer klaren Reaktionsmischung zu erkennen ist. Dabei macht sich vorteilhaft bemerkbar, dass in diesem Falle aufgrund der vorstehend beschriebenen Ligandenaustausch-Prozesse der jeweils eingesetzte Bis(diarylphenol)-Ligand der Formel (I) ohne gesondert durchzuführende Hydrolyse freigesetzt wird.

Überraschenderweise wurde gefunden, dass Isopulegol aus den Aluminium-haltigen Reaktionsprodukten der Cyclisierung von Citronellal ohne vorherige Hydrolyse der jeweils als Katalysator eingesetzten (bis(Diarylphenoxy)-Aluminium-Verbindungen (gegebenenfalls nach der destillativen Entfernung eines eingesetzten Lösungsmittels und/oder zusätzlich eingesetzter Hilfsstoffe) in hohen Reinheiten abdestilliert werden kann. Dabei bilden sich im Destillationssumpf in der Regel keine erkennbaren unerwünschten oder störenden Nebenprodukte. In einer speziellen Ausführung erfolgt vor oder während der destillativen Auftrennung in Schritt a) die Zugabe eines geeigneten, inerten, hochsiedenden Lösungsmittels. Dann erhält man im Destillationssumpf eine Lösung des Liganden der Formel (I) in dem erwärmten, jeweils eingesetzten Hochsieder.

Das erfindungsgemäße Verfahren eignet sich, wie bereits erwähnt, in gleichem Maße zur Cyclisierung von racemischem wie auch nicht-racemischem, d. h. optisch aktivem Citronellal zu racemischem wie nicht-racemischem Isopulegol.

Daher dient das erfindungsgemäße Verfahren in einer bevorzugten Ausführungsform zur Herstellung von optisch aktivem Isopulegol der Formel (IV.a) 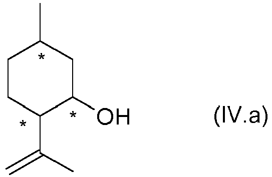 durch Cyclisierung von aktivem Citronellal der Formel (V.a) 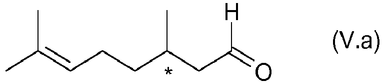 wobei (*) jeweils ein asymmetrisches Kohlenstoffatom bezeichnet.

Insbesondere dient das erfindungsgemäße Verfahren zur Herstellung von L-(-)-Ispulegol durch Cyclisierung von D-(+)-Citronellal.

Das so hergestellte racemische bzw. nicht-racemische Isopulegol stellt ein wertvolles Intermediat zur Herstellung von racemischem bzw. nicht-racemischem Menthol, einem der weltweit bedeutendsten Riech- bzw. Aromastoffe dar. Menthol kann durch dem Fachmann an sich bekannte Methoden der Hydrierung, speziell der katalytischen Hydrierung an geeigneten Übergangsmetallkatalysatoren, wie beispielsweise in Pickard et al., J. Chem. Soc. 1920, 1253; Ohloff et al., Chem. Ber. 1962, 95, 1400; Pavia et al., Bull. Soc. Chim. Fr. 1981, 24, Otsuka et al., Synthesis 1991, 665 oder in der EP 1 053 974 A beschrieben, aus Isopulegol erhalten werden. Dabei bleibt bei geeigneter Wahl der Reaktionsbedingungen die relative bzw. absolute Konfiguration des eingesetzten Isopulegols weitgehend, in vielen Fällen vollständig erhalten.

Ein weiterer Gegenstand der vorliegenden Erfindung betrifft daher ein Verfahren zur Herstellung von Menthol, umfassend die Schritte:
A) Herstellung von Isopulegol der Formel (IV) nach einem erfindungsgemäßen Verfahren
B) Hydrierung der ethylenischen Doppelbindung des so erhaltenen Isopulegols.

In einer bevorzugten Ausführungsform dient dieses Verfahren zur Herstellung von optisch aktivem Menthol, speziell zur Herstellung von L-(-)-Menthol aus optisch aktivem L-(-)-Isopulegol.

Bezüglich der bevorzugten Ausführungsformen des erfindungsgemäßen Verfahrens zur Herstellung von Isopulegol wird im vollen Umfang auf die zuvor genannten Bevorzugungen verwiesen.

Im Folgenden werden Ausführungsbeispiele der Erfindung mit Bezug zu den Zeichnungen erläutert.
- Figur 1: zeigt den Aufbau eines ersten Beispiels der Vorrichtung zum Abtrennen eines Stoffes aus einer Lösung,
- Figur 2: zeigt den Aufbau der Streulichtsonde, welche bei der in Figur 1 gezeigten Vorrichtung verwendet wird,
- Figur 3: zeigt ein Diagramm, bei dem ein Beispiel des Temperaturverlaufs und der detektierten Intensität für ein Ausführungsbeispiel des erfindungsgemäßen Verfahrens aufgetragen ist,
- Figur 4: zeigt ein Diagramm, welches den Zusammenhang zwischen der detektierten Intensität und der Temperatur für ein Ausführungsbeispiel des erfindungsgemäßen Verfahrens wiedergibt, und
- Figur 5: zeigt ein weiteres Beispiel einer Vorrichtung zum Abtrennen eines Stoffes aus einer Lösung.

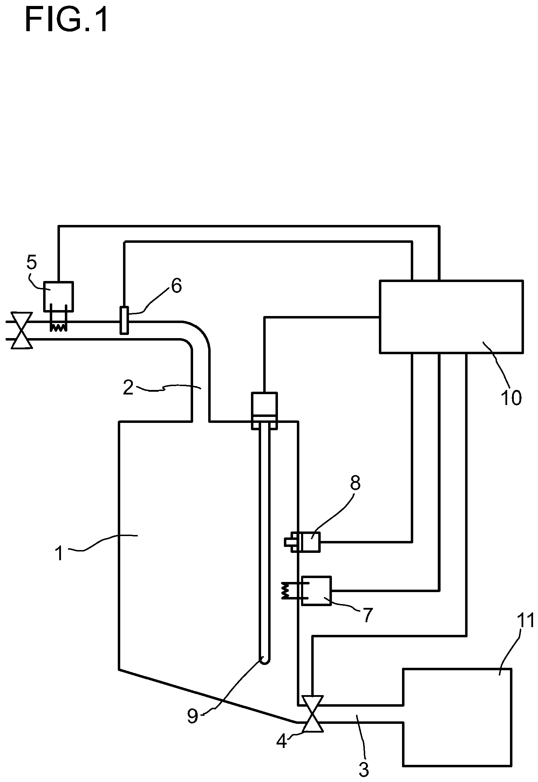

Mit Bezug zu Figur 1 wird der Aufbau des ersten Beispiels der Vorrichtung zum Abtrennen eines Stoffes aus einer Lösung erläutert:
Die Vorrichtung umfasst ein Kristallisationsgefäß 1, welches eine Zuleitung 2 und eine Ableitung 3 aufweist. Über die Zuleitung 2 wird die Lösung in das Kristallisationsgefäß 1 eingeleitet. Damit die eingeleitete Lösung zunächst in dem Kristallisationsgefäß 1 verbleibt, ist in der Ableitung 3 ein elektronisch ansteuerbares Ventil 4 vorgesehen, welches zunächst geschlossen ist. Nachdem das Kristallisationsverfahren in dem Kristallisationsgefäß 1 durchgeführt worden ist, wird die Suspension mit den Kristallen durch die Ableitung 3 aus dem Kristallisationsgefäß 1 herausgeführt.

In der Zuleitung 2 oder alternativ in einem Vorratsgefäß ist eine Temperiereinrichtung 5 vorgesehen. Mittels dieser Temperiereinrichtung 5 kann die Temperatur der Lösung, welche über die Zuleitung 2 in das Kristallisationsgefäß 1 eingeleitet wird, geregelt werden. Für die Temperaturregelung ist des Weiteren ein Temperatursensor 6 in der Zuleitung 2 vorgesehen. Des Weiteren ist auch in dem Kristallisationsgefäß 1 eine Temperiereinrichtung 7 und ein Temperatursensor 8 vorgesehen, mittels derer die Temperatur der Lösung, welche sich in dem Kristallisationsgefäß 1 befindet, gemessen und geregelt wird.

Schließlich befindet sich innerhalb des Kristallisationsgefäßes 1 eine Streulichtsonde 9, welche später im Detail erläutert wird. Das Ventil 4, die Temperiereinrichtungen 5 und 7, die Temperatursensoren 6 und 8 sowie die Streulichtsonde 9 sind datentechnisch mit einer Regeleinheit 10 gekoppelt. Auf diese Weise werden die Messwerte der Temperatursensoren 6 und 8 und die Messwerte der Streulichtsonde 9 an die Regeleinheit 10 übertragen. Des Weiteren steuert die Regeleinheit 10 die Lichtemission der Streulichtsonde 9, wie es später erläutert wird, und die Heiz- oder Kühlleistung der Temperiereinrichtungen 5 und 7. Des Weiteren kann mittels der Regeleinheit 10 das Ventil 4 geöffnet und geschlossen werden.

Die Ableitung 3, durch welche die Suspension dem Kristallisationsgefäß 1 entnommen wird, ist mit einer Abtrenneinheit 11 verbunden. Die Abtrenneinheit 11 kann als an sich bekannte Filtervorrichtung ausgebildet sein.

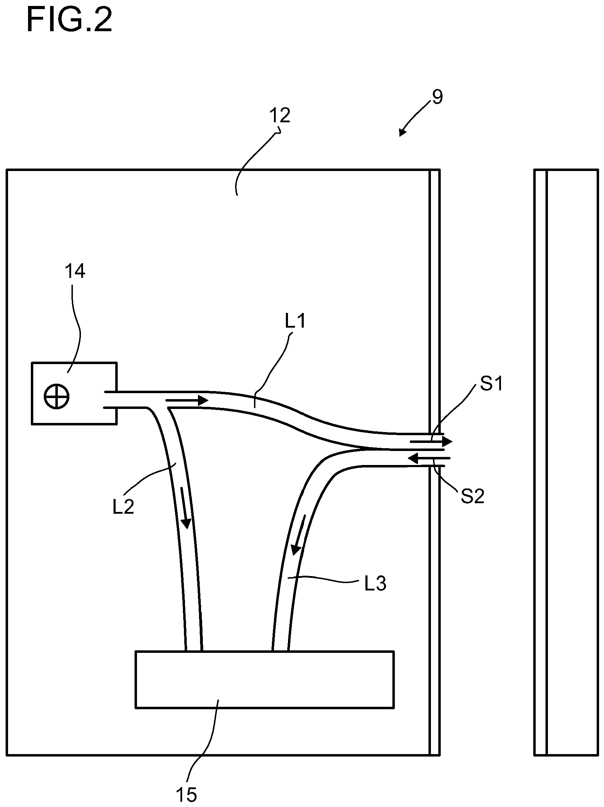

Im Folgenden wird mit Bezug zur Figur 2 die Streulichtsonde 9 im Detail beschrieben:
Die Streulichtsonde 9 umfasst ein Rohr 12, in dem sich die Wellenleiter L1 und L3 befinden. Am Ende des Rohres 12, das in das Kristallisationsgefäß 1 eintaucht, besitzt der Wellenleiter L1 eine Auskoppelfläche und der Wellenleiter L3 eine Einkoppelfläche.

In der Streulichtsonde 9 ist eine Strahlungsquelle 14 bzw. ein Emitter für elektromagnetische Strahlung vorgesehen. Die von der Strahlungsquelle 14 emittierte elektromagnetische Strahlung wird über eine Einkoppelfläche in den Wellenleiter L1 eingekoppelt, über welchen die elektromagnetische Strahlung zu der Auskoppelfläche des Wellenleiters L1 geführt wird. Die von der Strahlungsquelle 14 erzeugte elektromagnetische Strahlung wird somit als Strahlung S1 in die in dem Kristallisationsgefäß 1 befindliche Lösung eingestrahlt.

Der von der Strahlung S1 erzeugte Strahl besitzt beim Eintritt in die Lösung bzw. Suspension einen Querschnitt, der größer als 0,39 mm ist. Des Weiteren ist der Strahl mit einem Winkel von etwa +/- 12° divergent, d.h. der Öffnungswinkel des Strahls ist 24°.

Die Wellenleiter L1 und L3 sind parallel und flüssigkeitsdicht durch die Öffnung 13 der Streulichtsonde 9 durchgeführt. Sie sind insbesondere so ausgerichtet, dass die Richtung der in die Lösung bzw. Suspension eingestrahlten Strahlung S1 parallel zu der Detektionsrichtung für die Strahlung S2 ist, welche an den Kristallen gestreut wurde und welche in den Wellenleiter L3 eingekoppelt wird. Das Rohr 12 der Streulichtsonde 9 ist so in das Kristallisationsgefäß 1 eingetaucht, dass bei einer klaren Lösung, bei der sich keine Kristalle in der Lösung befinden, keine Strahlung in den Wellenleiter L3 gelangt, die eine Wellenlänge hat, in welcher die Strahlungsquelle 14 Strahlung emittiert, wenn über den Wellenleiter L1 Strahlung in die klare Lösung eingestrahlt wird.

Auch der Strahl der eingehenden Strahlung S2, welche in den Wellenleiter L3 eintritt, ist mit demselben Öffnungswinkel divergent, so dass der Sende- und Empfangsbereich der Streulichtsonde 9 räumlich überlappend ist. Es ergeben sich zwei nebeneinander liegende Kegel, die sich im Raum schneiden. Daraus ergibt sich ein sehr großes Messvolumen, welches insbesondere bei sehr niedrigen Partikelkonzentrationen wichtig ist.

Die Streulichtsonde 9 besitzt keine Scheibe als Abschluss zwischen den Wellenleitern L1 und L3 einerseits und der Lösung bzw. Suspension andererseits. Der optische Offset der Streulichtsonde 9 geht deshalb gegen Null.

Bei dem hier beschriebenen Ausführungsbeispiel erzeugt die Strahlungsquelle 14 Infrarotstrahlung in einem Wellenlängenbereich von 800 nm bis 900 nm. Die in die Lösung eingestrahlte elektromagnetische Strahlung wird an den Oberflächen von Kristallen gestreut, welche sich in der Lösung befinden. Ein Teil der an den Kristallen rückgestreuten elektromagnetischen Strahlung S2 wird über die Einkoppelfläche des Wellenleiters L3 von diesem zu einem Detektor 15 geführt. Der Detektor 15 ist so ausgebildet, dass er die Intensität der elektromagnetischen Strahlung in dem Wellenlängenbereich messen kann, in dem die Strahlungsquelle 14 elektromagnetische Strahlung emittiert.

Der Detektor 15 besitzt eine Empfangselektronik, die einen sehr weiten Verstärkungsbereich von 2 mW/V bis 20 picoW/V ermöglicht. Dies bedeutet, dass die Empfangselektronik bei einer einfallenden Lichtleistung von 20 picoW, d.h. bei einer Lichtleistung von etwa 150 µLux/cm², eine Spannung von 1 V liefert. Damit ist der Detektor 15 extrem sensitiv.

In dem Wellenleiter L1 ist ferner eine Abzweigung vorgesehen. Ein Teil der von der Strahlungsquelle 14 erzeugten und in den Wellenleiter L1 eingekoppelten Strahlung wird in einen Wellenleiter L2 abgezweigt und dem Detektor 15 zugeführt. Die über den Wellenleiter L2 abgezweigte und dem Detektor 15 zugeführte Strahlung dient als Referenzstrahlung.

In dem Detektor 15 wird ein Spannungswert erzeugt, welcher in einem direkten Zusammenhang mit der von den Kristallen in der Lösung rückgestreuten Lichtintensität steht. Der von dem Detektor 15 erzeugte Referenzspannungswert, der durch die Referenzstrahlung bewirkt wird, berücksichtigt dabei die Intensität der in die Lösung eingestrahlten Strahlung S1. Der Spannungswert des Detektors 15 wird unter Berücksichtigung des Referenzspannungswertes vom Detektor 15 in einer Auswerteeinheit korrigiert und an die Regeleinheit 10 übertragen.

Als Streulichtsonde 9 kann beispielsweise eine Variation der photometrischen Messeinrichtung verwendet werden, wie sie in der EP 0 472 899 A1 beschrieben ist. Die in dieser Schrift beschriebene Streulichtsonde kann sowohl für eine Transmissionsmessung als auch für eine Rückstreumessung verwendet werden. Im vorliegenden Fall würden die Rückstreumessungen berücksichtigt werden.

Bei einer weiteren Variante umfasst die Streulichtsonde 9 eine Stabsonde, die in das Kristallisationsgefäß 1 eingetaucht ist. Der Detektor 15 ist dann mit der Stabsonde über Wellenleiter verbunden und außerhalb des Kristallisationsgefäßes 1angeordnet.

Die Messung kann ferner auch mit einem Detektor 15 ohne Referenzierung der Strahlungsquelle 14 durchgeführt werden. Für die Langzeitstabilität der Messung ist die Referenzierung mit einem zweiten Detektor allerdings vorteilhaft. Die Korrektur des Streusignals, das von dem Detektor 15 detektiert wird, erfolgt dann anhand des Referenzsignals, das von einem weiteren Detektor in einer Auswerteeinheit erfasst wird, die dann ein korrigiertes Streusignal erzeugt und an die Regeleinheit 10 überträgt.

In der Stabsonde kann/können sowohl ein als auch mehrere Wellenleiter als Sender und Empfänger dienen. Die Fasergeometrie muss nicht zwangsläufig mit parallelen Sende- und Empfangsfasern realisiert werden, auch wenn dies bevorzugt ist. Weiterhin könnte auch eine Lösung mit Scheibe vor den Faserenden mit abweichender Geometrie eingesetzt werden, was jedoch dann aufgrund der internen Reflexionen zu einem deutlich höheren Signaloffset und damit zu einer deutlich geringeren Sensitivität des Messsystems insbesondere bei sehr niedrigen Partikelkonzentrationen führt.

Die Streulichtsonde 9 detektiert nicht die komplette gestreute Strahlung. Aufgrund von Mehrfachstreuung, Transmission und Absorption und aufgrund des räumlich begrenzten Empfangskegels (Apertur) erfasst die Streulichtsonde 9 nur einen zur Partikeloberfläche proportionalen Anteil der gestreuten Strahlung.

Im Folgenden werden weitere Details der Vorrichtung sowie ein Ausführungsbeispiel des erfindungsgemäßen Verfahrens im Detail erläutert:
Bei dem beschriebenen Ausführungsbeispiel soll der einleitend beschriebene Ligand la₂-3, der in Phenylcyclohexan gelöst ist, abgetrennt werden. Die Lösung wurde als Sumpfprodukt aus der Cyclisierung von Citronellal in Gegenwart eines (Bis(diarylphenoxy))-Aluminium-Katalysators gewonnen. Bei dieser Lösung ergibt sich das Problem, dass das zuvor durchgeführte komplexe chemische Verfahren dazu führt, dass die genaue Zusammensetzung der Lösung und die sich einstellenden Konzentrationen lösender und nicht lösender Nebenkomponenten nicht genau bekannt sind und damit die Sättigungstemperatur, bei welcher der Ligand kristallisiert, stark schwanken kann.

Erfindungsgemäß werden daher vorab Referenzmessungen durchgeführt. Die Referenzmessungen können vorteilhaft auch im Labor durchgeführt werden. Dabei wird die Lösung in das Kristallisationsgefäß 1 bei einer Temperatur eingeleitet, die einige 10 K unter der erwarteten Sättigungstemperatur liegt. Beispielsweise wird die Lösung mit einer Temperatur von 80 °C zugeführt. Diese Temperatur wird mittels der Regeleinheit 10, den Temperiereinrichtungen 5 und 7 und den Temperatursensoren 6 und 8 eingestellt. Bei dieser Temperatur ist eine sehr große Menge an Kristallen des Liganden in der Lösung. Allerdings ist die Kristallgröße und - morphologie der Kristalle für die spätere Filtration in der Abtrenneinheit 11 ungeeignet. Nun wird die Temperatur der Lösung, die in das Kristallisationsgefäß 1 eingeleitet wird, mittels der Temperiereinrichtung 5 angehoben. Gleichzeitig wird mittels der Streulichtsonde 9 elektromagnetische Strahlung in die Lösung eingestrahlt. Von der Regeleinheit 10 wird dann kontinuierlich die Temperatur der Lösung mittels des Temperatursensors 8 erfasst. Ferner wird die Intensität der rückgestreuten elektromagnetischen Strahlung anhand des von der Auswerteeinheit übertragenen Spannungswertes erfasst. Während des Anstiegs der Temperatur sinkt das Signal für die Intensität der rückgestreuten elektromagnetischen Strahlung, da die Kristalle sich auflösen und sich somit die für die Rückstreuung zur Verfügung stehende Kristalloberfläche verkleinert.

Durch die Referenzmessungen wird diejenige Intensität der detektierten elektromagnetischen Strahlung ermittelt, bei welcher eine Menge an Impfkristallen des Liganden bzw. eine Kristalloberfläche dieser Impfkristalle vorliegt, die ideal für ein anschließend durchgeführtes Kristallisationsverfahren ist, bei welchem die Lösung wieder abgekühlt wird und sich Kristalle bilden sollen, welche eine für die anschließende Abtrennung ideale Kristallgröße und -morphologie haben. Bei den Referenzmessungen wird die Temperatur der Lösung daher soweit angehoben, bis die Intensität der rückgestreuten elektromagnetischen Strahlung bis zu einem bestimmten Wert abgefallen ist. Danach wird auf an sich bekannte Weise ein Kühlkristallisationsverfahren begonnen, bei dem mit einer bestimmten Abkühlkurve die Lösung wieder abgekühlt wird, sodass sich Kristalle des Liganden bilden. Die Kristalle werden dann in der Abtrenneinheit 11 herausgefiltert, und es wird die Größe und Morphologie dieser Kristalle untersucht.

Die Referenzmessungen werden nun für eine Vielzahl von Intensitäten durchgeführt, bei denen das anschließende Kristallisationsverfahren auf immer gleiche Weise durchgeführt wird. Es wird dann diejenige Referenzmessung bestimmt, bei welcher die für die Abtrennung ideale Kristallgröße und -morphologie erzeugt worden sind. Die Intensität der rückgestreuten elektromagnetischen Strahlung zu Beginn des Kristallisationsverfahrens dieser Referenzmessung, d. h. die minimale Intensität der rückgestreuten elektromagnetischen Strahlung bei dieser Referenzmessung, wird als Soll-Intensität I_{S} definiert. Bei dieser Soll-Intensität I_{S} ist die Größe der Kristalloberfläche, die von den Impfkristallen des Liganden gebildet wird, ideal für das anschließend durchgeführte Kristallisationsverfahren.

Des Weiteren wird vorab ein Anfangstemperaturwert T_{A} festgelegt, bei dem die Lösung zu Beginn des Verfahrens in das Kristallisationsgefäß 1 eingeleitet wird. Dieser Anfangstemperaturwert T_{A} liegt deutlich unter dem Temperaturwert T_{K}, welcher der Soll-Intensität I_{S} entspricht, d. h. der Ausgangstemperatur für das Kristallisationsverfahren. Im vorliegenden Beispiel ist der Anfangstemperaturwert T_{A} etwa 90 °C. Dieser Anfangstemperaturwert T_{A} kann im Übrigen auch aus der Soll-Intensität I_{S} dadurch bestimmt werden, dass die dem Anfangstemperaturwert T_{A} zugeordnete Anfangsintensität I_{A} für die rückgestreute elektromagnetische Strahlung als die x-fache Intensität der Soll-Intensität I_{S} gewählt wird. Der Wert x kann dabei in einem Bereich von 1,2 bis 10 liegen. Im vorliegenden Fall liegt der Wert x bei 6,5.

Das Verfahren zum Abtrennen des Liganden aus der über die Zuleitung 2 zugeführten Lösung wird nach der Bestimmung der Soll-Intensität und des Anfangstemperaturwerts dann wie folgt durchgeführt:
Die Lösung wird über die Zuleitung 2 mit dem Anfangstemperaturwert T_{A} zugeführt. Sobald so viel Lösung in das Kristallisationsgefäß 1 eingeleitet worden ist, dass sich die Streulichtsonde 9 innerhalb der Lösung befindet, wird die Intensität I der an den Kristallen rückgestreuten elektromagnetischen Strahlung von der Regeleinheit 10 erfasst.

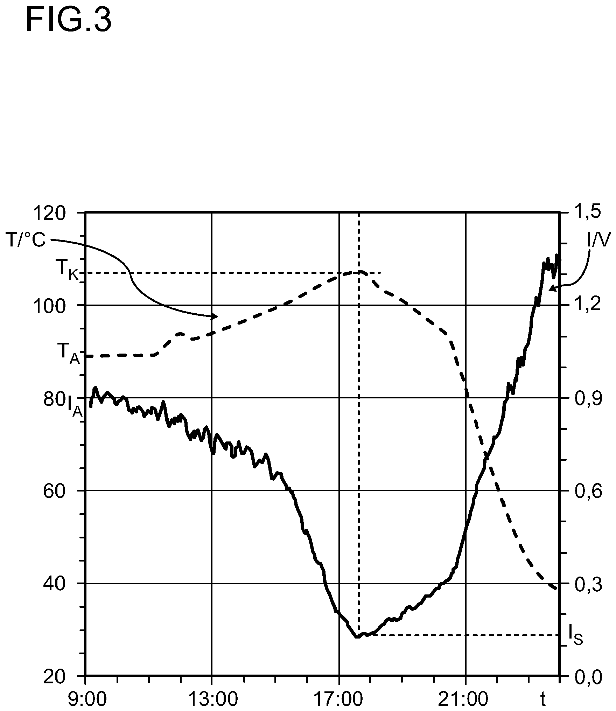

In Figur 3 ist der zeitliche Verlauf des Signals I der Streulichtsonde 9, welches mit der Intensität I der rückgestreuten elektromagnetischen Strahlung korreliert, sowie der zugehörige zeitliche Verlauf der Temperatur T der Lösung gezeigt. Der Anfangstemperaturwert T_{A} ist in diesem Fall 89,13 °C. Das zugehörige Signal IA der Streulichtsonde 9 ist 0,85 V. Im Folgenden wird begrifflich nicht zwischen dem Signal I der Streulichtsonde 9 und der Intensität I der rückgestreuten elektromagnetischen Strahlung unterschieden, da diese in einem direkten Zusammenhang stehen.

Mittels der Regeleinheit 10 wird nun die Temperatur der in das Kristallisationsgefäß 1 über die Zuleitung 2 eingeleiteten Lösung erhöht. Wie aus Figur 3 ersichtlich, steigt somit auch die Temperatur der Lösung innerhalb des Kristallisationsgefäßes 1 an. Gleichzeitig fällt das Signal I der Streulichtsonde 9 ab, da sich die Kristalle des Liganden auflösen. Die Temperatur der zugeführten Lösung wird so weit angehoben, bis das Signal I der Streulichtsonde 9 innerhalb eines Toleranzbereiches um die Soll-Intensität I_{S} liegt. Mit anderen Worten bedeutet dies, dass der Betrag der Differenz der detektierten Intensität I und der Soll-Intensität I_{S} kleiner als ein Grenzwert ist. Dieser Grenzwert kann beispielsweise 10% der Soll-Intensität I_{S} sein.

Im Idealfall ist bei der vollständigen Befüllung des Kristallisationsgefäßes 1 der Betrag der Differenz der detektierten Intensität I und der Soll-Intensität I_{S} kleiner als dieser Grenzwert. Falls dies nicht der Fall ist, wird über die Temperiereinrichtung 7 und die Regeleinheit 10 die Temperatur der Lösung, die sich innerhalb des Kristallisationsgefäßes 1 befindet, noch so feinjustiert, bis sich der Betrag dieser Differenz innerhalb dieses Grenzwertes befindet.

In diesem Zustand der in dem Kristallisationsgefäß 1 befindlichen Lösung ist die in den Referenzmessungen bestimmte ideale Menge an Impfkristallen des Liganden mit der idealen Kristalloberfläche vorhanden. Nun wird das eigentliche Kühlkristallisationsverfahren begonnen. Die Lösung wird, geregelt von der Regeleinheit 10, zunächst mit einer geringen Abkühlrate von etwa 3 K/h abgekühlt. Nach einer gewissen Zeit, d.h. wenn eine gewisse Menge an Kristallen einer bestimmten Größe vorliegt, kann die Abkühlrate beispielsweise bis auf etwa 20 K/h erhöht werden. Auf diese Weise bilden sich Kristalle des Liganden, die eine für die anschließende Abtrennung ideale Kristallgröße und -morphologie haben, in der kürzest möglichen Zeit. Die Suspension mit den Kristallen wird dann durch Öffnen des Ventils 4 über die Ableitung 3 der Abtrenneinheit 11 zugeführt, bei der die Suspension gefiltert wird und der Ligand der Formel la₂-3 als weißer Feststoff gewonnen werden kann.

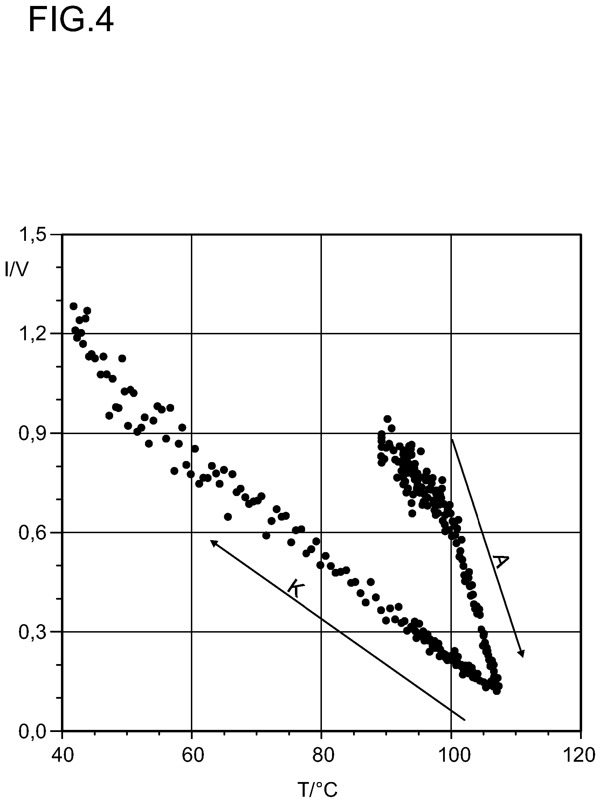

In Figur 4 ist der Zusammenhang zwischen der detektierten Intensität I der an den Kristallen gestreuten elektromagnetischen Strahlung und der Temperatur T gezeigt, und zwar für die in Figur 3 dargestellten Messwerte. Die Messwerte bei dem Pfeil A zeigen die Auflösung der Kristalle zu Beginn des Verfahrens, d.h. vor dem eigentlichen Kristallisationsverfahren, und die Messwerte entlang des Pfeils K zeigen die Kristallisation während des Kristallisationsverfahrens, das bei der Intensität I_{S} bzw. der Temperatur T_{K} begonnen wurde.

Es ergibt sich ein deutlicher Unterschied bei der Kurve für die Auflösung, d.h. beim Anheben der Temperatur bis zur Soll-Intensität Is, und der Kurve für die anschließende Kristallisation, d.h. beim Absenken der Temperatur beginnend mit dem Temperaturwert T_{K}. Bei derselben Temperatur ist die von der Streulichtsonde 9 gemessene Intensität während der Auflösung wesentlich höher als bei der Kristallisation. Während der Auflösung besitzen die Kristalle des Liganden somit eine größere spezifische Oberfläche. Dies bedeutet, dass sie sehr fein verteilt sind. Es handelt sich somit um kleine Kristalle. Dies ist für die anschließende Abtrennung der Kristalle unerwünscht. Bei der anschließenden Kristallisation ist die Intensität des von der Streulichtsonde 9 gemessenen Signals hingegen um den Faktor 2 bis 3 kleiner. Bei einer bestimmten Temperatur ist jedoch die gleiche Masse an Kristallen in Lösung. Die niedrigere Intensität der rückgestreuten Strahlung zeigt somit an, dass die spezifische Oberfläche der Kristalle kleiner ist. Daraus ergibt sich, dass die Kristalle größer sind, wie es für die anschließende Abtrennung der Kristalle erwünscht ist.

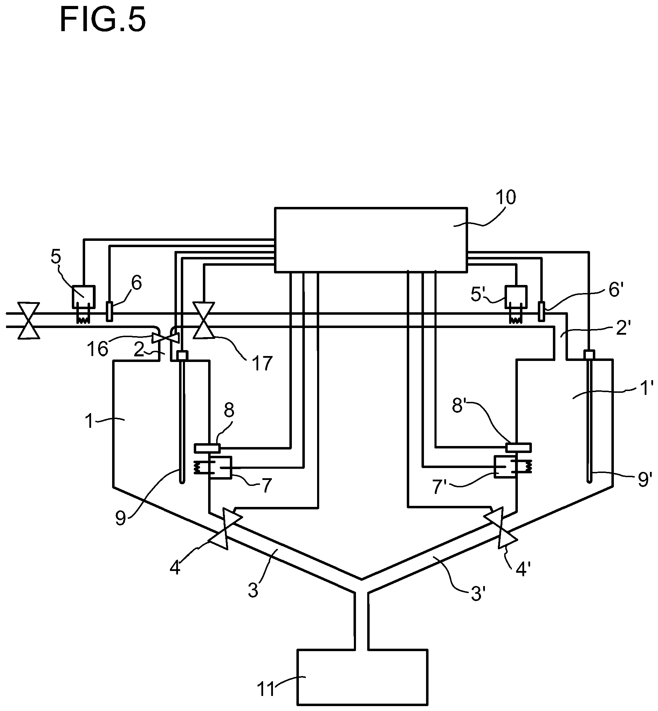

Im Folgenden wird ein zweites Beispiel einer Vorrichtung zum Abtrennen eines Stoffes aus einer Lösung und ein zweites Ausführungsbeispiel erfindungsgemäßen Verfahrens mit Bezug zu Figur 5 erläutert:
Die Vorrichtung des zweiten Beispiels umfasst die in Figur 1 gezeigte Vorrichtung des ersten Beispiels. Gleiche Teile sind daher mit den gleichen Bezugszeichen bezeichnet. Es wird entsprechend auf die vorstehende Beschreibung dieser Teile verwiesen. Die in Figur 5 gezeigte Vorrichtung des zweiten Beispiels weist jedoch ein weiteres Kristallisationsgefäß 1' auf. Wie das erste Kristallisationsgefäß 1 umfasst das zweite Kristallisationsgefäß 1' eine Zuleitung 2', eine Ableitung 3' mit einem Ventil 4'. In der Zuleitung 2' sind eine Temperiereinrichtung 5' und ein Temperatursensor 6' zum Regeln der Temperatur der Lösung vorgesehen, die in das zweite Kristallisationsgefäß 1' eingeleitet wird. Für das zweite Kristallisationsgefäß 1' sind eine Temperiereinrichtung 7' und ein Temperatursensor 8' sowie eine weitere Streulichtsonde 9' vorgesehen. Das Ventil 4', die Temperiereinrichtungen 5' und 7', die Temperatursensoren 6' und 8' sowie die Streulichtsonde 9' sind datentechnisch mit der Regeleinheit 10 gekoppelt.

Des Weiteren ist in der Zuleitung 2 für das erste Kristallisationsgefäß 1 ein elektronisch gesteuertes Ventil 16 angeordnet, gleichermaßen ist in der Zuleitung 2' für das zweite Kristallisationsgefäß 1' ein elektronisch ansteuerbares Ventil 17 angeordnet. Die Ventile 16 und 17 sind auch datentechnisch mit der Regeleinheit 10 gekoppelt.

Gemäß einem zweiten Ausführungsbeispiel des erfindungsgemäßen Verfahrens wird die in Figur 5 gezeigte Vorrichtung wie folgt betrieben:
Wie mit Bezug zu den Figuren 1 bis 3 erläutert, wird die Lösung über die Zuleitung 2 dem ersten Kristallisationsgefäß 1 zugeführt. In diesem Fall ist das Ventil 16 geöffnet und das Ventil 17 geschlossen, sodass keine Lösung in das zweite Kristallisationsgefäß 1' gelangt. Beim Zuführen der Lösung wird die Temperatur wie vorstehend erläutert geregelt, sodass die Temperatur der Lösung in dem ersten Kristallisationsgefäß 1, wenn dieses vollständig gefüllt ist, dem Temperaturwert T_{K} entspricht, welcher der Soll-Intensität I_{S} zugeordnet ist, bei welcher die gewünschte Menge an Impfkristallen vorliegt.

Anschließend wird das Ventil 16 geschlossen und in dem ersten Kristallisationsgefäß 1 beginnt die Kühlkristallisation, bei welcher die Temperatur der Lösung in dem ersten Kristallisationsgefäß 1 abgesenkt wird. Gleichzeitig wird mittels der Temperiereinrichtung 5 und dem Temperatursensor 6 die Temperatur der zuzuführenden Lösung wieder auf den Anfangstemperaturwert T_{A} gebracht. Anschließend wird das Ventil 17 geöffnet, sodass die Lösung dem zweiten Kristallisationsgefäß 1' zugeführt wird. Mittels der Temperiereinrichtung 5' und dem Temperatursensor 6' wird anschließend die Temperatur der dem zweiten Kristallisationsgefäß 1' zugeführten Lösung so geregelt, dass sich die von der Streulichtsonde 9' gemessene Intensität der rückgestreuten Strahlung der Soll-Intensität I_{S} annähert, wie dies bereits vorstehend für das erste Kristallisationsgefäß 1 beschrieben worden ist. Sobald der Betrag der Differenz der detektierten Intensität und der Soll-Intensität I_{S} kleiner als der Grenzwert ist, wird das Ventil 17 geschlossen und in dem zweiten Kristallisationsgefäß 1' wird das Kühlkristallisationsverfahren wie oben beschrieben durchgeführt, bei dem die Temperatur der Lösung verringert wird, sodass sich Kristalle des Liganden bilden. Während das Kristallisationsverfahren im zweiten Kristallisationsgefäß 1' durchgeführt wird, wird das Kristallisationsverfahren im ersten Kristallisationsgefäß 1 abgeschlossen und das Ventil 4 wird geöffnet, sodass die Suspension über die Ableitung 3 der Abtrenneinheit 11 zugeführt wird. In der Abtrenneinheit 11 werden die Kristalle des Liganden isoliert. Währenddessen kann das Ventil 4 wieder geschlossen werden, und die Lösung wird erneut dem ersten Kristallisationsgefäß 1 zugeführt.

Wenn das Kristallisationsverfahren in dem zweiten Kristallisationsgefäß 1' abgeschlossen ist, sind bereits die Kristalle aus der Suspension des ersten Kristallisationsgefäßes 1, welche über die Ableitung 3 der Abtrenneinheit 11 zugeführt worden sind, isoliert worden. Nun kann das Ventil 4' geöffnet werden, sodass die Suspension mit den Kristallen des Liganden aus dem zweiten Kristallisationsgefäß 1' über die Ableitung 3' der Abtrenneinheit 11 zugeführt werden kann. Dort werden dann die Kristalle des Liganden herausgefiltert.

Auf diese Weise kann mit der in Figur 5 gezeigten Vorrichtung das vorstehend mit Bezug zu Figur 1 beschriebene Verfahren wechselweise in den beiden Kristallisationsgefäßen 1 und 1' durchgeführt werden.

Im Folgenden wird ein Ausführungsbeispiel des Verfahrens zur Aufarbeitung eines Aluminium-haltigen Reaktionsprodukts aus der Herstellung von Isopulegol durch Cyclisierung von Citronellal beschrieben:
Es wird das Aluminium-haltige Reaktionsprodukt aufbereitet, wie es in der WO 2008/025852 A1 beschrieben ist. Im letzten Verfahrensschritt wird der Ligand der Formel la₂-3 gewonnen, wie es vorstehend mit Bezug zu den Figuren 1 bis 5 beschrieben worden ist.

Ein weiteres Ausführungsbeispiel der Erfindung betrifft ein Verfahren zur Herstellung von Isopulegol. Bei diesem Ausführungsbeispiel wird Isopulegol wie in der WO 2008/025852 A1 beschrieben hergestellt. Im Unterschied zu dem in dieser Schrift beschriebenen Verfahren wird jedoch der Ligand aus der organischen Phase nach einem Ausführungsbeispiel abgetrennt, wie es vorstehend mit Bezug zu den Figuren 1 bis 5 beschrieben worden ist.

Ein noch weiteres Ausführungsbeispiel betrifft ein Verfahren zur Herstellung von Menthol. In diesem Fall wird Isopulegol wie vorstehend beschrieben hergestellt. Menthol wird dann durch Hydrierung der ethylenischen Doppelbindung des so erhaltenen Isopulegols hergestellt.

### Bezugszeichenliste:

- 1, 1': Kristallisationsgefäß
- 2, 2': Zuleitung
- 3, 3': Ableitung
- 4, 4': Ventil
- 5, 5': Temperiereinrichtung
- 6, 6': Temperatursensor
- 7, 7': Temperiereinrichtung
- 8, 8': Temperatursensor
- 9, 9': Streulichtsonde
- 10: Regeleinheit
- 11: Abtrenneinheit
- 12: Rohr
- 14: Strahlungsquelle für elektromagnetische Strahlung
- 15: Detektor
- 16: Ventil
- 17: Ventil

## Patentansprüche

1. Verfahren zum Abtrennen eines Stoffes durch Kristallisation aus einer Lösung des Stoffes, bei dem
eine Suspension von Impfkristallen erzeugt und, wenn eine gewünschte Menge an Impfkristallen vorliegt, ein Kristallisationsverfahren begonnen wird, bei dem Kristalle des Stoffes gewonnen werden, die anschließend abgetrennt werden, wobei zum Erzeugen der gewünschten Menge an Impfkristallen:
elektromagnetische Strahlung in die Lösung eingestrahlt wird, wobei die in die Lösung eingestrahlte elektromagnetische Strahlung die Form eines Strahls aufweist, dessen Öffnungswinkel größer als 5 Grad ist, und im sichtbaren Spektralbereich liegt oder Infrarotstrahlung ist,
zum Einstellen einer gewünschten Menge an Impfkristallen eine Intensität der elektromagnetischen Strahlung, die von in der Lösung befindlichen Kristallen gestreut wurde, detektiert wird,
die detektierte Intensität mit einer Soll-Intensität (Is) verglichen wird,
die Temperatur der Lösung in Abhängigkeit von der Differenz der detektierten Intensität und der Soll-Intensität (Is) so geregelt wird, dass sich der Betrag dieser Differenz verkleinert,
wenn der Betrag der Differenz der detektierten Intensität und der Soll-Intensität (Is) kleiner als ein Grenzwert ist, die gewünschte Menge an Impfkristallen für das Kristallisationsverfahren vorliegt.

2. Verfahren nach Anspruch 1, **dadurch gekennzeichnet, dass** die Soll-Intensität (Iₛ) durch Referenzmessungen bestimmt wird, bei denen für die Lösung der Zusammenhang zwischen der Kristallgröße und/oder der Kristallmorphologie am Ende des Kristallisationsverfahrens und der detektierten Intensität zu Beginn des Kristallisationsverfahren bestimmt wird und hieraus die Soll-Intensität (Iₛ) als die Intensität gewählt wird, die der gewünschten Kristallgröße und/oder Kristallmorphologie zugeordnet ist.

3. Verfahren nach Anspruch 1 oder 2, **dadurch gekennzeichnet, dass** die Lösung oder ein Teil der Lösung in ein Kristallisationsgefäß bei einer Temperatur eingeleitet wird, die bei einem definierten Anfangstemperaturwert (T_{A}) liegt, der unterhalb der voraussichtlichen Sättigungstemperatur der Lösung liegt, und die Lösung dann erwärmt wird, bis der Betrag der Differenz der detektierten Intensität und der Soll-Intensität (Iₛ) kleiner als der Grenzwert ist.

4. Verfahren nach Anspruch 3, **dadurch gekennzeichnet, dass** der Anfangstemperaturwert (T_{A}) aus der Soll-Intensität (Iₛ) dadurch bestimmt wird, dass die dem Anfangstemperaturwert (T_{A}) zugeordnete Anfangsintensität (I_{A}) als die x-fache Intensität der Soll-Intensität (Iₛ) gewählt wird, wobei der Wert x in einem Bereich von 1,2 bis 10 liegt, und die Temperatur der Lösung so geregelt wird, bis die Intensität größer als die Anfangsintensität (I_{A}) ist, so dass zunächst eine ausreichend große Menge an Kristallen in der Lösung vorhanden ist, welche dann bei der Regelung sukzessive verringert wird, bis die Soll-Intensität für den Beginn des Kristallisationsverfahrens vorliegt.

5. Verfahren nach einem der vorhergehenden Ansprüche, **dadurch gekennzeichnet, dass** die elektromagnetische Strahlung eines Wellenlängenbereichs oder mehrerer Wellenlängenbereiche, der/die breiter als 20 nm ist/sind, in die Lösung eingestrahlt wird.

6. Verfahren nach einem der vorhergehenden Ansprüche, **dadurch gekennzeichnet, dass** die in die Lösung eingestrahlte elektromagnetische Strahlung die Form eines Strahls aufweist, dessen minimaler Querschnitt größer als 0,1 mm ist.

7. Verfahren nach einem der vorhergehenden Ansprüche, **dadurch gekennzeichnet, dass** die elektromagnetische Strahlung mittels einer Streulichtsonde (9) in die Lösung eingestrahlt wird und die Intensität der rückgestreuten elektromagnetischen Strahlung mittels der Streulichtsonde (9) detektiert wird.

8. Verfahren nach einem der vorhergehenden Ansprüche, **dadurch gekennzeichnet, dass** die Einstrahlrichtung der eingestrahlten elektromagnetischen Strahlung im Wesentlichen parallel zu der Detektionsrichtung ist, aus der die Intensität der rückgestreuten elektromagnetischen Strahlung detektiert wird.

9. Verfahren nach einem der Ansprüche 7 oder 8, **dadurch gekennzeichnet, dass**
die Lösung in ein Kristallisationsgefäß (1) mit einer Temperatur eingeleitet wird, die bei einem Anfangstemperaturwert (T_{A}) liegt,
wenn sich die Streulichtsonde (9) innerhalb der eingeleiteten Lösung befindet, die elektromagnetische Strahlung mittels der Streulichtsonde (9) in die Lösung eingestrahlt wird und die Intensität der elektromagnetischen Strahlung detektiert wird, die von den in der Lösung befindlichen Kristallen gestreut wurde,
die Temperatur der Lösung beim weiteren Einleiten der Lösung in das Kristallisationsgefäß (1) so geregelt wird, dass der Betrag der Differenz der detektierten Intensität und der Soll-Intensität (Iₛ) kleiner als der Grenzwert wird.

10. Verfahren nach einem der vorhergehenden Ansprüche, **dadurch gekennzeichnet, dass** der Stoff wenigstens einen Liganden der Formel (I), 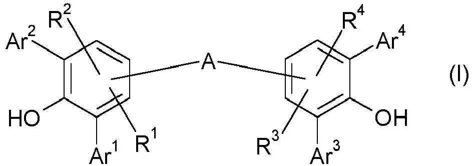 umfasst, wobei
Ar¹, Ar², Ar³, Ar⁴ unabhängig voneinander ausgewählt sind unter C₆-C₁₅-Arylresten oder C₂-C₁₅-Heteroarylresten, die gegebenenfalls jeweils 1 bis 7 gleiche oder verschiedene Substituenten, ausgewählt unter C₁-C₆-Alkyl, C₁-C₆-Perfluoralkyl, C₁-C₆-Alkoxy, C₇-C₁₂-Aralkyl, Halogen, SiR^{5a}R^{6a}R^{7a}, gegebenenfalls substituiertem C₆-C₁₀-Aryl, NR^{8a}R^{9a}, SR^{10a}, NO₂ tragen können,
R¹, R², R³, R⁴ unabhängig voneinander ausgewählt sind unter Wasserstoff, C₁-C₆-Alkyl, C₁-C₆-Perfluoralkyl, C₁-C₆-Alkoxy, C₇-C₁₂-Aralkyl, Halogen, SiR^{5b}R^{6b}R^{7b}, gegebenenfalls substituiertem C₆-C₁₀-Aryl, NR^{8b}R^{9b}, SR^{10b}, NO₂ und wobei
R¹ oder R² und/oder R³ oder R⁴ gemeinsam mit A einen aromatischen oder nicht-aromatischen Cyclus bilden kann, und
A für einen geradkettigen oder verzweigten und/oder cyclischen Kohlenwasserstoffrest mit 1 bis 25 C-Atomen steht, der gesättigt oder ein- oder mehrfach ungesättigt und/oder teilweise aromatisch sein kann und gegebenenfalls eines oder mehrere gleiche oder verschiedene Heteroatome, ausgewählt unter O, S, NR¹¹, und/oder eine oder mehrere gleiche oder verschiedene funktionelle Gruppen, ausgewählt unter den funktionellen Gruppen C(O), S(O), S(O)₂, aufweisen kann und gegebenenfalls einen oder mehrere gleiche oder verschiedene Substituenten, ausgewählt unter der Substituenten C₁-C₆-Alkyl, C₁-C₆-Perfluoralkyl, C₁-C₆-Alkoxy, C₁-C₁₀-Acyloxy, C₇-C₁₂-Aralkyl, Halogen, -SiR^{5c}R^{6c}R^{7c}, gegebenenfalls substituiertes C₆-C₁₀-Aryl, substituiertes oder unsubstituiertes C₂-C₁₀-Hetaryl, NR^{8c}R^{9c}, SR^{10c}, NO₂, C₁-C₁₂-Acyl, C₁-C₁₀-Carboxyl tragen kann, oder
für einen C₆-C₁₅-Arylrest oder einen C₂-C₁₅-Heteroarylrest steht, der gegebenenfalls jeweils 1 bis 5 Substituenten, ausgewählt unter C₁-C₆-Alkyl, C₁-C₆-Perfluoralkyl, C₁-C₆-Alkoxy, C₇-C₁₂-Aralkyl, Halogen, SiR^{5d}R^{6d}R^{7d}, substituiertes oder unsubstituiertes C₆-C₁₀-Aryl, NR^{8d}R^{9d}, SR^{10d}, NO₂ tragen können, oder
für eine funktionelle Gruppe oder ein Heteroatom ausgewählt aus der Gruppe -O-, -S-, -N(R¹¹)-, -S(O)-, -C(O)-, -S(O)₂-, -P(R¹¹)-, -(R¹¹)P(O)- und -Si(R¹²R¹³) steht,
wobei die Reste R^{5a}, R^{6a}, R^{7a}, R^{8a}, R^{9a}, R^{10a} bis R^{5d}, R^{6d}, R^{7d}, R^{8d}, R^{9d}, R^{10d}, R¹¹, R¹² und R¹³ jeweils unabhängig voneinander ausgewählt sind unter C₁-C₆-Alkyl, C₇-C₁₂-Aralkyl und/oder substituiertes oder unsubstituiertes C₆-C₁₀-Aryl und wobei die Reste R^{8a} und R^{9a}, R^{8b} und R^{9b}, R^{8c} und R^{9c}, R^{8d} und R^{9d} unabhängig voneinander jeweils gemeinsam auch einen cyclischen Kohlenwasserstoffrest mit 2 bis 8 Kohlenstoffatomen bilden können, der eines oder mehrere gleiche oder verschieden Heteroatome ausgewählt aus der Gruppe O, S, NR^{11a} aufweisen kann und R^{11a} die für R¹¹ angegebenen Bedeutungen haben kann,
in freier und/oder komplexgebundener Form.

11. Verfahren nach Anspruch 10, **dadurch gekennzeichnet, dass** die Lösung oder ein Teil der Lösung in einem Kristallisationsgefäß (1) auf eine Temperatur gebracht wird, die geringer als 95°C ist.

12. Verfahren zum Gewinnen eines Stoffes aus einer Lösung mittels Kristallisation,
bei dem die Lösung in ein erstes Kristallisationsgefäß (1) eingeleitet wird und der Stoff mittels Kristallisation in dem ersten Kristallisationsgefäß (1) nach dem Verfahren gemäß einem der Ansprüche 1 bis 11 abgetrennt wird und
bei dem während der Durchführung des Kristallisationsverfahrens in dem ersten Kristallisationsgefäß (1) die Lösung in ein zweites Kristallisationsgefäß (1') eingeleitet wird und der Stoff mittels Kristallisation in dem zweiten Kristallisationsgefäß (1') nach dem Verfahren gemäß einem der Ansprüche 1 bis 11 abgetrennt wird.

13. Verfahren zur Aufarbeitung eines Aluminium-haltigen Reaktionsprodukts aus der Herstellung von Isopulegol durch Cyclisierung von Citronellal, enthaltend
i) Isopulegol,
ii) wenigstens einen Liganden der Formel (I), 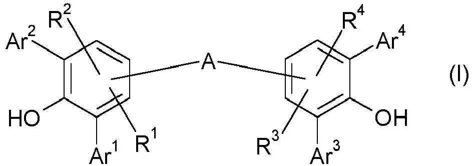 wobei
Ar¹, Ar², Ar³, Ar⁴ unabhängig voneinander ausgewählt sind unter C₆-C₁₅-Arylresten oder C₂-C₁₅-Heteroarylresten, die gegebenenfalls jeweils 1 bis 7 gleiche oder verschiedene Substituenten, ausgewählt unter C₁-C₆-Alkyl, C₁-C₆-Perfluoralkyl, C₁-C₆-Alkoxy, C₇-C₁₂-Aralkyl, Halogen, SiR^{5a}R^{6a}R^{7a}, gegebenenfalls substituiertem C₆-C₁₀-Aryl, NR^{8a}R^{9a}, SR^{10a}, NO₂ tragen können,
R¹, R², R³, R⁴ unabhängig voneinander ausgewählt sind unter Wasserstoff, C₁-C₆-Alkyl, C₁-C₆-Perfluoralkyl, C₁-C₆-Alkoxy, C₇-C₁₂-Aralkyl, Halogen, SiR^{5b}R^{6b}R^{7b}, gegebenenfalls substituiertem C₆-C₁₀-Aryl, NR^{8b}R^{9b}, SR^{10b}, NO₂ und wobei
R¹ oder R² und/oder R³ oder R⁴ gemeinsam mit A einen aromatischen oder nicht-aromatischen Cyclus bilden kann, und
A für einen geradkettigen oder verzweigten und/oder cyclischen Kohlenwasserstoffrest mit 1 bis 25 C-Atomen steht, der gesättigt oder ein- oder mehrfach ungesättigt und/oder teilweise aromatisch sein kann und gegebenenfalls eines oder mehrere gleiche oder verschiedene Heteroatome, ausgewählt unter O, S, NR¹¹, und/oder eine oder mehrere gleiche oder verschiedene funktionelle Gruppen, ausgewählt unter den funktionellen Gruppen C(O), S(O), S(O)₂, aufweisen kann und gegebenenfalls einen oder mehrere gleiche oder verschiedene Substituenten, ausgewählt unter der Substituenten C₁-C₆-Alkyl, C₁-C₆-Perfluoralkyl, C₁-C₆-Alkoxy, C₁-C₁₀-Acyloxy, C₇-C₁₂-Aralkyl, Halogen, -SiR^{5c}R^{6c}R^{7c}, gegebenenfalls substituiertes C₆-C₁₀-Aryl, substituiertes oder unsubstituiertes C₂-C₁₀-Hetaryl, NR^{8c}R^{9c}, SR^{10c}, NO₂, C₁-C₁₂-Acyl, C₁-C₁₀-Carboxyl tragen kann, oder
für einen C₆-C₁₅-Arylrest oder einen C₂-C₁₅-Heteroarylrest steht, der gegebenenfalls jeweils 1 bis 5 Substituenten, ausgewählt unter C₁-C₆-Alkyl, C₁-C₆-Perfluoralkyl, C₁-C₆-Alkoxy, C₇-C₁₂-Aralkyl, Halogen, SiR^{5d}R^{6d}R^{7d}, substituiertes oder unsubstituiertes C₆-C₁₀-Aryl, NR^{8d}R^{9d}, SR^{10d}, NO₂ tragen können, oder
für eine funktionelle Gruppe oder ein Heteroatom ausgewählt aus der Gruppe -O-, -S-, -N(R¹¹)-, -S(O)-, -C(O)-, -S(O)₂-, -P(R¹¹)-, -(R¹¹)P(O)- und -Si(R¹²R¹³) steht,
wobei die Reste R^{5a}, R^{6a}, R^{7a}, R^{8a}, R^{9a}, R^{10a} bis R^{5d}, R^{6d}, R^{7d}, R^{8d}, R^{9d}, R^{10d}, R¹¹, R¹² und R¹³ jeweils unabhängig voneinander ausgewählt sind unter C₁-C₆-Alkyl, C₇-C₁₂-Aralkyl und/oder substituiertes oder unsubstituiertes C₆-C₁₀-Aryl und wobei die Reste R^{8a} und R^{9a}, R^{8b} und R^{9b}, R^{8c} und R^{9c}, R^{8d} und R^{9d} unabhängig voneinander jeweils gemeinsam auch einen cyclischen Kohlenwasserstoffrest mit 2 bis 8 Kohlenstoffatomen bilden können, der eines oder mehrere gleiche oder verschieden Heteroatome ausgewählt aus der Gruppe O, S, NR^{11a} aufweisen kann und R^{11a} die für R¹¹ angegebenen Bedeutungen haben kann,
in freier und/oder komplexgebundener Form,
bei dem man
a) das Reaktionsprodukt einer destillativen Auftrennung unter Erhalt eines an Isopulegol angereicherten Kopfprodukts und eines an Isopulegol abgereicherten Sumpfprodukts unterzieht,
b) das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt bringt,
c) den Liganden der Formel (I) aus der organischen Phase nach dem Verfahren nach einem der Ansprüche 1 bis 11 abtrennt.

14. Verfahren zur Herstellung von Isopulegol der Formel (IV) 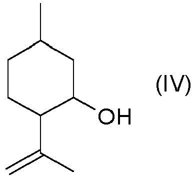 umfassend
α) die Cyclisierung von Citronellal der Formel (V) 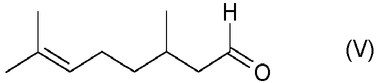 in Gegenwart eines Katalysators, der erhältlich ist durch Umsetzung eines Bis(diarylphenol)-Liganden der Formel (I), wie in dem Anspruch 10 definiert,
mit einer Aluminium-Verbindung der Formel (II),
(R¹⁴)₃₋ₚAlHₚ (II)
wobei
Al Aluminium bedeutet,
R¹⁴ einen verzweigten oder unverzweigten Alkylrest mit 1 bis 5 Kohlenstoffatomen und
p für 0 oder eine ganze Zahl von 1 bis 3 steht,
und/oder
mit einer Aluminium-Verbindung der Formel (III),
MAlH₄ (III)
wobei
Al Aluminium bedeutet und
M Lithium, Natrium oder Kalium bedeutet,
β) die Rückgewinnung des Bis(diarylphenol)-Liganden der Formel (I) nach erfolgter Umsetzung, indem man
a) das in Schritt α) erhaltene Reaktionsprodukt einer destillativen Auftrennung unter Erhalt eines an Isopulegol angereicherten Kopfprodukts und eines an Isopulegol abgereicherten Sumpfprodukts unterzieht,
b) das an Isopulegol abgereicherte Sumpfprodukts mit einer wässrigen Base unter erhalt einer Aluminium-haltigen wässrigen Phase und einer die Hauptmenge der Liganden der Formel (I) enthaltenden organischen Phase in innigen Kontakt bringt und
c) den Liganden der Formel (I) aus der organischen Phase nach dem Verfahren gemäß einem der Ansprüche 1 bis 11 abtrennt.

15. Verfahren zur Herstellung von Menthol umfassend die Schritte:
A) Herstellung von Isopulegol der Formel (IV) gemäß Anspruch 14 und
B) Hydrierung der ethylenischen Doppelbindung des so erhaltenen Isopulegols.

## Claims

1. A method for separating off a substance by crystallization from a solution of the substance, in which
a suspension of seed crystals is produced and, if a desired amount of seed crystals is present, a crystallization method is started, in which crystals of the substance are obtained which are then separated off, where, for producing the desired amount of seed crystals:
electromagnetic radiation is radiated into the solution, where the electromagnetic radiation radiated into the solution has the form of a beam, the aperture angle of which is greater than 5 degrees, and is in the visible spectral range or is infrared radiation,
for establishing a desired amount of seed crystals an intensity of the electromagnetic radiation which has been scattered by crystals located in the solution is detected,
the detected intensity is compared with a desired intensity (Iₛ),
the temperature of the solution is regulated depending on the difference between the detected intensity and the desired intensity (Iₛ) in such a way that the amount of this difference is reduced,
if the amount of the difference between the detected intensity and the desired intensity (Iₛ) is less than a limiting value, the desired amount of seed crystals for the crystallization method is present.

2. The method according to claim 1, wherein the desired intensity (Iₛ) is determined by reference measurements in which, for the solution, the relationship between the crystal size and/or the crystal morphology at the end of the crystallization method and the detected intensity at the start of the crystallization method is determined and from this the desired intensity (Iₛ) is selected as the intensity which is assigned to the desired crystal size and/or crystal morphology.

3. The method according to claim 1 or 2, wherein the solution or some of the solution is introduced into a crystallization vessel at a temperature which is at a defined starting temperature value (T_{A}), which is below the anticipated saturation temperature of the solution, and the solution is then heated until the amount of the difference between the detected intensity and the desired intensity (Iₛ) is less than the limiting value.

4. The method according to claim 3, wherein the starting temperature value (T_{A}) is determined from the desired intensity (Iₛ) by selecting the starting intensity (I_{A}) assigned to the starting temperature value (T_{A}) as the x-fold intensity of the desired intensity (Iₛ), where the value x is in a range from 1.2 to 10, and the temperature of the solution is regulated in such a way until the intensity is greater than the starting intensity (I_{A}), such that initially a sufficiently large amount of crystals is present in the solution which is then gradually reduced under regulation until the desired intensity for the start of the crystallization method is present.

5. The method according to any of the preceding claims, wherein the electromagnetic radiation of one wavelength range or two or more wavelength ranges which is/are wider than 20 nm is radiated into the solution.

6. The method according to any of the preceding claims, wherein the electromagnetic radiation radiated into the solution has the form of a beam, the minimum cross section of which is greater than 0.1 mm.

7. The method according to any of the preceding claims, wherein the electromagnetic radiation is radiated into the solution by means of a scattered-light probe (9) and the intensity of the back-scattered electromagnetic radiation is detected by means of the scattered-light probe (9).

8. The method according to any of the preceding claims, wherein the incident direction of the radiated electromagnetic radiation is essentially parallel to the detection direction, from which the intensity of the back-scattered electromagnetic radiation is detected.

9. The method according to either of claims 7 or 8, wherein
the solution is introduced into a crystallization vessel (1) at a temperature which is at a starting temperature value (T_{A}),
if the scattered-light probe (9) is located within the introduced solution, the electromagnetic radiation is radiated into the solution by means of the scattered-light probe (9) and the intensity of the electromagnetic radiation which has been scattered by the crystals located in the solution is detected,
the temperature of the solution upon further introduction of the solution into the crystallization vessel (1) is regulated such that the amount of the difference between the detected intensity and the desired intensity (Iₛ) is less than the limiting value.

10. The method according to any of the preceding claims, wherein the substance comprises at least one ligand of the formula (I) 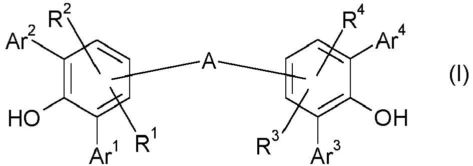 where
Ar¹, Ar², Ar³, Ar⁴, independently of one another, are chosen from C₆-C₁₅-aryl radicals or C₂-C₁₅-heteroaryl radicals, which may in each case optionally bear 1 to 7 identical or different substituents chosen from C₁-C₆-alkyl, C₁-C₆-perfluoroalkyl, C₁-C₆-alkoxy, C₇-C₁₂-aralkyl, halogen, SiR^{5a}R^{6a}R^{7a}, optionally substituted C₆-C₁₀-aryl, NR^{8a}R^{9a}, SR^{10a}, NO₂,
R¹, R², R³, R⁴, independently of one another, are chosen from hydrogen, C₁-C₆-alkyl, C₁-C₆-perfluoroalkyl, C₁-C₆-alkoxy, C₇-C₁₂-aralkyl, halogen, SiR^{5b}R^{6b}R^{7b}, optionally substituted C₆-C₁₀-aryl, NR^{8b}R^{9b}, SR^{10b}, NO₂ and where R¹ or R² and/or R³ or R⁴, together with A, can form an aromatic or non-aromatic cycle, and
A is a straight-chain or branched and/or cyclic hydrocarbon radical having 1 to 25 carbon atoms which may be saturated or mono- or polyunsaturated and/or partially aromatic and may optionally have one or more identical or different heteroatoms chosen from O, S, NR¹¹, and/or one or more identical or different functional groups chosen from the functional groups C(O), S(O), S(O)₂ and may optionally bear one or more identical or different substituents chosen from the substituents C₁-C₆-alkyl, C₁-C₆-perfluoroalkyl, C₁-C₆-alkoxy, C₁-C₁₀-acyloxy, C₇-C₁₂-aralkyl, halogen, -SiR^{5c}R^{6c}R^{7c}, optionally substituted C₆-C₁₀-aryl, substituted or unsubstituted C₂-C₁₀-hetaryl, NR^{8c}R^{9c}, SR^{10c}, NO₂, C₁-C₁₂-acyl, C₁-C₁₀-carboxyl, or
is a C₆-C₁₅-aryl radical or a C₂-C₁₅-heteroaryl radical which may optionally in each case bear 1 to 5 substituents chosen from C₁-C₆-alkyl, C₁-C₆-perfluoroalkyl, C₁-C₆-alkoxy, C₇-C₁₂-aralkyl, halogen, SiR^{5d}R^{6d}R^{7d}, substituted or unsubstituted C₆-C₁₀-aryl, NR^{8d}R^{9d}, SR^{10d}, NO₂, or
is a functional group or a heteroatom chosen from the group -O-, -S-, -N(R¹¹)-, -S(O)-, - C(O)-, -S(O)₂-, -P(R¹¹)-, -(R¹¹)P(O)- and -Si(R¹²R¹³),
where the radicals R^{5a}, R^{6a}, R^{7a}, R^{8a}, R^{9a}, R^{10a} to R^{5d}, R^{6d}, R^{7d}, R^{8d}, R^{9d}, R^{10d}, R¹¹, R¹² and R¹³ are in each case independently of one another chosen from C₁-C₆-alkyl, C₇-C₁₂-aralkyl and/or substituted or unsubstituted C₆-C₁₀-aryl and where the radicals R^{8a} and R^{9a}, R^{8b} and R^{9b}, R^{8c} and R^{9c}, R^{8d} and R^{9d}, independently of one another, may in each case together also form a cyclic hydrocarbon radical having 2 to 8 carbon atoms which may have one or more identical or different heteroatoms chosen from the group O, S, NR^{11a}, and R^{11a} can have the meanings given for R¹¹,
in free and/or complex-bound form.

11. The method according to claim 10, wherein the solution or some of the solution is brought in a crystallization vessel (1) to a temperature which is less than 95°C.

12. A method for obtaining a substance from a solution by means of crystallization,
in which the solution is introduced into a first crystallization vessel (1) and the substance is separated off by means of crystallization in the first crystallization vessel (1) by the method according to any of claims 1 to 11 and
in which, while carrying out the crystallization method in the first crystallization vessel (1), the solution is introduced into a second crystallization vessel (1') and the substance is separated off by means of crystallization in the second crystallization vessel (1') by the method according to any of claims 1 to 11.

13. A method for working up an aluminum-containing reaction product from the production of isopulegol by cyclizing citronellal, comprising
i) isopulegol,
ii) at least one ligand of the formula (I), 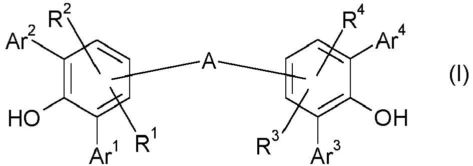 where
Ar¹, Ar², Ar³, Ar⁴, independently of one another, are chosen from C₆-C₁₅-aryl radicals or C₂-C₁₅-heteroaryl radicals, which may optionally in each case bear 1 to 7 identical or different substituents chosen from C₁-C₆-alkyl, C₁-C₆-perfluoroalkyl, C₁-C₆-alkoxy, C₇-C₁₂-aralkyl, halogen, SiR^{5a}R^{6a}R^{7a}, optionally substituted C₆-C₁₀-aryl, NR^{8a}R^{9a}, SR^{10a}, NO₂,
R¹, R², R³, R⁴, independently of one another, are chosen from hydrogen, C₁-C₆-alkyl, C₁-C₆-perfluoroalkyl, C₁-C₆-alkoxy, C₇-C₁₂-aralkyl, halogen, SiR^{5b}R^{6b}R^{7b}, optionally substituted C₆-C₁₀-aryl, NR^{8b}R^{9b}, SR^{10b}, NO₂ and where R¹ or R² and/or R³ or R⁴, together with A, may form an aromatic or non-aromatic cycle, and
A is a straight-chain or branched and/or cyclic hydrocarbon radical having 1 to 25 carbon atoms which may be saturated or mono- or polyunsaturated and/or partially aromatic and may optionally have one or more identical or different heteroatoms chosen from O, S, NR¹¹, and/or one or more identical or different functional groups chosen from the functional groups C(O), S(O), S(O)₂ and may optionally bear one or more identical or different substituents chosen from the substituents C₁-C₆-alkyl, C₁-C₆-perfluoroalkyl, C₁-C₆-alkoxy, C₁-C₁₀-acyloxy, C₇-C₁₂-aralkyl, halogen, -SiR^{5c}R^{6c}R^{7c}, optionally substituted C₆-C₁₀-aryl, substituted or unsubstituted C₂-C₁₀-hetaryl, NR^{8c}R^{9c}, SR^{10c}, NO_{2,} C₁₋C₁₂-acyl, C₁-C₁₀-carboxyl, or
is a C₆-C₁₅-aryl radical or a C₂-C₁₅-heteroaryl radical which may optionally in each case bear 1 to 5 substituents chosen from C₁-C₆-alkyl, C₁-C₆-perfluoroalkyl, C₁-C₆-alkoxy, C₇-C₁₂-aralkyl, halogen, SiR^{5d}R^{6d}R^{7d}, substituted or unsubstituted C₆-C₁₀-aryl, NR^{8d}R^{9d}, SR^{10d}, NO_{2,} or
is a functional group or a heteroatom chosen from the group -O-, -S-, -N(R¹¹)-, -S(O)-, - C(O)-, -S(O)₂-, -P(R¹¹)-, -(R¹¹)P(O)- and -Si(R¹²R¹³),
where the radicals R^{5a}, R^{6a}, R^{7a}, R^{8a}, R^{9a}, R^{10a} to R^{5d}, R^{6d}, R^{7d}, R^{8d}, R^{9d}, R^{10d}, R¹¹, R¹² and R¹³ are in each case independently of one another chosen from C₁-C₆-alkyl, C₇-C₁₂-aralkyl and/or substituted or unsubstituted C₆-C₁₀-aryl and where the radicals R^{8a} and R^{9a}, R^{8b} and R^{9b}, R^{8c} and R^{9c}, R^{8d} and R^{9d}, independently of one another, may in each case together also form a cyclic hydrocarbon radical having 2 to 8 carbon atoms which may have one or more identical or different heteroatoms chosen from the group O, S, NR^{11a}, and R^{11a} can have the meanings given for R¹¹,
in free and/or complex-bound form,
in which
a) the reaction product is subjected to distillative separation to obtain an isopulegol-enriched top product and an isopulegol-depleted bottom product,
b) the isopulegol-depleted bottom product is brought into close contact with an aqueous base to give an aluminum-containing aqueous phase and an organic phase comprising the majority of the ligands of the formula (I),
c) the ligand of the formula (I) is separated off from the organic phase by the method according to any of claims 1 to 11.

14. A method for producing isopulegol of the formula (IV) 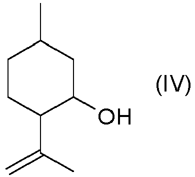 comprising
α) the cyclization of citronellal of the formula (V) 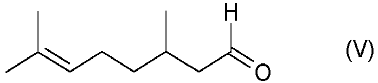 in the presence of a catalyst which is obtainable by reacting a bis(diarylphenol) ligand of the formula (I) as defined in claim 10,
with an aluminum compound of the formula (II),
(R14)₃₋ₚAlHₚ (II)
where
Al is aluminum,
R¹⁴ is a branched or unbranched alkyl radical having 1 to 5 carbon atoms and
p is 0 or an integer from 1 to 3,
and/or
with an aluminum compound of the formula (III),
MAlH₄ (III)
where
Al is aluminum and
M is lithium, sodium or potassium,
β) the recovery of the bis(diarylphenol) ligand of the formula (I) after the reaction has taken place by
a) subjecting the reaction product obtained in step α) to a distillative separation to obtain an isopulegol-enriched top product and an isopulegol-depleted bottom product,
b) bringing the isopulegol-depleted bottom product into close contact with an aqueous base to give an aluminum-containing aqueous phase and an organic phase comprising the majority of the ligands of the formula (I) and
c) separating off the ligand of the formula (I) from the organic phase by the method according to any of claims 1 to 11.

15. A method for producing menthol comprising the steps:
A) production of isopulegol of the formula (IV) according to claim 14 and
B) hydrogenation of the ethylenic double bond of the isopulegol obtained in this way.

## Revendications

1. Procédé pour la séparation d'une substance par cristallisation à partir d'une solution de la substance, dans lequel
une suspension de cristaux germes est produite et, lorsqu'une quantité désirée de cristaux germes est pré6sente, un processus de cristallisation est commencé, dans lequel on obtient des cristaux de la substance, qui sont ensuite séparés, dans lequel pour produire la quantité désirée de cristaux germes :
on irradie la solution avec un rayonnement magnétique, le rayonnement électromagnétique envoyé dans la solution ayant la forme d'un faisceau dont l'angle de divergence est supérieur à 5 grades, et se situe dans la région visible du spectre ou est un rayonnement infrarouge,
pour l'ajustement d'une quantité désirée de cristaux germes on détecte une intensité du rayonnement électromagnétique qui a été diffusé par les cristaux présents dans la solution,
on compare l'intensité détecté à une intensité à atteindre (Iₛ),
on règle la température de la solution en fonction de la différence entre l'intensité détectée et l'intensité à atteindre (Iₛ), de manière que la valeur de cette différence diminue,
lorsque la valeur de la différence entre l'intensité détectée et l'intensité à atteindre (Iₛ) est inférieure à une valeur limite, la quantité désirée de cristaux germes pour le processus de cristallisation est présente.

2. Procédé selon la revendication 1, **caractérisé en ce qu'**on détermine l'intensité à atteindre (Iₛ) par des mesures de référence dans lesquelles on détermine pour la solution la relation entre la taille des cristaux et/ou la morphologie des cristaux à la fin du processus de cristallisation et l'intensité détectée au début du processus de cristallisation et à partir de cette relation on choisit l'intensité à atteindre (Iₛ) en tant que l'intensité qui est affectée à la taille de cristaux désirée et/ou à la morphologie désirée des cristaux.

3. Procédé selon la revendication 1 ou 2, **caractérisé en ce qu'**on fait passer dans un récipient de cristallisation la solution ou une partie de la solution à une température qui se situe à une valeur de température initiale (T_{A}) définie, qui est inférieure à la température de saturation probable de la solution, et on chauffe ensuite la solution jusqu'à ce que la valeur de la différence entre l'intensité détectée et l'intensité à atteindre (Iₛ) soit inférieure à la valeur limite.

4. Procédé selon la revendication 3, **caractérisé en ce qu'**on détermine la température initiale (T_{A}) à partir de l'intensité à atteindre (Iₛ) en choisissant l'intensité initiale (I_{A}) affectée à la valeur de température initiale (T_{A}) en tant que l'intensité représentant x fois l'intensité à atteindre (Iₛ), la valeur x se situant dans une plage de 1,2 à 10, et on règle la température de la solution jusqu'à ce que l'intensité soit supérieure à l'intensité initiale (I_{A}), de telle façon que dans la solution est d'abord présente une quantité suffisamment grande de cristaux, qui est ensuite réduite successivement au cours du réglage, jusqu'à ce l'intensité à atteindre soit présente pour le début du processus de cristallisation.

5. Procédé selon l'une quelconque des revendications précédentes, **caractérisé en ce qu'**on irradie la solution avec le rayonnement magnétique d'une gamme de longueurs d'onde ou de plusieurs gammes de longueurs d'onde qui est/sont supérieure(s) à 20 nm.

6. Procédé selon l'une quelconque des revendications précédentes, **caractérisé en ce que** le rayonnement électromagnétique envoyé dans la solution a la forme d'un faisceau dont la section transversale minimale est supérieure à 0,1 mm.

7. Procédé selon l'une quelconque des revendications précédentes, **caractérisé en ce qu'**on envoie dans la solution le rayonnement électromagnétique au moyen d'une sonde de mesure de lumière diffusée (9) et on détecte au moyen de la sonde de mesure de lumière diffusée (9) l'intensité du rayonnement électromagnétique rétrodiffusé.

8. Procédé selon l'une quelconque des revendications précédentes, **caractérisé en ce que** la direction d'incidence du rayonnement électromagnétique incident est essentiellement parallèle à la direction de la détection dans laquelle on détecte l'intensité du rayonnement électromagnétique rétrodiffusé.

9. Procédé selon l'une quelconque des revendications 7 et 8, **caractérisé en ce**
**qu'**on fait passer la solution dans un récipient de cristallisation (1) à une température qui se situe à une valeur de température initiale (T_{A}),
lorsque la sonde de mesure de lumière diffusée (9) se trouve à l'intérieur de la solution introduite, on envoie dans la solution le rayonnement électromagnétique au moyen de la sonde de mesure de lumière diffusée (9) et on détecte l'intensité du rayonnement électromagnétique qui a été diffusé par les cristaux présents dans la solution,
on règle la température de la solution en continuant à faire passer la solution dans le récipient de cristallisation (1), de manière que la valeur de la différence entre l'intensité détectée et l'intensité à atteindre (Iₛ) soit inférieure à la valeur limite.

10. Procédé selon l'une quelconque des revendications précédentes, **caractérisé en ce que** la substance comprend au moins un ligand de formule (I), 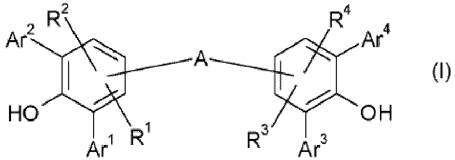 dans laquelle
Ar¹, Ar², Ar³, Ar⁴ sont choisis indépendamment les uns des autres parmi des radicaux aryle en C₆-C₁₅ ou des radicaux hétéroaryle en C₂-C₁₅, qui peuvent éventuellement porter chacun 1 à 7 substituants identiques ou différents, choisis parmi alkyle en C₁-C₆, perfluoroalkyle en C₁-C₆, alcoxy en C₁-C₆, aralkyle en C₇-C₁₂, halogéno, SiR^{5a}R^{6a}R^{7a}, aryle en C₆-C₁₀ éventuellement substitué, NR^{8a}R^{9a}, SR^{10a}, NO₂,
R¹, R², R³, R⁴ sont choisis indépendamment les uns des autres parmi un atome d'hydrogène, des groupes alkyle en C₁-C₆, perfluoroalkyle en C₁-C₆, alcoxy en C₁-C₆, aralkyle en C₇-C₁₂, des atomes d'halogène, des groupes SiR^{5b}R^{6b}R^{7b}, aryle en C₆-C₁₀ éventuellement substitué, NR^{8b}R^{9b}, SR^{10b}, NO₂ et R¹ ou R² et/ou R³ ou R⁴ pouvant former conjointement avec A un cycle aromatique ou non aromatique, et
A représente un radical hydrocarboné à chaîne droite ou ramifié et/ou cyclique ayant de 1 à 25 atomes de carbone, qui peut être saturé ou une ou plusieurs fois insaturé et/ou partiellement aromatique et peut éventuellement comporter un ou plusieurs hétéroatomes identiques ou différents, choisis parmi O, S, NR¹¹, et/ou un ou plusieurs groupes fonctionnels identiques ou différents, choisis parmi les groupes fonctionnels C(O), S(O), S(O)₂ et peut éventuellement porter un ou plusieurs substituants identiques ou différents, choisis parmi les substituants alkyle en C₁-C₆, perfluoroalkyle en C₁-C_{6,} alcoxy en C₁-C₆, acyloxy en Ci-Cio, aralkyle en C₇-C₁₂, halogéno, -SiR^{5c}R^{5c}R^{7c}, aryle en C₆-C₁₀ éventuellement substitué, hétéroaryle en C₂-C₁₀ substitué ou non substitué, NR^{8c}R^{9c}, SR^{10c}, NO₂, acyle en C₁-C₁₂, carboxy en C₁-C₁₀, ou
représente un radical aryle en C₆-C₁₅ ou un radical hétéroaryle en C₂-C₁₅, qui peut dans chaque cas éventuellement porter 1 à 5 substituants, choisis parmi alkyle en C₁-C₆, perfluoroalkyle en C₁-C₆, alcoxy en C₁-C₆, aralkyle en C₇-C₁₂, halogéno, SiR^{5d}R^{6d}R^{7d}, aryle en C₆-C₁₀ substitué ou non substitué, NR^{8d}R^{9d}, SR^{10d}, NO_{2,} ou
représente un groupe fonctionnel ou un hétéroatome choisi dans le groupe constitué par -O-, -S-, -N(R¹¹)-, -S(O)-, -C(O)-, -S(O)₂-, -P(R¹¹)-, - (R¹¹) P(O)- et -Si(R¹²R¹³),
les radicaux R^{5a}, R^{6a}, R^{7a}, R^{8a}, R^{9a}, R^{10a} à R^{5d}, R^{6d}, R^{7d}, R^{8d}, R^{9d}, R^{10d}, R¹¹, R¹² et R¹³ étant choisis chacun indépendamment les uns des autres parmi des groupes alkyle en C₁-C₆, aralkyle en C₇-C₁₂ et/ou aryle en C₆-C₁₀ substitué ou non substitué et les radicaux R^{8a} et R^{9a}, R^{8b} et R^{9b}, R^{8c} et R^{9c}, R^{8d} et R^{9d} pouvant également former chaque fois ensemble, indépendamment les uns des autres, un radical hydrocarboné cyclique ayant de 2 à 8 atomes de carbone, qui peut comporter un ou plusieurs hétéroatomes identiques ou différents, choisis dans le groupe constitué par O, S, NR^{11a}, et R^{11a} pouvant avoir les significations indiquées pour R¹¹,
sous forme libre et/ou liée en complexe.

11. Procédé selon la revendication 10, **caractérisé en ce qu'**on introduit la solution ou une partie de la solution dans un récipient de cristallisation (1) à une température qui est inférieure à 95 °C.

12. Procédé pour l'obtention d'une substance à partir d'une solution par cristallisation,
dans lequel on fait passer la solution dans un premier récipient de cristallisation (1) et on sépare la substance par cristallisation dans le premier récipient de cristallisation (1) conformément au procédé selon l'une quelconque des revendications 1 à 11 et
dans lequel pendant l'exécution du processus de cristallisation dans le premier récipient de cristallisation (1) on fait passer la solution dans un second récipient de cristallisation (1') et on sépare la substance par cristallisation dans le second récipient de cristallisation (1') conformément au procédé selon l'une quelconque des revendications 1 à 11.

13. Procédé pour le traitement final d'un produit de réaction contenant de l'aluminium, provenant de la production d'isopulégol par cyclisation du citronellal, contenant
i) de l'isopulégol,
ii) au moins un ligand de formule (I) 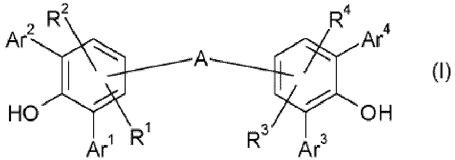 dans laquelle
Ar¹, Ar², Ar³, Ar⁴ sont choisis indépendamment les uns des autres parmi des radicaux aryle en C₆-C₁₅ ou des radicaux hétéroaryle en C₂-C₁₅, qui peuvent éventuellement porter chacun 1 à 7 substituants identiques ou différents, choisis parmi alkyle en C₁-C₆, perfluoroalkyle en C₁-C₆, alcoxy en C₁-C₆, aralkyle en C₇-C₁₂, halogéno, SiR^{5a}R^{6a}R^{7a}, aryle en C₆-C₁₀ éventuellement substitué, NR^{8a}R^{9a}, SR^{10a}, NO₂,
R¹, R², R³, R⁴ sont choisis indépendamment les uns des autres parmi un atome d'hydrogène, des groupes alkyle en C₁-C₆, perfluoroalkyle en C₁-C₆, alcoxy en C₁-C₆, aralkyle en C₇-C₁₂, des atomes d'halogène, des groupes SiR^{5b}R^{6b}R^{7b}, aryle en C₆-C₁₀ éventuellement substitué, NR^{8b}R^{9b}, SR^{10b}, NO₂ et R¹ ou R² et/ou R³ ou R⁴ pouvant former conjointement avec A un cycle aromatique ou non aromatique, et
A représente un radical hydrocarboné à chaîne droite ou ramifié et/ou cyclique ayant de 1 à 25 atomes de carbone, qui peut être saturé ou une ou plusieurs fois insaturé et/ou partiellement aromatique et peut éventuellement comporter un ou plusieurs hétéroatomes identiques ou différents, choisis parmi O, S, NR¹¹, et/ou un ou plusieurs groupes fonctionnels identiques ou différents, choisis parmi les groupes fonctionnels C(O), S(O), S(O)₂ et peut éventuellement porter un ou plusieurs substituants identiques ou différents, choisis parmi les substituants alkyle en C₁-C₆, perfluoroalkyle en C₁-C₆, alcoxy en C₁-C₆, acyloxy en C₁-C₁₀, aralkyle en C₇-C₁₂, halogéno, -SiR^{5c}R^{6c}R^{7c}, aryle en C₆-C₁₀ éventuellement substitué, hétéroaryle en C₂-C₁₀ substitué ou non substitué, NR^{8c}R^{9c}, SR^{10c}, NO_{2,} acyle en C₁-C₁₂, carboxy en C₁-C₁₀, ou
représente un radical aryle en C₆-C₁₅ ou un radical hétéroaryle en C₂-C₁₅, qui peut dans chaque cas éventuellement porter 1 à 5 substituants, choisis parmi alkyle en C₁-C₆, perfluoroalkyle en C₁-C₆, alcoxy en C₁-C₆, aralkyle en C₇-C₁₂, halogéno, SiR^{5d}R^{6d}R^{7d}, aryle en C₆-C₁₀ substitué ou non substitué, NR^{8d}R^{9d}, SR^{10d}, NO₂, ou
représente un groupe fonctionnel ou un hétéroatome choisi dans le groupe constitué par -O-, -S-, -N (R¹¹) -, -S(O)-, -C(O)-, -S(O)₂-, -P(R¹¹) -, - (R¹¹)P(O)- et -Si(R¹²R¹³),
les radicaux R^{5a}, R^{6a}, R^{7a}, R^{8a}, R^{9a}, R^{10a} à R^{5d}, R^{6d}, R^{7d}, R^{8d}, R^{9d}, R^{10d}, R¹¹, R¹² et R¹³ étant choisis chacun indépendamment les uns des autres parmi des groupes alkyle en C₁-C₆, aralkyle en C₇-C₁₂ et/ou aryle en C₆-C₁₀ substitué ou non substitué et les radicaux R^{8a} et R^{9a}, R^{8b} et R^{9b}, R^{8c} et R^{9c}, R^{8d} et R^{9d} pouvant également former chaque fois ensemble, indépendamment les uns des autres, un radical hydrocarboné cyclique ayant de 2 à 8 atomes de carbone, qui peut comporter un ou plusieurs hétéroatomes identiques ou différents, choisis dans le groupe constitué par O, S, NR^{11a}, et R^{11a} pouvant avoir les significations indiquées pour R¹¹,
sous forme libre et/ou liée en complexe.
dans lequel
a) on soumet le produit de réaction à une séparation par distillation avec obtention d'un produit de tête enrichi en isopulégol et d'un produit de bas de colonne appauvri en isopulégol,
b) on met le produit de bas de colonne appauvri en isopulégol en contact intime avec une base aqueuse pour obtenir une phase aqueuse contenant de l'aluminium et une phase organique contenant la quantité principale des ligands de formule (I),
c) on sépare les ligands de formule (I) d'avec la phase organique conformément au procédé selon l'une quelconque des revendications 1 à 11.

14. Procédé pour la production d'isopulégol de formule (IV) 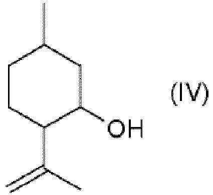 comprenant
α) la cyclisation du citronellal de formule (V) 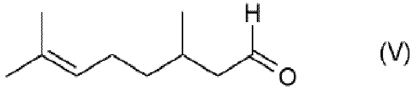 en présence d'un catalyseur qui peut être obtenu par mise en réaction d'un ligand bis(diarylphénol) de formule (I), telle que définie dans la revendication 10,
avec un composé d'aluminium de formule (II),
(R¹⁴)₃-ₚAlHₚ (II)
dans laquelle
Al signifie l'aluminium,
R¹⁴ représente un radical alkyle ramifié ou non ramifié ayant de 1 à 5 atomes de carbone et
p représente 0 ou un nombre entier de 1 à 3,
et/ou
avec un composé d'aluminium de formule (III),
MAlH₄ (III)
dans laquelle
Al signifie l'aluminium,
M représente le lithium, le sodium ou le potassium,
β) la récupération du ligand bis(diarylphénol) de formule (I) une fois effectuée la réaction,
a) en soumettant le produit de réaction obtenu dans l'étape α) à une séparation par distillation avec obtention d'un produit de tête enrichi en isopulégol et d'un produit de bas de colonne appauvri en isopulégol,
b) en mettant le produit de bas de colonne appauvri en isopulégol en contact intime avec une base aqueuse pour obtenir une phase aqueuse contenant de l'aluminium et une phase organique contenant la quantité principale des ligands de formule (I),
c) en séparant les ligands de formule (I) d'avec la phase organique conformément ai procédé selon l'une quelconque des revendications 1 à 11.

15. Procédé pour la production de menthol, comprenant les étapes :
A) production d'isopulégol de formule (IV) selon la revendication 14 et
B) hydrogénation de la double liaison éthylénique de l'isopulégol ainsi obtenu.
